# EUROPEAN PATENT SPECIFICATION

(11) **EP 1 664 796 B1**
(45) Date of publication and mention of the grant of the patent: **15.12.2010**
(21) Application number: 04788750.0
(22) Date of filing: 15.09.2004
(51) Int. Cl.: G01N 33/68

(54) **METHOD OF USING CYTOKINE ASSAYS TO DIAGNOSE, TREAT, AND EVALUATE ANKYLOSING SPONDYLITIS**
VERFAHREN ZUR VERWENDUNG VON CYTOKINTESTS ZUR DIAGNOSE, BEHANDLUNG UND BEURTEILUNG VON ANKYLOIDER SPONDYLITIS
METHODE D'UTILISATION DE MESURES DE CYTOKINES POUR DIAGNOSTIQUER, TRAITER ET EVALUER LA SPONDYLARTHRITE ANKYLOSANTE

(30) Priority: 15.09.2003 US 503131 P
(43) Date of publication of application: 07.06.2006
(60) Divisional application: 10176365.4
(73) Proprietor: Oklahoma Medical Research Foundation, Oklahoma City, OK 73104 (US); THE BOARD OF REGENTS OF THE UNIVERSITY OF OKLAHOMA, Norman, Oklahoma 73019 (US)
(72) Inventor: CHAPPELL, Cherie, M., Oklahoma City, OK 73104 (US); CENTOLA, Michael, B., Oklahoma City, OK 73104 (US); ALEX, Philip, J., Oklahoma City, OK 73179 (US); HENSLEY, Kenneth, Sylvania, OH 43560 (US)
(74) Representative: Dehmel, Albrecht
(86) International application number: PCT/US2004/030059
(87) International publication number: WO 2005/029091

(56) References cited:
- GRATACOS J ET AL: "Serum cytokines (IL-6, TNF-alpha, IL-1-beta and IFN-gamma) in ankylosing spondylitis: A close correlation between serum IL-6 and disease activity and severity" BRITISH JOURNAL OF RHEUMATOLOGY, vol. 33, no. 10, 1994, pages 927-931, XP009041297 ISSN: 0263-7103
- ZOU JIANXIANG ET AL: "Down-regulation of the nonspecific and antigen-specific T cell cytokine response in ankylosing spondylitis during treatment with infliximab." ARTHRITIS AND RHEUMATISM. MAR 2003, vol. 48, no. 3, March 2003 (2003-03), pages 780-790, XP001204159 ISSN: 0004-3591
- CHIU BO-CHIN ET AL: "Cytokine-chemokine networks in experimental mycobacterial and schistosomal pulmonary granuloma formation." AMERICAN JOURNAL OF RESPIRATORY CELL AND MOLECULAR BIOLOGY, vol. 29, no. 1, July 2003 (2003-07), pages 106-116, XP001204074 ISSN: 1044-1549
- BADEWA ABIODUN PHILIP ET AL: "Effects of eotaxin (CCL11), eotaxin-2 (CCL24) and eotaxin-3 (CCL26) on human eosinophil proinflammatory functions" FASEB JOURNAL, vol. 16, no. 4, 20 March 2002 (2002-03-20), pages A331-A332, XP009041281 & ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- XIA CHANG-QING ET AL: "Suppressed production of IL-10 contributes to up-regulated IL-12 secretion by dendritic cells stimulated with LPS or LPS plus IFN-gamma in the presence of ERK activation inhibitor (PD98059)" FASEB JOURNAL, vol. 16, no. 5, 22 March 2002 (2002-03-22), page A1230, XP009041279 & ANNUAL MEETING OF PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- JINUSHI M ET AL: "382 Autocrine IL-15 is required for MHC class I-related chain A and B expression on monocyte-derived dendritic cells in response to interferon A/B: impairment of interferon-induced IL-15 production in hepatitis C virus infection" HEPATOLOGY, WILLIAMS AND WILKINS, BALTIMORE, MD, US, vol. 38, 2003, page 343, XP004623598 ISSN: 0270-9139
- KLOSS CHRISTIAN U A ET AL: "Proliferation of ramified microglia on an astrocyte monolayer: Characterization of stimulatory and inhibitory cytokines" JOURNAL OF NEUROSCIENCE RESEARCH, vol. 49, no. 2, 1997, pages 248-254, XP009041278 ISSN: 0360-4012
- FURIHATA MUTSUO ET AL: "Lung squamous cell carcinoma producing both parathyroid hormone-related peptide and granulocyte colony stimulating factor" PATHOLOGY INTERNATIONAL, vol. 46, no. 5, 1996, pages 376-379, XP009041277 ISSN: 1320-5463
- METELITSA LEONID S ET AL: "Natural killer T cells infiltrate neuroblastomas expressing the chemokine CCL2." JOURNAL OF EXPERIMENTAL MEDICINE, vol. 199, no. 9, 3 May 2004 (2004-05-03), pages 1213-1221, XP001204357 ISSN: 0022-1007
- CHAPPELL CHERIE M ET AL: "Distinct Immunopathogenic Cytokine Profiles of the Spondylarthropathies" FASEB JOURNAL, vol. 18, no. 4-5, 2004, pages Abst. 148.5 URL-http://ww, XP009040784 & FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638
- SZODORAY P ET AL: "Circulating cytokines in primary Sjogren's syndrome determined by a multiplex cytokine array system" SCANDINAVIAN JOURNAL OF IMMUNOLOGY, vol. 59, no. 6, June 2004 (2004-06), pages 592-599, XP001204356 ISSN: 0300-9475
- MEGJUGORAC NICHOLAS J ET AL: "Virally stimulated plasmacytoid dendritic cells produce chemokines and induce migration of T and NK cells." JOURNAL OF LEUKOCYTE BIOLOGY, vol. 75, no. 3, March 2004 (2004-03), pages 504-514, XP009041275 ISSN: 0741-5400

## Description

### STATEMENT AS TO RIGHTS TO INVENTIONS MADE UNDER FEDERALLY-SPONSORED RESEARCH AND DEVELOPMENT

### BACKGROUND OF THE INVENTION

### A. Field of the Invention

The invention relates generally to inflammatory, infectious, and autoimmune disorders. More particularly the invention relates to chronic inflammatory disease. An implementation of the invention relates to the diagnosis, prognosis, evaluation, or treatment response of a disease such as spondyloarthropathy, Ankylosing spondylitis, psoriatic arthritis, reactive arthritis, enteropathic arthritis, ulcerative colitis, Crohn's disease, irritable bowel disease, rheumatoid arthritis, juvenile rheumatoid arthriris, systemic lupus erythematosus, familial Mediterranean fever, amyotrophic lateral sclerosis, Sjogren's syndrome, early arthritis, viral arthritis, multiple sclerosis, or psoriasis.

### B. Discussion of the Related Art

Many inflammatory, infectious, and autoimmune diseases are now recognized to involve inflammatory reactions as a major pathologic feature. Inflammatory processes are driven by cytokine and chemokine mechanisms. As used herein, the term "cytokine" is defined as any of several regulatory proteins, such as the interleukins and lymphokines, that are released by cells of the immune system and act as intercellular mediators in the generation of an immune response. Cytokines are secreted by immune or other cells, whose action is on cells of the immune system, such as, but not limited to, T-cells, B-cells, NK cells and macrophages. "Chemokines" are defined as chemotactic cytokines produced by a variety of cell types in acute and chronic inflammation that mobilize and activate white blood cells. Chemokines can be subdivided into classes on the basis of the arrangement of a pair of conserved cysteines.

Cytokines and chemokines are important cell signaling proteins, mediating a wide range of physiological responses, including immunity, inflammation, and hematopoiesis. Recently, new biological therapeutic agents, primarily directed at cytokines, have shown great promise in the treatment of many inflammatory arthritic diseases. However, despite the advances in pharmaceutical technology, physicians still prescribe these expensive, powerful and potentially dangerous therapeutic agents with no indication of whether patients have the specific inflammatory mediator antagonized by the pharmaceutical, or whether patients will positively respond to the medications.

Despite a number of reports linking the presence or absence of certain individual cytokines and chemokines to disease states, it is possible that some assay procedures detect very little cytokine, whereas others pick up none at all. This difference may be related to the assay system, to the cytokine, or both. The problem has been reported by the observations of Cannon *et al.* (1988), in which the authors showed that some plasma substance inhibited the assay, affecting detection. The authors recommend chloroform extraction of plasma to remove interfering substances, but it is not clear from this study if the plasma factors simply affect the performance of the assay or are related to the cytokine itself. This question was further described by Capper *et al.* (1990), which showed that IL-1α and IL-1β are bound by proteins and that the dissociation of these molecules from these serum binding proteins by acidifying the plasma changes the detectable levels. Thus, there have been many attempts to measure endogenous cytokines in blood and other body fluids. However, it is apparent that there is wide variation in the reported results with regard to cytokine concentration in the blood and to fluctuations of cytokines concentration in the blood.

The following publications are relevant for the present invention to a certain extent:
Gratacos et al., British Journal of Rheumatology 33, 927-931 (1994): Serum interleukin-6 (IL-6), tumor necrosis factor-alpha (TNF-alpha), interleukin-1 beta (IL-1 beta) and interferon-gamma (IFN-gamma) levels in patients with AS were examined and their relationship with disease activity assessed. IL-6 and TNF-alpha serum levels, but not IL-1 beta and IFN-gamma, were significantly higher in AS than in NIBP. However, patients with RA showed higher serum levels of IL-6, TNF-alpha and IFN-gamma than both AS and NIBP patients. In AS, IL-6 correlated with clinical parameters of disease activity with significant correlation being observed with laboratory parameters of inflammation such as ESR, CRP, platelet count and clinical parameters of severity such as vertebral mobility. TNF-alpha did not correlate with laboratory or clinical parameters of activity. Macrophagic cytokines (TNF-alpha and IL-6), were increased in AS patients and IL-6 closely correlated with the activity of the disease.

Zou et al., Arthritis & Rheumatism 48, 780-790 (2003): Cytokine expression of CD4+ and CD8+ T cells was investigated before and 6 and 12 weeks after the start of treatment in 10 patients treated with infliximab, and before and after 6 weeks of treatment and 6 weeks after placebo was switched to infliximab in 10 patients treated initially with placebo. Infliximab was shown to down-regulate both IFN gamma and TNF alpha secreted by T cells, but not induce a change in cytokines produced by monocytes during 3 months of treatment.

Chiu et al., American Journal of Respiratory Cell and Molecular Biology 29, 106-116 (2003): Type-1 and type-2 lung granulomas display different patterns of chemokine expression. Expression profiles for 16 chemokines for these granulomas were determined and *the in vivo* effects of neutralizing antibodies against interferon-gamma (IFN-gamma), interleukin (IL)-4, IL-10, IL-12, and IL-13 were assessed. Transcripts for CXCL2, -5, -9, -10, and -11 and the CCL chemokine, CCL3, and lymphotactin (XCL1), were largely enhanced by Thl-related cytokines, IFN-gamma or IL-12. Transcripts for CCL11, CCL22, CCL17, and CCL1 were enhanced largely by Th2-related cytokines, IL-4, IL-10, or IL-13. Transcripts for CCL4, CCL2, CCL8, CCL7, and CCL12 were potentially induced by either Th1- or Th2-related cytokines, although some of these showed biased expression. IFN-gamma and IL-4 enhanced the greatest complement of transcripts, and their neutralization had the greatest anti-inflammatory effect on type-1 and type-2 granulomas, respectively.

Badewa et al., Experimental Biology: Meeting Abstracts A331-A332, 245.6: CCL11, CCL24 and CCL26 directly stimulate superoxide anion release and EPO degranulation. Anti-eotaxin antibody abrograted the effects. Culturing EOS with IL-5 further enhanced responses. Thus CCL11, CCL24 and CCL26 modulate EOS functions directly, as priming/potentiating agents and by acting cooperatively with IL-5.

Xia et al., Experimental Biology: Meeting Abstracts A1230, 929.8: Human monocyte-derived CD1a+ DC activated with LPS produced more IL-10 than IL-12, but if IFNγ was added to LPS, the cells produced more IL-12 and lesser IL-10. Exogenous IL-10 inhibited production of IL-12 by 4-5-times, whereas exogenous IL-12 had no effect on IL-10 production.

Jinushi et al., Hepatology 38, 343A (2003): Induction of MICA/B on DCs by various innate cytokines was examined and DCs from either healthy donors or HCV-infected individuals, upon IL-15 stimulation, were shown to express MICA/B and activate NK cells. IL-15- and type I IFN-mediated induction of MICA/B in healthy donors was completely inhibited when DCs are incubated in the presence of anti-IFN-alpha/betaR or anti-IL-15Ralpha. Indeed, DCs produced IL-15 in response to type I IFN, whereas they directly produced IFN-beta, in response to IL-15, which was followed by the production of IFN-alpha. In HCV-infected individuals, type I IFN-mediated production of IL-15 was virtually absent, but IL-15-mediated production of type I IFN was not compromised, which is consistent with the distinct ability of these cytokines to induce MICA/B in these patients.

Kloss et al., J. Neuroscience Res. 49, 248-254 (1997): The effect of different cytokines on the proliferation of ramified microglia was studied in vitro. Addition of macrophage colony-stimulating factor (MCSF), granulocyte-macrophage colony-stimulating factor (GMCSF), and interleukin-3 (IL3), stimulated the proliferation of ramified microglia, with a 7.2-fold, 3.5-fold, and 1.5-fold increase, respectively. Of all the other cytokines tested (IL1, IL2, IL4, IL6, IL10, interferon-gamma (IFNgamma), leukemia inhibitory factor (LIF), and tumor necrosis factor-alpha (TNFalpha) only IL1 strongly enhanced proliferation. However, this effect of IL1 was indirect and could be neutralized by antibodies against MCSF and GMCSF. IL2, IL4, IL10, TNFalpha, and IFNgamma inhibited microglial proliferation.

Furihata et al., Pathology Internat. 46, 376-379 (1996): A primary squamous cell carcinoma of the lung exhibited high levels of serum parathyroid hormone-related peptide (PTHrP) and granulocyte colony stimulating factor (GCSF). The tumor was successfully transplanted into nude mice in which the high levels of serum PTHrP and GCSF were reproduced.

Thus, despite the need to identify cytokine associations with various inflammatory diseases, there has yet to be established a definitive link between cytokine expression and diagnosis, prognosis, and treatment response of such pathologic states.

### SUMMARY OF THE INVENTION

Thus, in accordance with the present invention, there are provided methods in accordance with the attached claims 1 to 7.

The patient sample used in these methods may comprise peripheral blood, serum, plasma, cerebrospinal fluid, tissue sample, skin, or other body fluid sample. The patient may have mild, intermediate, or severe disease. The predefined levels may comprise information about a median level of the cytokine found in the patient sample. The patient sample may be from a healthy subject, a diseased patient or a patient having major joint destruction and/or extra-articular involvement.

As used herein, the term "cytokine" is defined any of several regulatory proteins, such as the interleukins and lymphokines, that are released by cells of the immune system and act as intercellular mediators in the generation of an immune response. Cytokines are secreted by immune or other cells, whose action is on cells of the immune system, such as, but not limited to, T-cells, B-cells, NK cells, neutrophils, and macrophages. The cytokines assayed according to the methods of the invention are interleukin-4 (IL-4), interleukin-6 (IL-6), interleukin-8 (IL-8/CXCL8), interleukin-13 (IL13), interleukin-17 (IL-17), tumor necrosis factor-α (TNF-α), interferon-γ (INF-γ), granulocyte-monocyte colony stimulating factor (GM-CSF), granulocyte colony stimulating factor (G-CSF) monocyte chemoattractant protein-1 (MCP-1/CCL2), and macrophage inflammatory protein-1β (MIP-1β/CCL4), Eotaxin (CCL11), variable endothelial growth factor (VEGF), endotheial growth factor (EGF), or fibroblast growth factor (FGF).

Where the method disease state is psoriatic arthritis, the cytokine is selected from the group consisting of GM-CSF, IL-17, IL-2, IL-10, IL-13, IFN-γ, IL-6, CCL4/MIP-1β, CCL11/Eotaxin, EGF, and CCL2/MCP-1.

Where the method disease state is reactive arthritis, the cytokine is selected from the group consisting of IL-12, IFN-γ, IL-1β, IL-13, IL-17, CCL4/MCP-1, TNF-α, IL-4, GM-CSF, CCL11/Eotaxin, EGF, and IL-6.

Where the method disease state is enteropathic arthritis, the cytokine is selected from the group consisting of CXCL8/IL-8, IL-1β, IL-4, G-CSF. CCL2/MCP-1, CCL11/Eotaxin, EGF, IFN-γ, and TNF-α.

Where the method disease state is ulcerative colitis (UC), the cytokine is selected from the group consisting of IL-7, CXCL8/IL-8, IFN-γ, TNF-α, EGF, VEGF, and IL-1β

Where the method disease state is Crohn's Disease (CD), the cytokine is selected from the group consisting of TNF-α, IFN-γ, IL-1β, IL-6, IL-7, IL-13, IL-2, IL-4, GM-CSF, G-CSF, CCL2/MCP-1, EGF, VEGF, and CXCL8/IL-8.

Where the method disease state is rheumatoid arthritis, the cytokine is selected from the group consisting of IFN-γ, IL-1β, TNF-α, G-CSF, GM-CSF, IL-6, IL-4, IL-10, IL-13, IL-5, CCL4/MIP-1β, CCL2/MCP-1, EGF, VEGF, and IL-7.

Where the method disease state is systemic lupus erythematosus, the cytokine is selected from the group consisting of IL-10, IL-2, IL-4, IL-6, IFN-γ, CCL2/MCP-1, CCL4/MIP-1β, CXCL8/IL-8, VEGF, EGF, and IL-17.

Where the method disease state is Familial Mediterranean Fever (FMF), the cytokine is selected from the group consisting of G-CSF, IL-2, IFN-γ, TNF-α, IL-1β, and CXCL8/IL-8.

Where the method disease state is amyotrophic lateral sclerosis (ALS), the cytokine is selected from the group consisting of CCL2/MIP-1β, CXCL8/IL-8, IL-12, IL-1β, VEGF, and IL-13.

Where the method disease state is Irritable Bowel Syndrome (IBS), the cytokine is selected from the group consisting of TNF-α, IFN-γ, IL-1β, IL-6, IL-7, GM-CSF, G-CSF, CCL2/MCP-1, and CXCL8/IL-8.

Where the method disease state is Juvenile Rheumatoid Arthritis (JRA), the cytokine is selected from the group consisting of IFN-γ, IL-1β, TNF-α, G-CSF, GM-CSF, IL-6, IL-4, IL-10, IL-13, IL-5, and IL-7.

Where the method disease state is Sjogren's Syndrome, the cytokine is selected from the group consisting of CCL2/MCP-1, IL-12, CXCL8/IL-8, CCL11/Eotaxin, TNFα, IL-2, IFNα, IL-15, IL17, IL-1α, IL-1β, IL-6, and GM-CSF.

Where the method disease state is early arthritis, the cytokine is selected from the group consisting of CCL4/MIP1β, CXCL8/IL-8, IL-2, IL-12, IL-17, IL-13, TNFα, IL-4, IL-5, and IL-10.

Where the method disease state is neuroinflammation, the cytokine is selected from the group consisting of CCL2/MCP-1, IL-12, GM-CSF, G-CSF, M-CSF, IL-6, and IL-17.

The method may comprise determining the level of 2, 3, 4, 5, 6, 7, 8, 9, 10, 11, 12, 13, 14, 15, 16, 17, 18, 19 or 20 cytokines.

Also provided is a method for assessing treatment for an inflammatory or autoimmune disease state comprising (a) subjecting an inflammatory or autoimmune disease state patient to a treatment; (b) obtaining a sample from said patient; (c) measuring the level of a plurality of cytokines within the patient sample; (d) comparing the level of a plurality of cytokines with predefined cytokine levels; and (e) determining if the treatment is efficacious.

The method may further comprise making a decision regarding modifying the therapeutic regimen based on said determination of efficacy. The patient sample may comprise peripheral blood, serum, plasma, cerebrospinal fluid, tissue sample, skin, or other body fluid sample. The predefined levels may comprise information about a median level of the cytokine found in the patient sample. The predefined levels may comprise pretreatment levels of said one or more cytokines, levels of said one or more cytokines observed in healthy subjects and/or a patient with the disease. The patient sample may be from a patient having extra-articular involvement and/or having major joint destruction.

Where the method disease state is ankylosing spondylitis, the cytokine is selected from the group consisting of CCL4, CCL2, CCL11, EGF, IL-1β, IL-2, IL-5, IL-6, IL-7, CXCL8, IL-10, IL-12, IL-13, IL-15, IL-17, TNF-α, IFNγ, GM-CSF, or G-CSF.

Where the method disease state is psoriatic arthritis, the cytokine is selected from the group consisting of GM-CSF, IL-17, IL-2, IL-10, IL-13, IFN-γ, IL-6, CCL4/MIP-1β, CCL11/Eotaxin, EGF, and CCL2/MCP-1.

Where the method disease state is reactive arthritis, the cytokine is selected from the group consisting of IL-12, IFN-γ, IL-1β, IL-13, IL-17, CCL4/M4CP-1, TNF-α, IL-4, GM-CSF, CCL11/Eotaxin, EGF, and IL-6.

Where the method disease state is enteropathic arthritis, the cytokine is selected from the group consisting of CXCL8/IL-8, IL-1β, IL-4, G-CSF. CCL2/MCP-1, CCL11/Eotaxin, EGF, IFN-γ, and TNF-α.

Where the method disease state is ulcerative colitis (UC), the cytokine is selected from the group consisting of IL-7, CXCL8/IL-8, IFN-γ, TNF-α, EGF, VEGF, and IL-1β.

Where the method disease state is Crohn's Disease (CD), the cytokine is selected from the group consisting of TNF-α, IFN-γ, IL-1β, IL-6, IL-7, IL-13, IL-2, IL-4, GM-CSF, G-CSF, CCL2/MCP-1, EGF, VEGF, and CXCL8/IL-8.

Where the method disease state is rheumatoid arthritis, the cytokine is selected from the group consisting of IFN-γ, IL-1β, TNF-α, G-CSF, GM-CSF, IL-6, IL-4, IL-10, IL-13, IL-5, CCL4/MIP-1β, CCL2/MCP-1, EGF, VEGF, and IL-7.

Where the method disease state is systemic lupus erythematosus, the cytokine is selected from the group consisting of IL-10, IL-2, IL-4, IL-6, IFN-γ, CCL2/MCP-1, CCL4/MIP-1β, CXCL8/IL-8, VEGF, EGF, and IL-17.

Where the method disease state is Familial Mediterranean Fever (FMF), the cytokine is selected from the group consisting of G-CSF, IL-2, IFN-γ, TNF-α, IL-1β, and CXCL8/IL-8.

Where the method disease state is amyotrophic lateral sclerosis (ALS), the cytokine is selected from the group consisting of CCL2/MIP-1β, CXCL8/IL-8, IL-12, IL-1β, VEGF, and IL-13.

Where the method disease state is Irritable Bowel Syndrome (IBS), the cytokine is selected from the group consisting of TNF-α, IFN-γ, IL-1β, IL-6, IL-7, GM-CSF, G-CSF, CCL2/MCP-1, and CXCL8/IL-8.

Where the method disease state is Juvenile Rheumatoid Arthritis (JRA), the cytokine is selected from the group consisting of IFN-γ, IL-1β, TNF-α, G-CSF, GM-CSF, II-6, IL-4, IL-10, IL-13, IL-5, and IL-7.

Where the method disease state is Sjogren's Syndrome, the cytokine is selected from the group consisting of CCL2/MCP-1, IL-12, CXCL8/IL-8, CCL11/Eotaxin, TNFα, IL-2, IFNα, IL-15, IL17, IL-1α, IL-1β, IL-6, and GM-CSF.

Where the method disease state is early arthritis, the cytokine is selected from the group consisting of CCL4/MIP1β, CXCL8/IL-8, IL-2, IL-12, IL-17, IL-13, TNFα, IL-4, IL-5, and IL-10.

Where the method disease state is neuroinflammation, the cytokine is selected from the group consisting of CCL2/MCP-1, IL-12, GM-CSF, G-CSF, M-CSF, IL-6, and IL-17.

The method may comprise determining the level of 2, 3, 4, 5, 6, 7, 8, 9, 10, 11, 12, 13, 14, 15, 16, 17, 18, 19 or 20 cytokines.

In yet another embodiment, there is provided a method for determining if a patient with an inflammatory or autoimmune disease state is predisposed to develop severe inflammatory or autoimmune disease state, comprising (a) obtaining a patient sample; (b) measuring the level of a plurality of cytokines within the patient sample; (c) comparing cytokine levels with predefined levels of the cytokines found in patients developing or having severe an inflammatory or autoimmune disease state; and (d) determining if said patient is predisposed to develop severe an inflammatory or autoimmune disease state.

The patient sample may comprise peripheral blood, serum, plasma, cerebrospinal fluid, tissue sample, or other body fluid sample. The predefined levels may comprise information about a median level of the cytokine found in the patient sample. The method may further comprise obtaining a plurality of patient symptoms. The patient sample may be from a patient having extra-articular involvement and/or having major joint destruction.

Where the method disease state is ankylosing spondylitis, the cytokine is selected from the group consisting of CCL4, CCL2, CCL11, EGF, IL-1β, IL-2, IL-5, IL-6, IL-7, CXCL8, IL-10, IL-12, IL-13, IL-15, IL-17, TNF-α, IFNγ, GM-CSF, or G-CSF.

Where the method disease state is psoriatic arthritis, the cytokine is selected from the group consisting of GM-CSF, IL-17, IL-2, IL-10, IL-13, IFN-γ, IL-6, CCL4/MIP-1β, CCL11/Eotaxin, EGF, and CCL2/MCP-1.

Where the method disease state is reactive arthritis, the cytokine is selected from the group consisting of IL-12, IFN-γ, IL-1β, IL-13, IL-17, CCL4/MCP-1, TNF-α, IL-4, GM-CSF, CCL11/Eotaxin, EGF, and IL-6.

Where the method disease state is enteropathic arthritis, the cytokine is selected from the group consisting of CXCL8/IL-8, IL-1β, IL-4, G-CSF. CCL2/MCP-1, CCL11/Eotaxin, EGF, IFN-γ, and TNF-α.

Where the method disease state is ulcerative colitis (UC), the cytokine is selected from the group consisting of IL-7, CXCL8/IL-8, IFN-γ, TNF-α, EGF, VEGF, and IL-1β.

Where the method disease state is Crohn's Disease (CD), the cytokine is selected from the group consisting of TNF-α, IFN-γ, IL-1β, IL-6, IL-7, IL-13, IL-2, IL-4, GM-CSF, G-CSF, CCL2/MCP-1, EGF, VEGF, and CXCL8/IL-8.

Also provided is a kit in accordance with attached claim 8 or antibody probe for determining the cytokine level of two or more of the cytokines selected from the group consisting of GM-CSF, IL-17, IL-2, IL-10, IL-13, IFN-γ, IL-6, CCL4/MIP-1β, CCL11/Eotaxin, EGF, and CCL2/MCP-1;
for providing diagnostic information about reactive arthritis comprising a nucleic acid or antibody probe for determining the cytokine level of two or more of the cytokines selected from the group consisting of lL-12, IFN-γ, IL-1β, IL-13, IL-17, CCL4/MCP-1, TNF-α, IL-4, GM-CSF, CCL11/Eotaxin, EGF, and IL-6;
for providing diagnostic information about enteropathic arthritis comprising a nucleic acid or antibody probe for determining the cytokine level of two or more of the cytokines selected from the group consisting of CXCL8/IL-8, IL-1β, IL-4, G-CSF, CCL2/MCP-1, CCL11/Eotaxin, EGF, IFN-γ, and TNF-α;
for providing diagnostic information about an ulcerative colitis comprising a nucleic acid or antibody probe for determining the cytokine level of two or more of the cytokines selected from the group consisting of IL-7, CXCL8/IL-8, IFN-γ, TNF-α, EGF, VEGF, and IL-1β;
for providing diagnostic information about Crohn's disease comprising a nucleic acid or antibody probe for determining the cytokine level of two or more of the cytokines selected from the group consisting of TNF-α, IFN-γ, IL-1β, IL-6, IL-7, IL-13, IL-2, IL-4, GM-CSF, G-CSF, CCL2/MCP-1, EGF, VEGF, and CXCL8/IL-8;
for providing diagnostic information about rheumatoid arthritis comprising a nucleic acid or antibody probe for determining the cytokine level of two or more of the cytokines selected from the group consisting of IFN-γ, IL-1β, TNF-α, G-CSF, GM-CSF, IL-6, IL-4, IL-10, IL-13, IL-5, CCL4/MIP-1β, CCL2/MCP-1, EGF, VEGF, and IL-7;
for providing diagnostic information about systemic lupus erythematosus comprising a nucleic acid or antibody probe for determining the cytokine level of two or more of the cytokines selected from the group consisting of IL-10, IL-2, IL-4, IL-6, IFN-γ CCL2/MCP-1, CCL4/MIP-1β CXCL8/IL-8, VEGF, EGF, and IL-17;
for providing diagnostic information about Familial Mediterranean Fever comprising a nucleic acid or antibody probe for determining the cytokine level of two or more of the cytokines selected from the group consisting of G-CSF, IL-2, IFN-γ TNF-α, IL-1β and CXCL8/IL-8;

As used herein, "a" or "an" may mean one or more. As used herein in the claim(s), when used in conjunction with the word "comprising," the words "a" or "an" may mean one or more than one. As used herein "another" may mean at least a second or more.

### BRIEF DESCRIPTION OF THE DRAWINGS

The drawings accompanying and forming part of this specification are included to depict certain aspects of the invention. A clearer conception of the invention, and of the components and operation of systems provided with the invention, will become more readily apparent by referring to the exemplary, and therefore non-limiting, embodiments illustrated in the drawings; wherein like reference numerals (if they occur in more than one view) designate the same elements. The invention may be better understood by reference to one or more of these drawings in combination with the description presented herein. It should be noted that the features illustrated in the drawings are not necessarily drawn to scale.

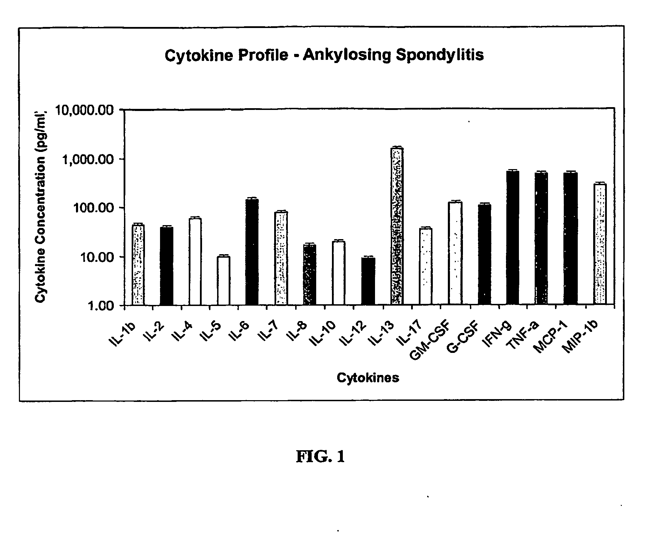

**FIG. 1** **-** Cytokine Profile of Ankylosing Spondylitis patients.

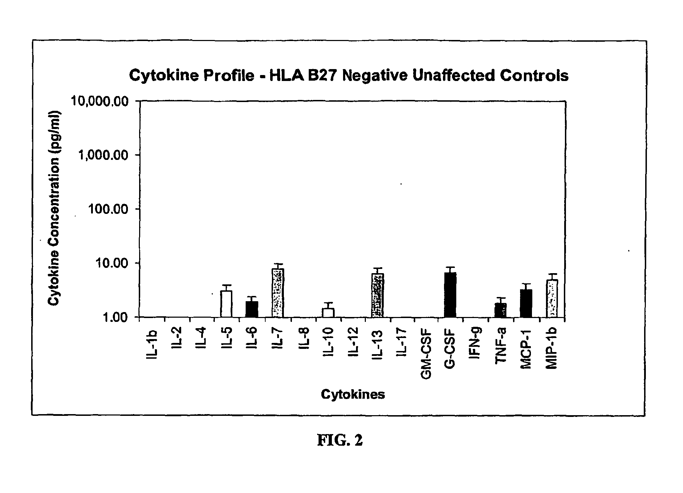

**FIG. 2** **-** Cytokine Profile from HLA B27 Negative Unaffected Controls.

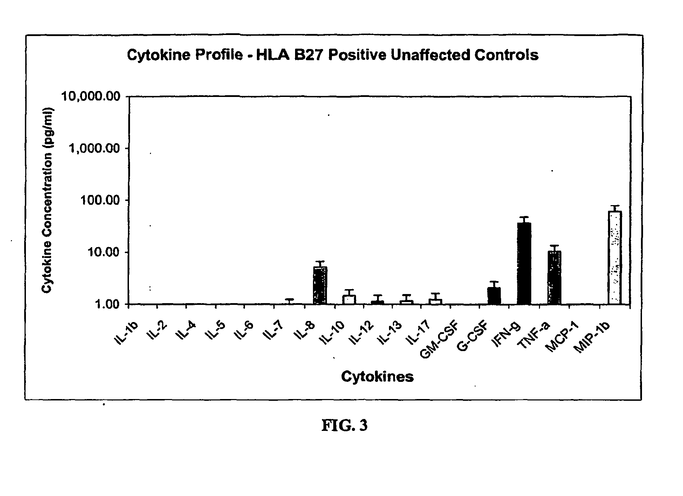

**FIG. 3****-** Cytokine Profile from HLA B27 Positive Unaffected Controls.

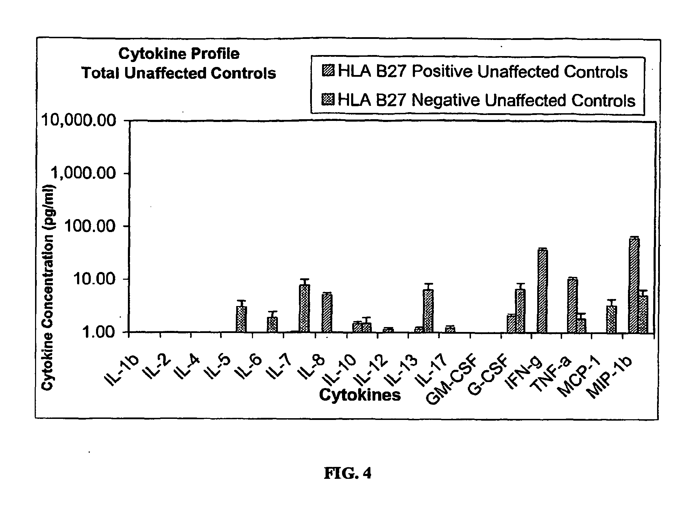

**FIG. 4** **-** Cytokine Profile from Total Unaffected Controls.

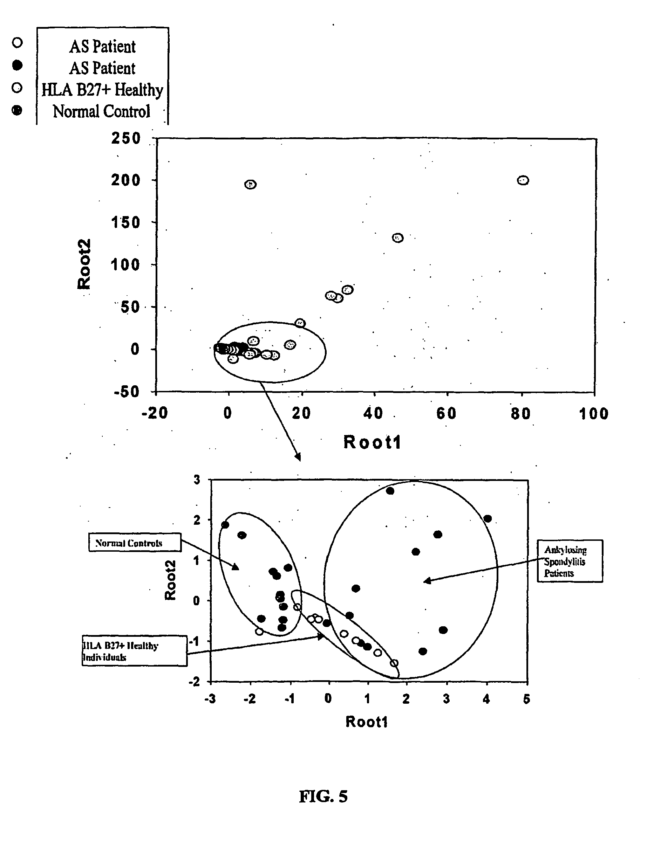

**FIG. 5** **-** Stepwise Discriminate Function Analysis Graph Showing Clustering of Normal Controls (red), HLA B27 positive Individuals (grey), and AS patients (blue/green). Roots used as coordinates were calculated as described in Table 1 legend. Note that the HLA B27 positive individuals (grey) appear to form a link between the normal controls (red) and clinically diagnosed AS patients (blue/green), once again confirming the importance of HLA B27 positive in disease development. It may also be of clinical interest to note that a few of the HLA B27 positive healthy individuals presented some early symptoms of AS, but those symptoms were insufficient to qualify them for a diagnosis of AS under the Bath criteria. These data indicate that an earlier disease diagnosis may be warranted in those cases.

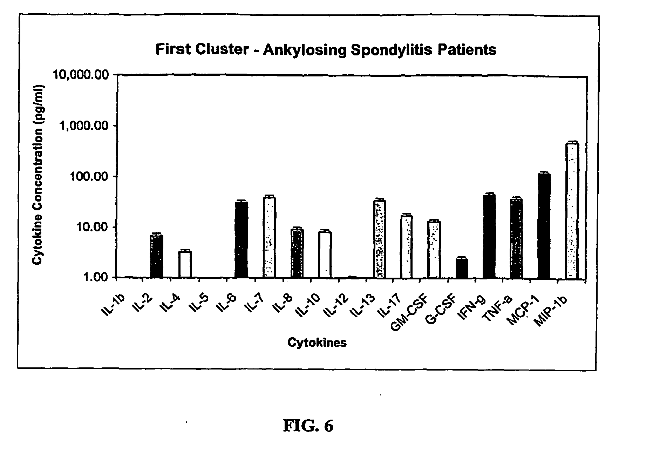

**FIG. 6** **-** First Cluster - Ankylosing Spondylitis Patients - Serum Cytokine Levels.

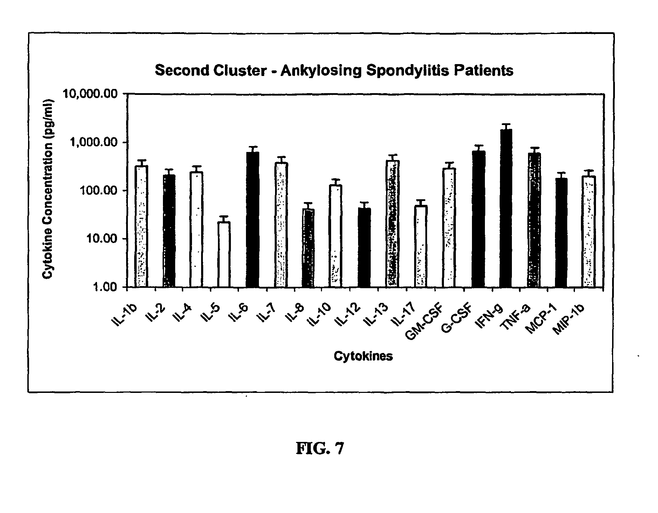

**FIG. 7** **-** Second Cluster - Ankylosing Spondylitis Patients - Serum Cytokine Levels.

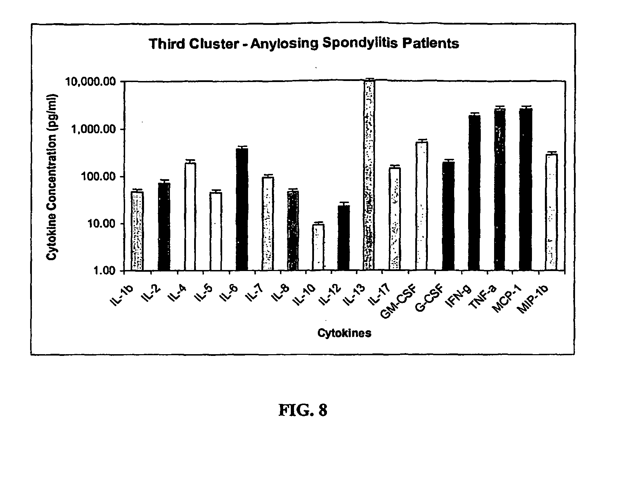

**FIG. 8** **-** Third Cluster - Ankylosing Spondylitis Patients - Serum Cytokine Levels.

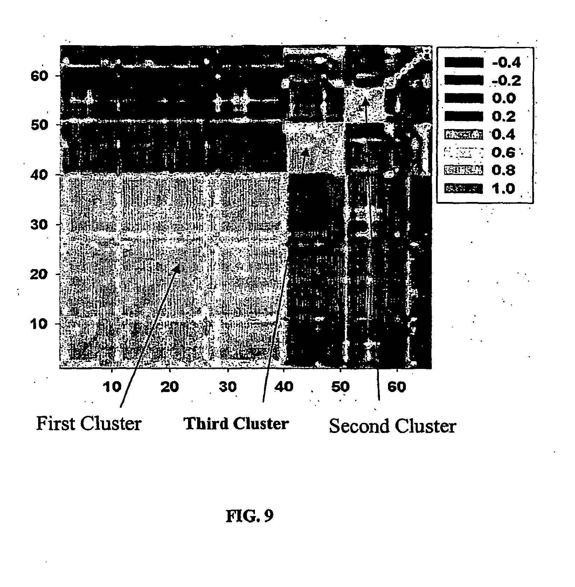

**FIG. 9****. -** Contour Graph Showing Distinct Clusters of AS Patients and Unaffected Controls.

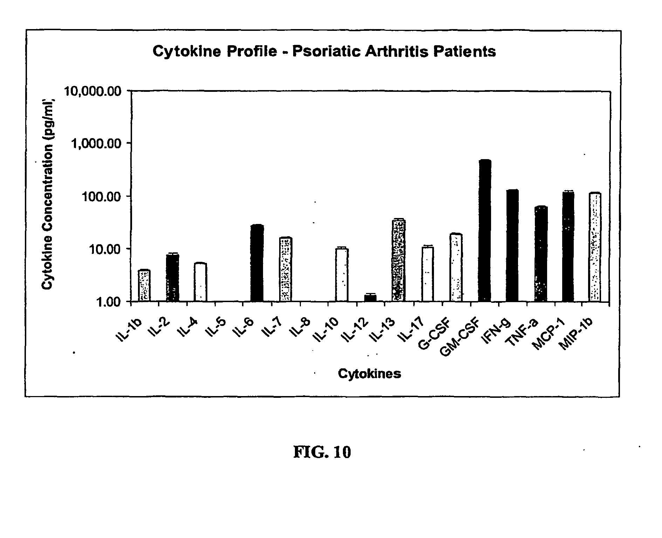

**FIG. 10** **-** Cytoldne Profile of Psoriatic Artritis Patients.

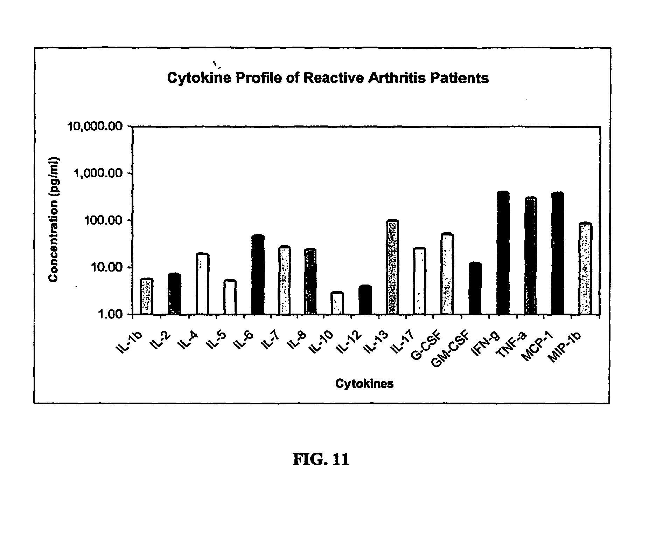

**FIG. 11** **-** Cytokine Profile of Reactive Arthritis Patients.

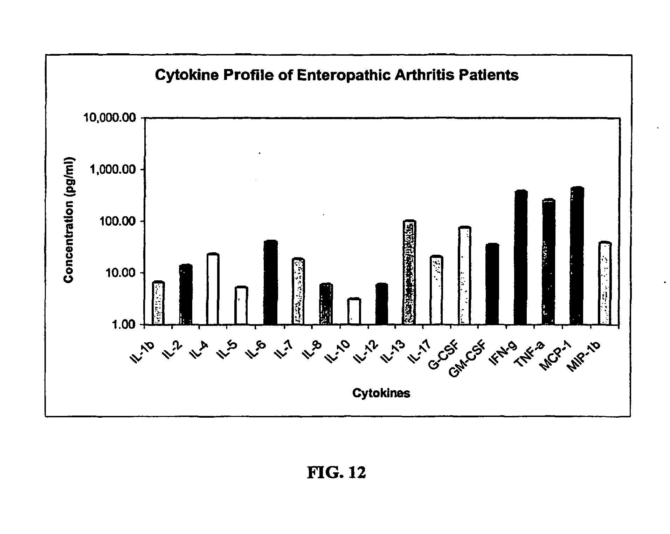

**FIG. 12** **-** Cytokine Profile of Enteropathic Arthritis Patients.

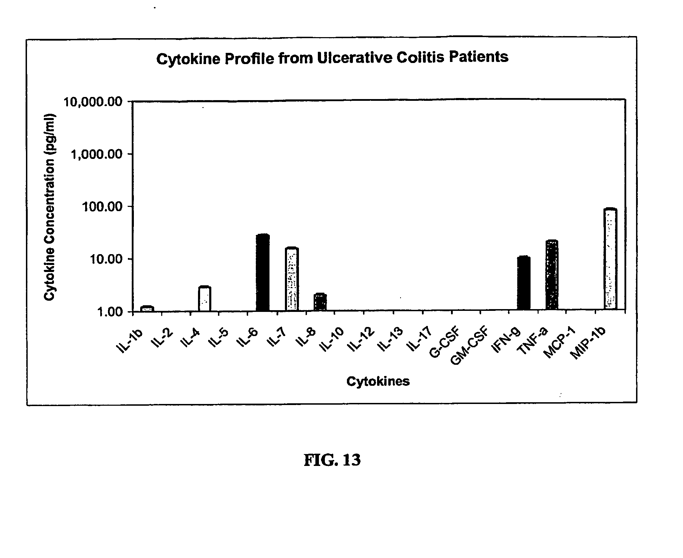

**FIG. 13** **-** Cytokine Profile of Ulcerative Colitis Patients.

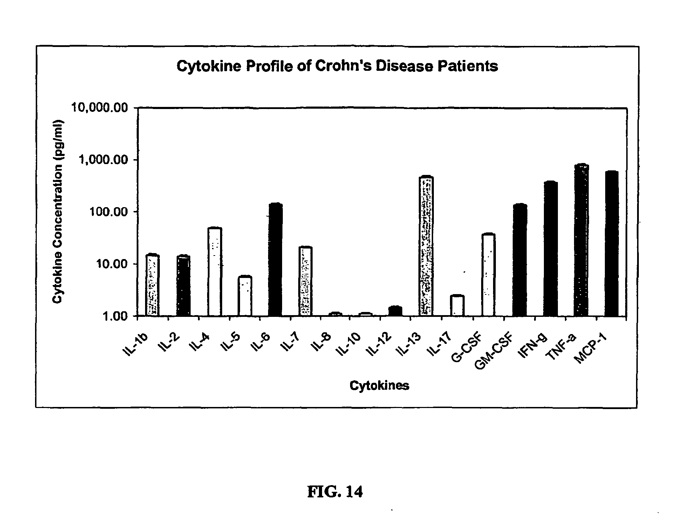

**FIG. 14** **-** Cytokine Profile of Crohn's Disease Patients.

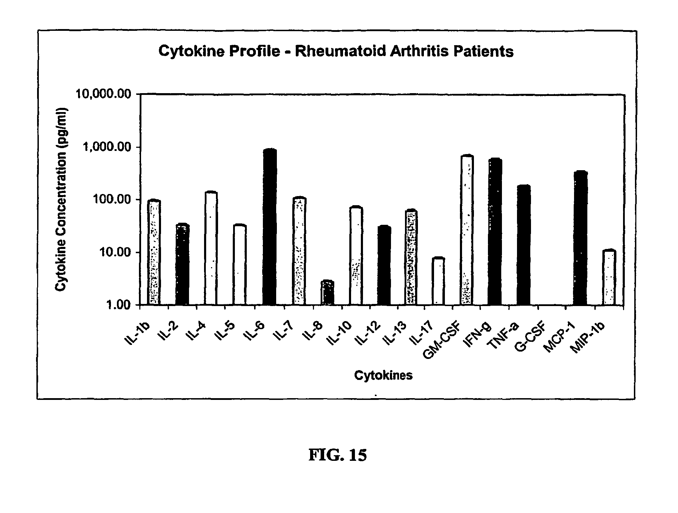

**FIG. 15** **-** Cytokine Profile of Rheumatoid Arthritis Patients.

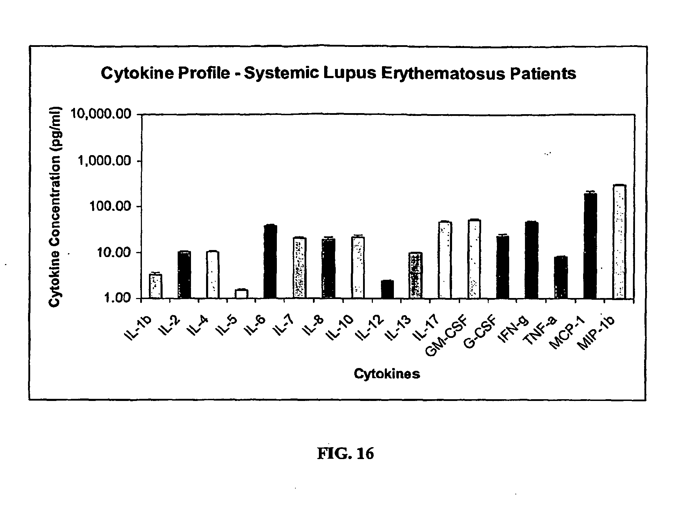

**FIG. 16** **-** Cytokine Profile of Systemic Lupus Erythematosus Patients.

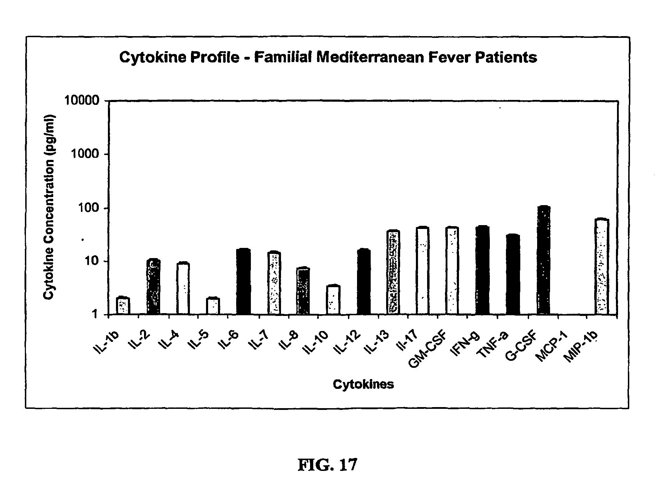

**FIG. 17** **-** Cytokine, Profile of Familial Mediterranean Fever Patients.

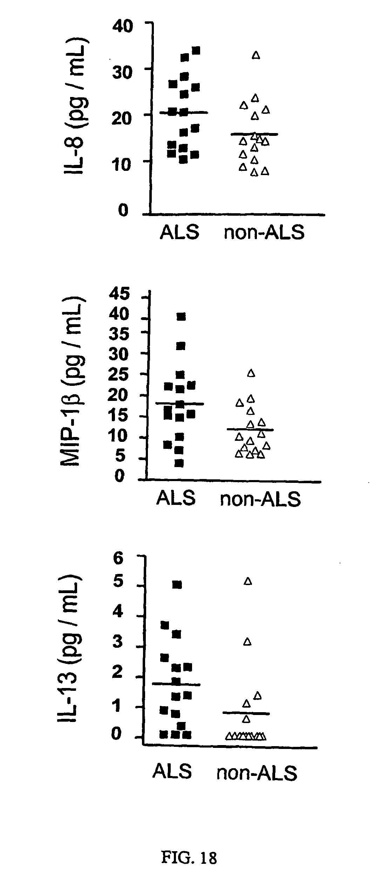

**FIG. 18** **-** Scatter plots of MIP-1β, IL-8 and IL-13 levels in CSF of ALS and non-ALS subjects.

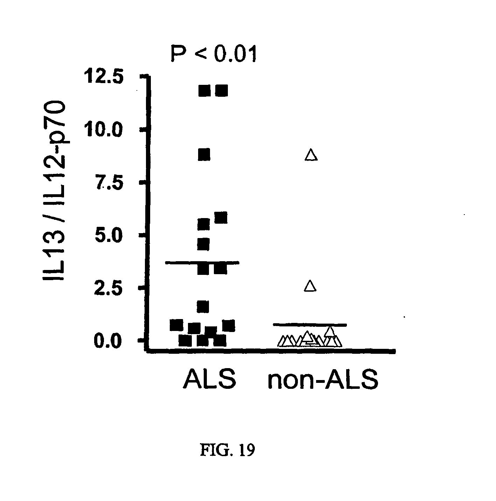

**FIG. 19** **-** Distribution of IL-13/IL-12 ratios in CSF from ALS and non-ALS subjects.

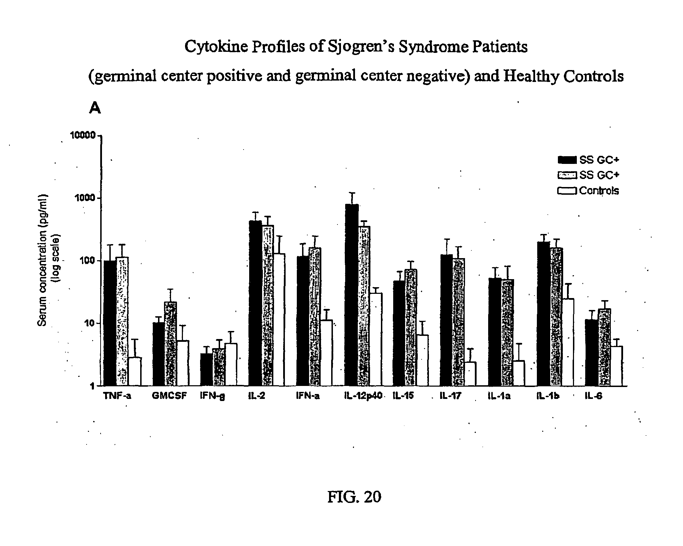

**FIG. 20** **-** Cytokine Profile of Sjogren's Syndrome Patients.

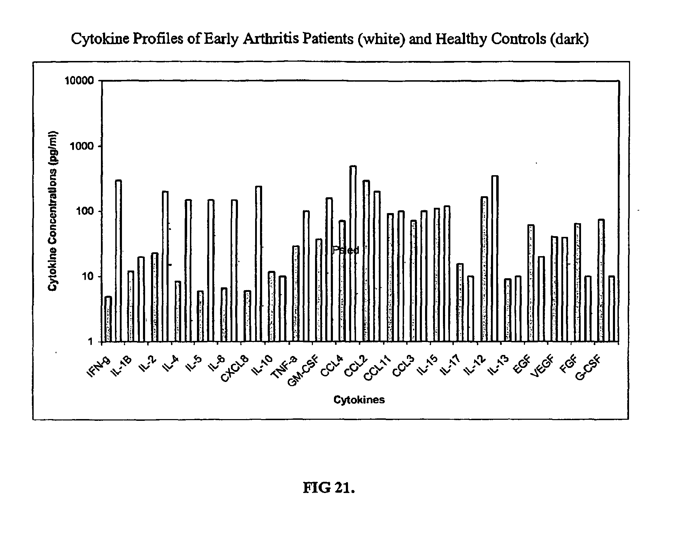

**FIG. 21** **-** Cytokine Profile of Early Arthritis Patients.

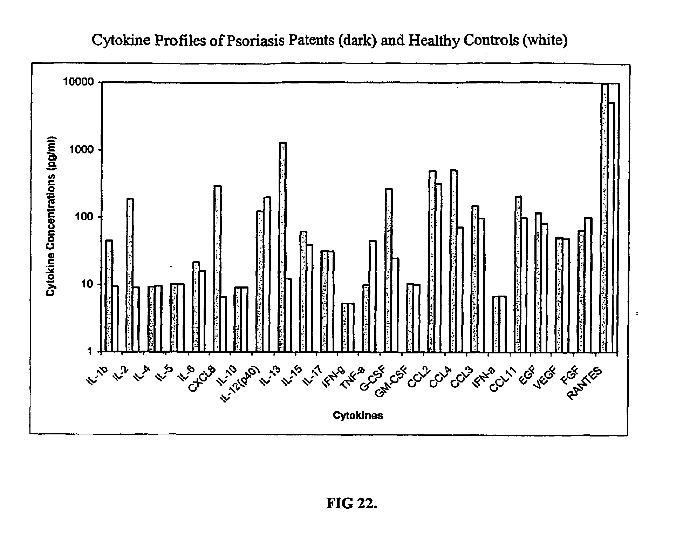

**FIG. 22** **-** Cytoldne Profile of Psoriasis Patients.

### DETAILED DESCRIPTION OF PREFERRED EMBODIMENTS

### I. Cytokine Involvement in Disease

Interleukin-1α (IL-1α) is expressed in large amounts by human keratinocytes. IL-1-α is also produced also by activated macrophages from different sources (alveolar macrophages, Kupffer cells, adherent spleen and peritoneal macrophages) and also by peripheral neutrophil granulocytes, endothelial cells, fibroblasts, smooth muscle cells, keratinocytes, Langerhans cells of the skin, osteoclasts, astrocytes, epithelial cells of the thymus and the cornea, T-cells, and B-cells.

The concentrations of IL-1 observed in the cerebrospinal fluid are due to local synthesis and also due to the direct transport of IL-1 through the blood-brain barrier by means of a saturable carrier system and the ability of activated T-lymphocytes to pass this barrier. Interleukin-1β (IL-1-β) is constitutively expressed in the brain.

IL-1α and IL-1β are biologically more or less equivalent pleiotropic factors that act locally and also systemically. Only a few functional differences between the factors have been described.

Some of the biological activities of IL-1 are mediated indirectly by the induction of the synthesis of other mediators including ACTH (Corticotropin), PGE2, PF4 (platelet factor-4), CSF (colony stimulating factors), IL-6, and CXCL8 (IL-8).

The synthesis of IL-1 can be induced by other cytokines including TNF-α, IFN-α IFN-γ, and IFN- β and also by bacterial endotoxins, viruses, mitogens, and antigens. In human skin fibroblasts, IL-1α and TNF-α induce the synthesis of IL-1β. Human mononuclear cells are very sensitive to bacterial endotoxins and synthesize IL-1 in response to picogram/mL amounts of endotoxins. In human monocytes bacterial lipopolysaccharides induce approximately tenfold more mRNA and the respective proteins for IL-1β than for IL-1α
The synthesis of IL-1 is controlled by a complex feedback loop because IL-1 is capable also of inhibiting or promoting its own synthesis, depending on conditions and cell types.

The main biological activity of IL-1 is the stimulation of T-helper cells, which are induced to secrete IL-2 and to express IL-2 receptors. Virus-infected macrophages produce large amounts of an IL-1 inhibitor (IL-1ra) that may support opportunistic infections and transformation of cells in patients with T-cell maturation defects.

IL-1 acts directly on B-cells, promoting their proliferation and the synthesis of immunoglobulins. IL-1 also functions as one of the priming factor that makes B-cells responsive to IL-5. IL-1 stimulates the proliferation and activation of NK-cells and fibroblasts, thymocytes, glioblastoma cells. It also promotes the proliferation of astroglia and microglia and may be involved in pathological processes such as astrogliosis and demyelination. The IL-1 mediated proliferation of lymphocytes is inhibited byTGF-β-1 and TGF-β-2.

A mechanism of autocrine growth control by IL-1 has been suggested for leukemic blast cells in which the uncontrolled synthesis of IL-1 is thought to lead to the production of colony stimulating factors (CSF) that in turn promote the proliferation of these cells. In combination with other cytokines, IL-1 appears to be an autocrine growth modulator for human gastric and thyroid carcinoma cells. The growth-promoting activities of IL-1 are mediated indirectly in some systems by regulating the expression of high affinity receptors for another cytokine, FGF. IL-1 has been shown also to be radioprotective.

IL-1 also has antiproliferative and cytocidal activities on certain tumor cell types. It supports the monocyte mediated tumor cytotoxicity and induces tumor regression. IL-1 is cytotoxic for insulin-producing beta cells of the Langerhans islets of the pancreas.
IL-1 inhibits the growth of endothelial cells *in vivo* and *in vitro.* IL-1 causes many alterations of endothelial functions *in vivo*. It promotes thrombotic processes and attenuates anticoagulatory mechanisms. IL-1 therefore plays an important role in pathological processes such as venous thrombosis, arteriosclerosis, vasculitis, and disseminated intravasal coagulation.

IL-1 promotes the adhesion of neutrophils, monocytes, T-cells, and B-cells by enhancing the expression of adhesion molecules such as CAM-1 (intercellular adhesion molecule) and ELAM (endothelial leukocyte adhesion molecule). The expression of membrane-associated thrombomodulin is decreased by IL-1.

IL-1 also influences the functional activities of Langerhans cells of the skin. These cells are not capable of eliciting primary immune responses (for example, contact sensibilisation). IL-1 (and also GM-CSF) convert these cells into potent immunostimulatory dendritic cells. The Langerhans cells therefore constitute an *in situ* reservoir for immunologically immature lymphoid dendritic cells. The increased ability of maturated Langerhans cell to process antigens is decreased by TNF-α

IL-1 in combination with other cytokines is an important mediator of inflammatory reactions. IL-1 markedly enhances the metabolism of arachidonic acid (in particular of prostacyclin and PGE2) in inflammatory cells such as fibroblasts, synovial cells, chondrocytes, endothelial cells, hepatocytes, and osteoclasts. In addition one observes an increased secretion of inflammatory proteins such as neutral proteases (collagenase, elastase and plasminogen activator). This activity of IL-1 antagonizes the effects of TGF-β on the extracellular matrix.

IL-1 is also a strong chemoattractant for leukocytes. In vivo the injection of IL-1 leads to the local accumulation of neutrophils at the site of injection. IL-1 also activates oxidative metabolism in neutrophils.

In combination with TNF, IL-1 appears to be involved in the generation of lytic bone lesions. IL-1 activates osteoclasts and therefore suppresses the formation of new bone. Low concentrations of TL-1, however, promote new bone growth: IL-1 inhibits the enzyme lipoprotein lipase in adipocytes. In vascular smooth muscle cells and skin fibroblasts IL-1 induces the synthesis of bFGF, which is a mitogen for these cells.

Like Interleukin-2 (IL-2), IL-1 also modulates the electrophysiological behavior of neurons. IL-1 also directly affects the central nervous system as an afferent signal modulating the release of a number of hormones and activates the hypothalamic-pituitary-adrenocortical (HPA) axis. IL-1 activates the serotoninergic system. IL-1 also functions as an endogenous pyrogen and induces a significant elevation of body temperature by causing the release of prostaglandins in the thermoregulatory center of the hypothalamus. This activity is inhibited by Alpha-Melanocyte stimulating hormone (alpha-MSH). IL-1, like IL6, stimulates the synthesis of ACTH (corticotropin) in the pituitary. Glucocorticosteroids on the other hand increase the expression of IL-1 receptors. In blood serum IL-1 has been shown to decrease plasma concentrations of iron and zinc. In Schwann cells and fibroblast-like cells of nervous tissue IL-1 induces the synthesis of NGF.

IL-1β immunoreactive nerve fibers in human hypothalamus innervate those endocrine and autonomous nuclei controlling central aspects of acute phase reaction. Astrocytes proliferate in the presence of IL-1 and release IL-3, which is a growth factor for microglia and peritoneal macrophages. In Astrocytes IL-1 also promotes the synthesis of GM-CSF, IL-6 and TNF. In the central nervous system IL-1 is involved also in the induction of the so-called slow-wave sleep. IL-1β can prevent the depression of antibody responses observed during sleep deprivation.

IL-1 synergises with some other factors. The effect of IL-1 on the proliferation of thymocytes involves the stimulation of IL-2 synthesis and the expression of IL-2 receptors on T-cells. For certain T-helper cell populations and also for B-cells IL-1 functions as an additional growth factor. IL-1 potentiates the effects of colony stimulating factors (CSFs) and promotes the generation of myeloid progenitor cells from stem cells. IL-1 synergises with GM-CSF in the induction of macrophage colony growth. IL-1 can also induce the synthesis of G-CSF and M-CSF by bone marrow stromal cells and stimulates the synthesis of GM-CSF and G-CSF by human skin cells and of GM-CSF by peripheral blood lymphocytes. By enhancing the expression of receptors for colony stimulating factors IL-1 is involved in various processes of hematopoiesis. IL-1 also induces the proliferation of pluripotent bone marrow progenitor cells.

Peter *et al.* (1991) examined interleukin-1β (IL-1β) and tumor necrosis factor (TNF) in cerebrospinal fluid (CSF) and serum of multiple sclerosis patients and normal controls. They concluded that the levels of these cytokines, in both fluids, were not of prognostic or diagnostic utility. Westacott *et al.* (1990) used immunoassays to measure cytokines in synovial fluid of patients with rheumatic disease. A factor with IL-1-like bioactivity was detected in the gingival fluid of clinically normal humans (Oppenheim *et al.,* 1982), the activity being higher in inflamed than non-inflamed gingival regions. The gingival fluid factor exhibited molecular weights corresponding to both IL-1 and epidermal thymocyte-activating factor (Charon *et al.,* 1982). Studies by Jandinski and colleagues (Jandinski *et al.,* 1988a; 1988b; 1988c) reported the presence of IL-1β in periodontal tissue, while IL-1 predominated in gingival crevicular fluid of patients with periodontal disease. Another study (Kabashimi *et al.,* 1990) utilizing polyclonal antisera to recombinant human IL-1α and IL-1β and measurement by Western blotting, disclosed that the majority of the IL-1 bioactivity found in gingival crevicular fluid of patients with chronic inflammatory periodontal disease was IL-1, generally considered the membrane-bound form of IL-1. The suggestion was made that the IL-1 was derived by enzymatic cleavage from the cell surface. In this latter study, special care was taken to avoid contamination of the gingival fluid with saliva. These facts argue strongly against a salivary origin for the gingival fluid IL-1.

Under physiological conditions, interleukin-2 (IL-2) is produced mainly by T-cells expressing the surface antigen CD4 following cell activation by mitogens or allogens. Resting cells do not produce IL-2 (Smith, 1988). Transformed T-cells and B-cells, leukemia cells, LAK cells (lymphocyte-activated killer cells) and NK-cells also secrete IL-2. IL-2 displays significant anti-tumor activity for a variety of tumor cell types because it supports the proliferation and clonal expansion of T-cells that specifically attack certain tumor types (Williams *et al.,* 1991). IL-2 is increasingly used to treat patients with cancers refractory to conventional treatment (Waldman *et al.,* 1993). Objective and long-lived clinical responses have been documented also in a proportion of patients with melanoma or acute myeloid leukemia (Broom *et al*., 1992).

Interleukin-4 (IL-4) is produced mainly by a subpopulation of activated T-cells (Th2), which are the biologically most active helper cells for B-cells and which also secrete IL-5 and IL-6. Another subpopulation of T-cells (Th1) also produces IL-4, but to a lesser extent. Non-T/Non-B-cells of the mast cell lineage also produce IL-4 (Boulay *et al.,* 1992). IL-4 promotes the proliferation and differentiation of activated B-cells, the expression of class II MHC antigens, and of low affinity IgE receptors in resting B-cells (Jansen *et al.,* 1990). IL-4 is thought to be an autocrine growth modulator for Hodgkin's lymphomas (Okabe *et al.,* 1992). IL-4 enhances expression of class II MHC antigens on B-cells. It can promote their capacity to respond to other B-cell stimuli and to present antigens for T-cells (Paul *et al.,* 1991). This may be one way to promote the clonal expansion of specific B-cells and the immune system may thus be able to respond to very low concentrations of antigens. The production of IL-4 by Non-B Non-T-cells is stimulated if these cells interact with other cells via their Fc receptors for IgE or IgG (Callard *et al*., 1991) This effect can be enhanced by IL-3. IL-4 also inhibits cell activation of NK-cells induced by IL-2 (Paul *et al.,* 1987).

IL-4 may be of clinical importance in the treatment of inflammatory diseases and autoimmune diseases since it inhibits the production of inflammatory cytokines such as IL-1, IL-6, and TNF-α by monocytes and T-cells (Dullens *et al.,* 1992). IL-4 may be useful also in the treatment of solid tumors, of hematopoietic systemic diseases, and of immune defects. IL4 inhibits the growth of colon and mammary carcinomas (Toi *et al.,* 1992). It has been shown to augment the development of lymphokine-activated killer cells (LAK cells). IL-4 may play an essential role in the pathogenesis of chronic lymphocytic leukemia disease, which is characterized by the accumulation of slow-dividing and long-lived monoclonal B-cells arrested at the intermediate stage of their differentiation by preventing both the death and the proliferation of the malignant B-cells. It protects chronic lymphocytic leukemic B-cells from death by apoptosis and upregulates the expression of a protective gene, BCL-2 (Dancescu *et al.,* 1992).

Interleukin-5 (IL-5) is produced by T-cells. IL-5 is a specific hematopoietic growth factor that is responsible for the growth and differentiation of eosinophils (Takatsu *et al.,* 1992). IL-5 strongly stimulates the proliferation, cell activation, and differentiation of eosinophilic granulocytes. B-cells can be made responsive to IL-5 by treatment with suboptimal doses of IL-1. IL-5 also promotes the generation of cytotoxic T-cells from thymocytes (Sanderson *et al.,* 1988). A possible clinical application is suggested by the activity of IL-5 on eosinophils. Animal experiments have shown that eosinophilia elicited by nematode infections in mice and the concomitant infiltration of the lung with eosinophils can be prevented by administration of monoclonal animals directed against IL-5 (Coffman *et al.,* 1989).

Many different cell types produce interleukin-6 (IL-6). The main sources *in vivo* are stimulated monocytes, fibroblasts, and endothelial cells. Macrophages, T-cells and B-lymphocytes, granulocytes, smooth muscle cells, eosinophils, chondrocytes, osteoblasts, mast cells, glial cells, and keratinocytes also produce IL-6 after stimulation (Akira *et al.,* 1990). Glioblastoma cells constitutively produce IL-6 and the factor can be detected also in the cerebrospinal fluid. Human milk also contains IL-6 (Bauer *et al.,* 1991).

The determination of IL-6 serum levels may be useful to monitor the activity of myelomas and to calculate tumor cell masses. Low IL-6 serum levels are observed in monoclonal gammopathies and in smoldering myelomas while IL-6 serum levels are markedly increased in patients with progressive disease and also in patients with plasma cell leukemia (Van Oers *et al.,* 1993). A blockade of the IL-6 receptor or the inhibition of IL-6 by monoclonal antibodies may be a way to delay or prevent the maturation of B-cells into plasma cells.

The deregulated expression of IL-6 is probably one of the major factor involved in the pathogenesis of a number of diseases (Leger-Ravet *et al.,* 1991). The excessive overproduction of IL-6 has been observed in various pathological conditions such as rheumatoid arthritis, multiple myeloma, Lennert syndrome (histiocytic lymphoma), Castleman's disease (lymphadenopathy with massive infiltration of plasma cells, hyper gamma-globulinemia, anemia, and enhanced concentrations of acute phase proteins), cardiac myxomas and liver cirrhosis (Hsu *et al.,* 1993). The constitutive synthesis of IL-6 by glioblastomas and the secretion of IL-6 into the cerebrospinal fluid may explain the elevated levels of acute phase proteins and immune complexes in the serum (Mule *et al.* 1991).

IL-6 probably also plays a role in the pathogenesis of chronic polyarthritis because excessive concentrations of IL-6 are found in the synovial fluid. It has been suggested that IL-6, due to its effects on hematopoietic cells, may be suitable for the treatment of certain types of anemia and thrombocytopenia (Brach *et al.,* 1992). Pretreatment with IL-3 and subsequent administration of IL-6 has been shown to increase platelet counts. In combination with other cytokines (for example, IL-2) IL-6 may be useful in the treatment of some tumor types (Dullens *et al,* 1991).

Very high levels of IL-6 in the cerebrospinal fluid are observed frequently in bacterial and viral meningitis. The detection of elevated concentrations of IL-6 in the urine of transplanted patients may be an early indicator of a graft-versus-host reaction (Kishimoto *et al.,* 1990). The detection of IL-6 in the amniotic fluid may be an indication of intra-amniotic infections. In inflammatory intestinal diseases elevated plasma levels of IL-6 may be an indicator of disease status (Wolvekamp *et al.,* 1990). In patients with mesangioproliferative glomerulonephritis elevated urine levels of IL-6 are also an indicator of disease status (Van Snick *et al.,* 1990). Monitoring of postoperative serum IL-6 levels may be more helpful than monitoring of C-reactive protein levels for estimation of inflammatory status and early detection of an acute phase reaction (Ohzato *et al.,* 1992). Serum and urinary IL-6 levels have been shown to be predicting factors of Kawasaki disease activity (Furukawa *et* al., 1992).

Interleukin-7 (IL-7) is secreted constitutively into the conditioned medium of adherent bone marrow stromal cells and thymic cells. Murine and human keratinocytes have been shown also to express and secrete IL-7. IL-7 may be of clinical significance for adoptive immunotherapy because it is capable *in vivo* to cause the CD4(+)T-cell-dependent destruction of tumor cells (Hock *et al.,* 1991). IL-7 has been shown also to induce LAK cells activity comparable quantitatively to that induced by IL-2 in cells obtained from patients early after autologous or syngeneic bone marrow transplantation (Pavletic *et al.,* 1993). It induces an even greater LAK cell activity *in vitro* in peripheral blood mononuclear cells obtained after autologous bone marrow transplantation and preactivated *in vivo* by IL-2 therapy (Stotter *et al.,* 1991). It has therefore been suggested that IL-7, alone or in combination with IL-2, may be used as a consolidative immunotherapy for malignancies in patients after autologous bone marrow transplantation.

Participation of IL-7 in the pathogenesis of inflammatory skin diseases and cutaneous T-cell lymphomas is suggested by the growth-promoting effects of IL-7 and its synthesis by keratinocytes. IL-7 may contribute to disturbances of immune regulatory T-cells in ulcerative colitis because a serum factor from patients with ulcerative colitis that induces proliferation of intrathymic T-cells has been found to be identical with IL-7 (Watanabe *et al.,* 1997).

Interleukin-8 (IL-8), now called CXCL8, is produced by stimulated monocytes but not by tissue macrophages and T-lymphocytes. CXCL8 is produced also by macrophages, fibroblasts, endothelial cells, keratinocytes, melanocytes, hepatocytes, chondrocytes, and a number of tumor cell line (Koch *et al.,* 1991). In many cell types the synthesis of CXCL8 is strongly stimulated by IL-1 and TNF-α. The synthesis of CXCL8 is induced also by phytohemagglutinins, concanavalin A, double-stranded RNA, phrobol esters, sodium urate crystals, viruses, and bacterial lipopolysaccharides (Zwahlen *et al.,* 1993). The expression of CXCL8 from resting and stimulated human blood monocytes is up-regulated by IL-7. In chondrocytes the synthesis of CXCL8 is stimulated by IL-1β, TNF-α, and bacterial lipopolysaccharides. In human astrocytes, the synthesis and secretion of CXCL8 is induced by IL-1 and TNF-α. CXCL8 is constitutively and commonly produced by various carcinoma cell lines (Matsushima *et al.,* 1991). In epithelial, endothelial, and fibroblastic cells secretion of CXCL8 is induced by IL-17 (Baggiolini *et al.,* 1994).

CXCL8 may be of clinical relevance in psoriasis and rheumatoid arthritis. Elevated concentrations are observed in psoriatic scales and this may explain the high proliferation rate observed in these cells (Gillitzer *et al.,* 1991). CXCL8 may be also a marker of different inflammatory processes. CXCL8 probably plays a role in the pathogenesis of chronic polyarthritis because excessive amounts of this factor are found in synovial fluids (Peichl *et al.,* 1991). The activation of neutrophils may enhance the migration of cells into the capillaries of the joints. These cells are thought to pass through the capillaries and enter the surrounding tissues thus causing a constant stream of inflammatory cells through the joints.

In humans, interleukin-10 (IL-10) is produced by activated CD8(+) peripheral blood T-cells, by T-helper CD4(+) T-cell clones (resembling Th0, Th1, and Th2) after both antigen-specific and polyclonal activation, by B-cell lymphomas, and by monocytes following cell activation by bacterial lipopolysaccharides and mast cells (Howard *et al.*, 1992). B-cell lines derived from patients with acquired immunodeficiency syndrome and Burkitt's lymphoma constitutively secrete large quantities of IL-10 into the conditioned medium (de Waal-Malefyt *et al.,* 1992). The synthesis of IL-10 by monocytes is inhibited by IL-4 and IL-10 (Zlotnik *et al.,* 1991). IL-10 has been detected in the sera of a subgroup of patients with active non-Hodgkin's lymphoma. IL-10 levels appear to correlate with a poor survival in patients with intermediate or high-grade non-Hodgkin's lymphoma (Blay *et al.,* 1993).

Interleukin-12 (IL-12) is secreted by peripheral lymphocytes after induction (Trinchieri *et al*., 1992). It is produced mainly by B-cells and to a lesser extent by T-cells (Trinchieri *et al.,* 1993). The most powerful inducers of IL-12 are bacteria, bacterial products, and parasites. IL-12 is produced after stimulation with phorbol esters or calcium ionophores by human B-lymphoblastoid cells. IL-12 may be useful in expanding an antigen-specific T-cell population. The culture of cytotoxic T-lymphocytes with IL-12 and low-dose IL-2 leads to proliferation only in response to an antigen co-signal (Chehimi *et al.,* 1993). IL-12 has been shown to augment natural killer-cell mediated cytotoxicity in a number of conditions, including patients with hairy cell leukemia (Bigda *et al*., 1993).

Interleukin-13 (IL-13) down-modulates macrophage activity, reducing the production of pro-inflammatory cytokines and chemokines in response to IFN-γ or bacterial lypopolysaccharides (McKenzie *et al.,* 1993). IL-13 enhances the production of the IL-1 receptor antagonist (IL-1ra) (Minty *et al.,* 1993). IL-13 also decreases the production of nitric oxide by activated macrophages, leading to a decrease in parasiticidal activity. IL-13 induces human monocyte differentiation, enhances survival time in culture, and also induces B-cell differentiation and proliferation and isotype switching (Herbert *et al.,* 1993). It induces IL-4 independent IgG4 and IgE synthesis in human B-cells and germ-line IgE heavy chain gene transcription (Punnonen *et al.,* 1993). IL-13 induces considerable levels of IgM and IgG, but no IgA, in cultures of highly purified surface IgD-positive or total B-cells in the presence of an activated CD4(+) T-cell clone. IL-13 induces proliferation and differentiation of human B-cells activated by the CD40 ligand (Cocks *et al.,* 1993). IL-13 synergizes with IL-2 in regulating IFN-γ synthesis in large granular lymphocytes. IL-13 has been shown to inhibit strongly tissue factor expression induced by bacterial lipopolysaccharides and to reduce the pyrogenic effects of IL-1 or TNF, thus protecting endothelial and monocyte surfaces against inflammatory mediator induced procoagulant changes.

IL-13 inhibits human immunodeficiency virus type-1 production in primary blood-derived human macrophages in *vitro.* Human basophils produce IL-13 in response to IgE-receptor (IgER) crosslinking, IL-3 , IL-3 plus C5a, but not C5a alone (Ochensberger *et al.,* 1996). IL-13, like IL-4, induces CD23 expression on B-cells and enhances CD72 and class II major histocompatibility complex antigen expression. IL-13 has been observed to increase the killer activity of LAK cells induced by IL-2 (Minty *et al*., 1993).

Interleukin-15 (IL-15) has been shown to be produced by human fetal astrocytes and microglia in response to IL-1β, IFN-γ, and TNF-α and is thought to play a role in T-cell mediated immune responses in the human central nervous system. Some of the biological activities of IL-15 resemble those of IL-2. IL-15 stimulates proliferation of T-cells. In addition, IL-15 is also able to induce generation of cytolytic cells and (lymphokine activated killer) LAK cells. IL-15 appears to function as a specific maturation factor for NK-cells. It is also been shown to function as an NK-cell survival factor *in vivo.* High affinity IL-15 binding has been observed on many lymphoid cell types, including peripheral blood monocytes, and NK-cells.

IL-15 induces mast cell proliferation in the absence of functional IL-2 receptor components. The mast cell IL-15 receptor recruits JAK2 and STATS instead of JAK1, JAK3, STAT3, and STAT5 that are activated in Tcells. IL-15 inhibits apoptosis induced by deprivation of cytokines in activated T-cells.

Interleukin-17 (IL-17) functions as a mediator of angiogenesis that stimulates vascular endothelial cell migration and cord formation and regulates production of a variety of growth factors promoting angiogenesis (Numasaki *et al.,* 2002). IL-17 binds to a receptor that binds to HVS13 (referred to as viral IL-17) and to CTLA-8 (Yao *et al.,* 1995).

Interleukin-18 (IL-18) encodes an inducer of IFN-γ production by T-cells (Okamura *et al.,* 1995; Micallef *et al.,* 1996) and natural killer cells (Tsutsui *et al.,* 1996) that is a more potent inducer than IL-12 (Kikkawa *et al.,* 2001). have demonstrated that monocytes and macrophages produce large amounts of various IL-18 species.

IL-18 is produced during the acute immune response by macrophages and immature dendritic cells. IL-18 is expressed by a variety of immune and non-immune cells, including monocytes and macrophages (Okamura *et al.,* 1995; Ushio *et al.,* 1996), Kupffer cells (Okamura *et al.,* 1995; Seki *et al.,* 2001), T-cells and B-cells (Nakanishi *et al.,* 2001; Klein *et al.,* 1999), dendritic cells (Stober *et al.,* 2001; Gardella *et al.* 2000; Stoll *et al.,* 1998; de Saint-Vis *et al.,* 1998), osteoblasts (Udagawa *et al.,* 1997); Torigoe *et al.,* 1997), epidermal keratinocytes (Stoll *et al.,* 1998), intestinal epithelial cells (Takeuchi *et al.,* 1997; Pizarro *et al.,* 1999), corneal epithelial cells (Burbach *et al.,* 2001), glucocorticoid-secreting adrenal cortex cells (Conti *et al.,* 1997), astrocytes, and microglia (Conti *et al.,* 1998; Suk *et al.,* 2001).

IL-18 is considered one of the pro-inflammatory cytokines. An important function of IL-18 is the regulation of functionally distinct subsets of T-helper cells required for cell mediated immune responses (Nakanishi *et al.,* 2001). IL-18 functions as a growth and differentiation factor for Th1 cells.

IL-18 is a pleiotropic cytokine. IL-18 induces activated B-cells to produce IFN-γ that inhibits IgE production (Yoshimoto *et al.,* 1997). IL-18 has been shown to strongly augment the production of IFN-γ by T-cells and NK-cells (Micallef *et al.,* 1996). The ability of IL-18 to enhance IFN-γ production by NK cells is dependent on the presence of IL-12 (Walker *et al.,* 1999). IL-18 has also been found to enhance also the production of GM-CSF (Udagawa *et al*., 1997) have shown that IL-18 produced by osteoblastic stromal cells acts via GM-CSF and not via IFN-γ to inhibit osteoclast formation.

Morel *et al.* (2001) have observed that IL-18 induces the expression of CXCL8 (IL-8), MGSA, and ENA-78 in rheumatoid arthritis synovial fibroblast. IL-18 inhibits osteoclast formation via T-cell production of GM-CSF (Udagawa *et al*., 1997; Horwood *et al*., (1998)).

Granulocyte-Colony Stimulating Factor (G-CSF) is secreted by monocytes, macrophages and neutrophils after cell activation (Demetri *et al.,* 1991). It is produced also by stromal cells, fibroblasts, and endothelial cells. Epithelial carcinomas, acute myeloid leukemia cells and various tumor cell lines (bladder carcinomas, medulloblastomas), also express this factor. The synthesis of G-CSF can be induced by bacterial endotoxins, TNF, IL-1, and GM-CSF. Prostaglandin E2 inhibits the synthesis of G-CSF. In epithelial, endothelial, and fibroblastic cells secretion of G-CSF is induced by IL-17 (Moore *et al*., 1991).

G-CSF can be used to expand the myeloid cell lineage (Gabrilove *et al.,* 1992). It has been shown that the pretreatment with recombinant human G-CSF prior to marrow harvest can improve the graft by increasing the total number of myeloid lineage restricted progenitor cells, resulting in stable but not accelerated myeloid engraftment of autologous marrow (Lieschke *et al.,* 1992). One general effect of treatment with G-CSF appears to be a marked reduction of severe infections and episodes of fever, which are normally observed to occur in patients with Kostmann syndrome (Jakubowski *et al.,* 1989). G-CSF treatment also allows dose intensification with various antitumor drug regimes (Gianni *et al.,* 1992).

Granulocyte-Monocyte-Colony Stimulating Factor (GM-CSF) is secreted together with other factors by T-cells and macrophages following cell activation by antigens or mitogens (Moore *et al.,* 1991). Approximately 90 percent of the secreted colony stimulating activities are due GM-CSF (Ruef *et al.,* 1990). The synthesis of GM-CSF by various other cell types, for example, endothelial cells and fibroblasts, is inducible by TNF-α, TNF-β, IL-1, IL-2, and IFN. Some cell types express GM-CSF constitutively (Freund *et al.,* 1992). GM-CSF can be employed for the physiological reconstitution of hematopoiesis in all diseases characterized either by an aberrant maturation of blood cells or by a reduced production of leukocytes. GM-CSF can be used also to correct chemotherapy induced cytopenias and to counteract cytopenia-related predisposition to infections and hemorrhages (Fan *et al.,* 1991). Several studies have demonstrated that the use of GM-CSF enhances tolerance to cytotoxic drug treatment and can be used to prevent dose reductions necessitated by the side effects of cytotoxic drug treatment (Negrin *et al.,* 1992). GM-CSF treatment frequently permits to increase the doses of cytotoxic drugs per course. At present, GM-CSF represents an important advance in bone marrow transplantation and has become a standard therapy (Armitage *et al.,* 1992). GM-CSF enhances the reconstitution of the hematopoietic system in patients undergoing autologous or allogenic bone marrow transplantation and patients with delayed engraftment after bone marrow transplantation (Schuster *et al.,* 1992).

Interferon-α (IFN-α) forms are produced by monocytes/macrophages, lymphoblastoid cells, fibroblasts, and a number of different cell types following induction by viruses, nucleic acids, glucocorticoid hormones, and low-molecular weight substances (n-butyrate, 5-bromodeoxy uridine). All known subtypes of IFN-α show the same antiviral antiparasitic, antiproliferative activities in suitable bioassays although they may differ in relative activities. IFN-α inhibits the expression of a number of cytokines in hematopoietic progenitor cells, which in turn induce a state of competence in these cells allowing them to pass from the G0 into the S-phase of the cell cycle.

The growth of some tumor cell types in vitro is inhibited by IFN-α which may simulate also the synthesis of tumor-associated cell surface antigens. In renal carcinomas IFN-α reduces the expression of receptors for EGF. IFN-α also inhibits the growth of fibroblasts and monocytes in vitro. IFN-α also inhibits the proliferation of B-cell in vitro and blocks the synthesis of antibodies. IFN-α also selectively blocks the expression of some mitochondrial genes.

Interferon-β (IFN-β) is produced mainly by fibroblasts and some epithelial cell types. The synthesis of IFN-beta can be induced by common inducers of interferons, including viruses, double-stranded RNA, and micro-organisms. It is induced also by some cytokines such as TNF and IL1. IFN-β is involved in the regulation of unspecific humoral immune responses and immune responses against viral infections. IFN-β increases the expression of HLA class I antigens and blocks the expression of HLA class II antigens stimulated by IFN-γ. IFN-beta stimulates the activity of NK-cells and hence antibody-dependent cytotoxicity. The activity of T suppressor cells elicited by several stimuli is stimulated also by IFN-β. IFN-β enhances the synthesis of the low affinity IgE receptor CD23. In activated monocytes IFN-β induces the synthesis of neopterin. It also enhances serum concentrations of β2-microglobulin. IFN-β selectively inhibits the expression of some mitochondrial genes.

Interferon-γ (IFN-γ) is produced mainly by T-cells and natural killer cells activated by antigens, mitogens, or alloantigens (De Maeyer *et al.,* 1992). It is produced by lymphocytes expressing the surface antigens CD4 and CD8 (Gray *et al.,* 1987). Like the other interferons, IFN-γ can be used as an antiviral and antiparasitic agent (Stuart-Harris *et al.,* 1992). IFN-γ has been shown to be effective in the treatment of chronic polyarthritis (Machold *et al.,* 1992). This treatment, which probably involves a modulation of macrophage activities, significantly reduces joint aches and improves various clinical parameters and allows reduction of corticosteroid doses. IFN-γ may be of value in the treatment of opportunistic infections in AIDS patients. It has been shown also to reduce inflammation, clinical symptoms, and eosinophilia in severe atopic dermatitis (Hanifin *et al.,* 1993).

Tumor necrosis factor-α. (TNF-α.) is secreted by macrophages, monocytes, neutrophils, T-cells, NK-cells following their stimulation by bacterial lipopolysaccharides (Beutler *et al.,* 1988). Cells expressing CD4 secrete TNF-α while CD8(+) cells secrete little or no TNF-α. Stimulated peripheral neutrophilic granulocytes but also unstimulated cells and also a number of transformed cell lines, astrocytes, microglial, smooth muscle cells, and fibroblasts also secrete TNF-α. The synthesis of TNF-α is induced by many different stimuli including interferons, IL-2, GM-CSF, substance P, bradykinin, immune complexes, inhibitors of cyclooxygenase and platelet activating factor (PAF) (Strieter *et al.,* 1993).

In contrast to chemotherapeutic drugs TNF-α specifically attacks malignant cells. Extensive preclinical studies have documented a direct cytostatic and cytotoxic effect of TNF-α against subcutaneous human xenografts and lymph node metastases in nude (immunodeficient) mice, as well as a variety of immunomodulatory effects on various immune effector cells, including neutrophils, macrophages, and T-cells (Gifford *et al.,* 1991).

There are some indications that inhibitors of TNF-α may be of advantage. Because TNF-α is found in the synovial fluid of patients suffering from arthritis, these inhibitors may be helpful in ameliorating the disease and this has been shown to be the case in animal models of severe collagen induced arthritis (Williams *et al.,* 1992) and in Crohn's disease (Derkx *et al.,* 1993). Inhibitors may ameliorate also the severe consequences of systemic inflammatory response syndrome. TNF-α appears to be an important autocrine modulator promoting the survival of hairy cell leukemia cells (Lindemann *et al.,* 1989). TNF-α has been shown also to protect hematopoietic progenitors against irradiation and cytotoxic agents, suggesting that it may have some potential therapeutic applications in aplasia induced by chemotherapy or bone marrow transplantation (Hersh *et al.,* 1991).

Macrophage Chemotactic Protein-1 (MCP-1), now called CCL2, belongs to the family of chemotactic cytokines known as chemokines. CCL2 is chemotactic for monocytes but not neutrophils (Leonard *et al.,* 1990). Maximal induction of migration is observed at a concentration of 10 ng/ml (Leonard *et al.,* 1991). Point mutations have been described at two amino acid positions, which alter the factor so that it is then also chemotactic for neutrophils (Beall *et al.,* 1992). Elevated levels of CCL2 are observed in macrophage-rich atherosclerotic plaques (Ylä-Herttuala *et al.,* 1991). The factor activates the tumoricidal activity of monocytes and macrophages *in vivo*. It regulates the expression of cell surface antigens (CD11c, CD11b) and the expression of cytokines IL-1 and IL-6 (Jiang *et al.,* 1992). CCL2 is a potent activator of human basophils, inducing the degranulation and the release of histamines (Bischoff *et al.,* 1992). In basophils activated by IL-3, IL-5, or GM-CSF, CCL2 enhances the synthesis of leukotriene C4 (Bischoff *et al.,* 1993).

IL-1, TNF-α, PDGF, TGF-β, and LIF induce the synthesis of CCL2 in human articular chondrocytes, which may play a role in the initiation and progression of degenerative and inflammatory arthropathies by promoting monocyte influx and activation in synovial joints (Villiger *et al.,* 1992). CCL2 has been shown to exhibit biological activities other than chemotaxis. It can induce the proliferation and activation of killer cells known as CHALK (CC-Chemokine activated killer), which are similar to cells activated by IL-2 (LAK cells) (Hora *et al.,* 1992). CCL2 is also one of the strongest histamine inducing factors.

Macrophage inflammatory protein 1-α (MIP-1α), now called CCL3, belong to the family of chemotactic cytokines known as chemokines. CCL3 is one of the major factors produced by macrophages following their stimulation with bacterial endotoxins. Both proteins are involved in the cell activation of human granulocytes (neutrophils, eosinophils, and basophils) and appear to be involved in acute neutrophilic inflammation. CCL3 stimulates the production of reactive oxygen species in neutrophils and the release of lysosomal enzymes. It also induces the synthesis of other pro-inflammatory cytokines such as IL-1, IL-6, and TNF in fibroblasts and macrophages. CCL3 is a potent basophil agonist, inducing a rapid change of cytosolic free calcium, the release of histamine and sulfido-leukotrienes, and chemotaxis.

CCL3 enhances the activities of GM-CSF and promote the growth of more mature hematopoietic progenitor cells. CCL3 also acts as an inhibitor of the proliferation of immature hematopoietic stem cells and has therefore been called stem cell inhibitor. CCL3 also exhibits biological activities other than chemotaxis. It can induce the proliferation and activation of killer cells known as CC-chemokine-activated killer (CHAK) cells, which are similar to cells activated by IL-2, lymphokine-activated killer (LAK) cells.

Macrophage inflammatory protein-1β (MIP-1β), now called CCL4, is called also endogenous pyrogen. CCL4 is one of the major factors produced by macrophages following their stimulation with bacterial endotoxins. CCL4 is involved in the cell activation of human granulocytes (neutrophils, eosinophils, and basophils) and appears to be involved in acute neutrophilic inflammation. CCL4 stimulates the production of reactive oxygen species in neutrophils and the release of lysosomal enzymes. It also induces the synthesis of other pro-inflammatory cytokines such as IL-1, IL-6, and TNF-α in fibroblasts and macrophages. CCL4 antagonizes the inductive effects of CCL3. In human monocytes the production of CCL4 can be induced by bacterial lipopolysaccharides and IL-7 (Lord *et al*., 1992).

CCL4 is most effective at augmenting adhesion of CD8(+) T-cells to the vascular cell adhesion molecule VCAM-1 and it does so by being present on the surface of endothelial cells complexed with endothelial proteoglycans (Cocchi *et al.,* 1995). CCL4 also synergises with hematopoietic growth factors (hematopoietins). CCL4 enhances the activities of GM-CSF and promotes the growth of more mature hematopoietic progenitor cells. CCL4 has been shown to exhibit biological activities other than chemotaxis. It can induce the proliferation and activation of killer cells known as CHAK (CC-Chemokine activated killer) cells, which are similar to cells activated by IL-2 (LAK cells).

RANTES (regulated upon activation, normal T-cell expressed, and presumably secreted) is now called CCL5 (CCL5). CCL5 belongs to the family of chemotactic cytokines known as chemokines. CCL5 is expressed by an early response gene. Synthesis of CCL5 is induced by TNF-α and IL-1α.

The expression of CCL5 is inhibited following stimulation of T-lymphocytes. CCL5 is chemotactic for T-cells, human eosinophils and basophils and plays an active role in recruiting leukocytes into inflammatory sites. CCL5 also activates eosinophils to release, for example, eosinophilic cationic protein. It changes the density of eosinophils and makes them hypodense, which is thought to represent a state of generalized cell activation and is associated most often with diseases such as asthma and allergic rhinitis. CCL5 also is a potent eosinophil-specific activator of oxidative metabolism.

CCL5 increases the adherence of monocytes to endothelial cells. It selectively supports the migration of monocytes and T-lymphocytes expressing the cell surface markers CD4 and UCHL1. These cells are thought to be pre-stimulated T-helper cells with memory T-cell functions. CCL5 can induce the proliferation and activation of killer cells known as chemokine-activated killer (CHAK) cells. CCL5 is expressed by human synovial fibroblasts and may participate, therefore, in the ongoing inflammatory process in rheumatoid arthritis.

Variable endothelial growth factor (VEGF) is a highly specific mitogen for vascular endothelial cells. VEGF does not appear to enhance the proliferation of other cell types. VEGF significantly influence vascular permeability and is a strong angiogenic protein in several bioassays and is thought to plays a role in neovascularisation under physiological conditions. A potent synergism between VEGF and fibroblast growth factor basic (bFGF) in the induction of angiogenesis has been observed. VEGF is released from smooth muscle cells and macrophages and is thought to play a role in the development of arteriosclerotic diseases. In endothelial cells VEGF induces the synthesis of von Willebrand factor. It is also a potent chemoattractant for monocytes and thus has procoagulatory activities. In microvascular endothelial cells VEGF induces the synthesis of plasminogen activator and plasminogen activator inhibitor type-1. VEGF also induces the synthesis of the metalloproteinase, interstitial collagenase, which degrades interstitial collagen type 1, collagen type 2, and collagen type 3 under normal physiological conditions.

VEGF is important in the pathophysiology of neuronal and other tumors, probably functioning as a potent promoter of angiogenesis for human gliomas. Its synthesis is induced also by hypoxia. The extravasation of cells observed as a response to VEGF may be an important factor determining the colonization of distant sites. Due to its influences on vascular permeability VEGF may be involved also in altering blood-brain-barrier functions under normal and pathological conditions.

Epidermal growth factor (EGF) has been shown to inhibit the secretion of gastric acids. It also modulates the synthesis of a number of hormones, including the secretion of prolactin. In the central nervous system EGF influences the activity of some types of GABAergic and dopaminergic neurons. EGF is a strong mitogen for many cells or ectodermal, mesodermal, and endodermal origin. EGF controls and stimulates the proliferation of epidermal and epithelial cells, including fibroblasts, kidney epithelial cells, human glial cells, ovary granulosa cells, and thyroid cells. EGF acts as a differentiation factor for some cell types. It strongly influences the synthesis and turn-over of proteins of the extracellular matrix, including fibronectin, collagens, laminin, and glycosaminoglycans. EGF increases the release of calcium from bone tissue and, like TGF-α, thus promotes bone resorption. To a limited extent EGF also augments angiogenesis because it is mitogenic for endothelial cells. The mitogenic activity of EGF for endothelial cells can be potentiated by thrombin.

EGF is a strong chemoattractant for fibroblasts and epithelial cells. EGF alone and also in combination with other cytokines is an important factor mediating wound healing processes. EGF may be a trophic substance for the gastrointestinal mucosa and may play a gastroprotective role due to its ability to stimulate the proliferation of mucosa cells. EGF has been shown to effectively promote healing of ulcers at concentrations that do not inhibit the synthesis of gastric acids.

Fibroblast growth factors (FGF) constitutes a family of related 16-18 kDa proteins controlling normal growth and differentiation of mesenchymal, epithelial, and neuroectodermal cell types. FGF-2 (formerly known as basic FGF or bFGF) is the prototype of the FGF family.

Eotaxin (CCL11) is a chemokine that is a potent stimulator of eosinophils in vitro. CCL11 does not possess suppressive activity against immature subsets of myeloid progenitors stimulated to proliferate by multiple growth factors (Broxmeyer *et al.,* [INSERT]). Bartels *et al.* ([INSERT]), have demonstrated the presence of CCL11 sequence variants and of low constitutive CCL11 mRNA expression in human dermal fibroblasts, which is upregulated by IL-1α or TNF-α within 6 hrs and modulated by IFN-γ. Induction by IL-1α is transient while long-term stimulation with TNF-α results in a further increase of CCL11 mRNA.

### II. Disease States

### A. Ankylosing Spondylitis

AS is a disease subset within a broader disease classification of spondyloarthropathy. Patients affected with the various subsets of spondyloarthropathy have disease etiologies that are often very different, ranging from bacterial infections to inheritance. Yet, in all subgroups, the end result of the disease process is axial arthritis. Despite the early clinically differences seen in the various patient populations, many of them end up nearly identical after a disease course of ten-to-twenty years. Recent studies suggest the mean time to clinical diagnosis of ankylosing spondylitis from disease onset of disease is 7.5 years (Khan, 1998). These same studies suggest that the spondyloarthropathies may have prevalence close to that of rheumatoid arthritis (Feldtkeller *et al.,* 2003; Doran *et al.,* 2003).

AS is a chronic systemic inflammatory rheumatic disorder of the axial skeleton with or without extraskeletal manifestations. Sacroiliac joints and the spine are primarily affected, but hip and shoulder joints, and less commonly peripheral joints or certain extra-articular structures such as the eye, vasculature, nervous system, and gastrointestinal system may also be involved. Its etiology is not yet fully understood (Wordsworth, 1995; Calin and Taurog, 1998). It is strongly associated with the major histocompatibility class I (MHC I) HLA-B27 allele (Calin and Taurog, 1998). AS affects individuals in the prime of their life and is feared because of its potential to cause chronic pain and irreversible damage of tendons, ligaments, joints, and bones (Brewerton *et al.,* 1973; Brewerton *et al.,* 1973; Schlosstein *et al.,* 1973). AS may occur alone or in association with another form of spondyloarthropathy such as reactive arthritis, psoriasis, psoriatic arthritis, enthesitis, ulcerative colitis, irritable bowel disease, or Crohn's disease, in which case it is classified as secondary AS.

Typically, the affected sites include the discovertebral, apophyseal, costovertebral, and costotransverse joints of the spine, and the paravertebral ligamentous structures. Inflammation of the entheses, which are sites of musculotendinous and ligamentous attachment to bones, is also prominent in this disease (Calin and Taurog, 1998). The site of enthesitis is known to be infiltrated by plasma cells, lymphocytes, and polymorphonuclear cells. The inflammatory process frequently results in gradual fibrous and bony ankylosis, (Ball, 1971; Khan, 1990).

Delayed diagnosis is common because symptoms are often attributed to more common back problems. A dramatic loss of flexibility in the lumbar spine is an early sign of AS. Other common symptoms include chronic pain and stiffness in the lower back which usually starts where the lower spine is joined to the pelvis, or hip.

Although most symptoms begin in the lumbar and sacroiliac areas, they may involve the neck and upper back as well. Arthritis may also occur in the shoulder, hips and feet. Some patients have eye inflammation, and more severe cases must be observed for heart valve involvement.

The most frequent presentation is back pain, but disease can begin atypically in peripheral joints, especially in children and women, and rarely with acute iritis (anterior uveitis). Additional early symptoms and signs are diminished chest expansion from diffuse costovertebral involvement, low-grade fever, fatigue, anorexia, weight loss, and anemia. Recurrent back pain - often nocturnal and of varying intensity - is an eventual complaint, as is morning stiffness typically relieved by activity. A flexed or bent-over posture eases back pain and paraspinal muscle spasm; thus, some degree of kyphosis is common in untreated patients.

Systemic manifestations occur in 1/3 of patients. Recurrent, usually self-limited, acute iritis (anterior uveitis) rarely is protracted and severe enough to impair vision. Neurologic signs can occasionally result from compression radiculitis or sciatica, vertebral fracture or subluxation, and cauda equina syndrome (which consists of impotence, nocturnal urinary incontinence, diminished bladder and rectal sensation, and absence of ankle jerks). Cardiovascular manifestations can include aortic insufficiency, angina, pericarditis, and ECG conduction abnormalities. A rare pulmonary finding is upper lobe fibrosis, occasionally with cavitation that may be mistaken for TB and can be complicated by infection with *Aspergillus.*

AS is characterized by mild or moderate flares of active spondylitis alternating with periods of almost or totally inactive inflammation. Proper treatment in most patients results in minimal or no disability and in full, productive lives despite back stiffness. Occasionally, the course is severe and progressive, resulting in pronounced incapacitating deformities. The prognosis is bleak for patients with refractory iritis and for the rare patient with secondary amyloidosis.

The ESR and other acute-phase reactants (*e.g*., C-reactive protein and serum Ig levels) are mildly elevated in most patients with active AS. Tests for IgM rheumatoid factor and antinuclear antibodies are negative. A positive test for HLA-B27 is usual but not invariable and not specific (a negative test is more useful in helping to exclude AS than a positive test is in diagnosing it). This test is not necessary in patients with typical disease.

Diagnosis must be confirmed by x-ray. The earliest abnormalities (pseudo-widening from subchondral erosions, sclerosis or later narrowing) occur in the sacroiliac joints. Early changes in the spine are upper lumbar vertebral squaring and demineralization, spotty ligamentous calcification, and one or two evolving syndesmophytes. The classic bamboo spine with prominent syndesmophytes and diffuse paraspinal ligamentous calcification is not useful for early diagnosis; these changes develop in a minority of patients over an average period of 10 years.

The severity of joint involvement and the degree of systemic symptoms vary greatly from one individual to another. Early, accurate diagnosis and therapy may minimize years of pain and disability.

Joint discomfort may be relieved with drugs. Treatment plans usually address prevention, delay, or correction of the deformity and psychosocial and rehabilitation needs. For proper posture and joint motion, daily exercise and other supportive measures (*e.g*., postural training, therapeutic exercise) are vital to strengthen muscle groups that oppose the direction of potential deformities (*i.e*., strengthen the extensor rather than flexor muscle groups). Reading while lying prone and thus extending the neck may help keep the back flexible.

NSAIDs facilitate exercise and other supportive measures by suppressing articular inflammation, pain, and muscle spasm. Most NSAIDs are of proven value in AS, but tolerance and toxicity, rather than marginal differences in efficacy, dictate drug choice. Patients should be monitored and warned of potential adverse reactions. The daily dose of NSAIDs should be as low as possible, but maximum doses of a drug such as indomethacin may be needed with active disease. Drug withdrawal should be attempted only slowly, after systemic and articular signs of active disease have been suppressed for several months. Several new NSAIDs, referred to as COX-2 drugs because they inhibit cyclooxygenase-2, provide equal effectiveness to drugs that inhibit COX-1 with less chance of adverse effects on the gastric mucosa, and platelet aggregation.

Corticosteroids have limited therapeutic value; long-term use is associated with many serious adverse effects, including osteoporosis of the stiff spine. For acute iritis, topical corticosteroids (and mydriatics) usually are adequate; oral corticosteroids are rarely indicated. Intra-articular corticosteroids may be beneficial, particularly when one or two peripheral joints are more severely inflamed than others, thereby compromising exercise and rehabilitation.

Most slow-acting (remitting) drugs for RA (*e.g*., gold given IM) either have not been studied or are not effective for AS. Sulfasalazine may be helpful, particularly when the peripheral joints are involved. Dosage should be started at 500 mg/day and increased by 500 mg/day at 1-wk intervals to 1g bid maintenance (*see also* Rheumatoid Arthritis in Ch. 50). The most common side effect is nausea, which is mainly central, but enteric-coated tablets are better tolerated. Dose reduction may help.

Narcotics, other strong analgesics, and muscle relaxants lack anti-inflammatory properties and should be prescribed only short-term as adjuncts to help control severe back pain and spasm. Radiotherapy to the spine, although effective, is recommended as a last resort because it increases the risk of acute myelogenous leukemia ten-fold.

Rehabilitation therapies are essential. Proper sleep and walking positions, coupled with abdominal and back exercises, help maintain posture. Exercises help maintain joint flexibility. Breathing exercises enhance lung capacity, and swimming provides aerobic exercise. Even with optimal treatment, some people will develop a stiff or "ankylosed" spine, but they will remain functional if this fusion occurs in an upright position. Continuing care is critical. AS is a lifelong problem, and people often fail to continue treatment, in which case permanent posture and mobility losses occur.

### B. Psoratic Arthritis

Psoriasis is an inflammatory and proliferative skin disorder with a prevalence of 1.5-3%. Approximately 20% of patients with psoriasis develop a characteristic form of arthritis that has several patterns (Gladman, 1992; Moll & Wright, 1991; Jones *et al.,* 1994; Gladman *et al.,* 1995). Some individuals present with joint symptoms first but in the majority, skin psoriasis presents first. About one-third of patients have simultaneous exacerbations of their skin and joint disease (Gladman *et al.,* 1987) and there is a topographic relationship between nail and distal interphalangeal joint disease (Jones *et al.,* 1994; 33:834-9; V. Wright, 1956). Although the inflammatory processes which link skin, nail and joint disease remain elusive, an immune-mediated pathology is implicated.

Psoriatic arthritis (PsA) is a chronic inflammatory arthropathy characterized by the association of arthritis and psoriasis and was recognized as a clinical entity distinct from rheumatoid arthritis (RA) in 1964 (Blumberg *et al.,* 1964). Subsequent studies have revealed that PsA shares a number of genetic, pathogenic and clinical features with other spondyloarthropathies (SpAs), a group of diseases that comprise ankylosing spondylitis, reactive arthritis and enteropathic arthritis (Wright, V., 1979). The notion that PsA belongs to the SpA group has recently gained further support from imaging studies demonstrating widespread enthesitis in the, including PsA but not RNA (McGonagle *et al.,* 1999; McGonagle *et al.,* 1998). More specifically, enthesitis has been postulated to be one of the earliest events occurring in the SpAs, leading to bone remodeling and ankylosis in the spine, as well as to articular synovitis when the inflamed entheses are close to peripheral joints. However, the link between enthesitis and the clinical manifestations in PsA remains largely unclear, as PsA can present with fairly heterogeneous patterns of joint involvement with variable degrees of severity (Marsal *et al.,* 1999; Salvarani *et al.,* 1998). Thus, other factors must be posited to account for the multifarious features of PsA, only a few of which (such as the expression of the HLA-B27 molecule, which is strongly associated with axial disease) have been identified. As a consequence, it remains difficult to map the disease manifestations to specific pathogenic mechanisms, which means that the treatment of this condition remains largely empirical.

Family studies have suggested a genetic contribution to the development of PsA (Moll & Wright, 1973). Other chronic inflammatory forms of arthritis, such as ankylosing spondylitis and rheumatoid arthritis, are thought to have a complex genetic basis. However, the genetic component of PsA has been difficult to assess for several reasons. There is strong evidence for a genetic predisposition to psoriasis alone that may mask the genetic factors that are important for the development of PsA. Although most would accept PsA as a distinct disease entity, at times there is a phenotypic overlap with rheumatoid arthritis and ankylosing spondylitis. Also, PsA itself is not a homogeneous condition and various subgroups have been proposed. Although not all these confounding factors were overcome in the present study, we concentrated on investigating candidate genes in three broad categories of patients with PsA that cover the disease spectrum.

Polymorphisms in the promoter region of the *TNFA* region are of considerable interest as they may influence levels of TNF-α secretion (Jacob *et al.,* 1990; Bendzen *et al.,* 1988). Increased amounts of TNF-α have been reported in both psoriatic skin (Ettehadi *et al*., 1994) and synovial fluid (Partsch *et al.,* 1997).

Recent trials have shown a positive benefit of anti-TNF treatment in both PsA (Mease *et al.,* 2000) and ankylosing spondylitis (Brandt *et al.,* 2000). Furthermore, the locus for TNF-α resides within the class III region of the MHC and thus may provide tighter associations with PsA than those provided by flanking class I and class II regions. There were relatively weak associations with the *TNFA* alleles in our total PsA group. The uncommon *TNFA* -238A allele was increased in frequency in the group with peripheral polyarthritis and absent in those patients with spondylitis, although this finding may be explained by linkage disequilibrium with HLA-Cw*0602. Whether there are functional consequences associated with polymorphisms at the *TNFA* -238 allele is unclear (Pociot *et al.,* 1995). Nonetheless, it is possible that the pattern of arthritis that develops in patients with psoriasis may be linked directly or indirectly to this particular allele.

Hohler et al. (A TNF-α promoter polymorphism is associated with juvenile onset psoriasis and psoriatic arthritis, J Invest Dermatol, 1997; 109:562-5) found an increase in the frequency of the *TNFA* -238A allele in patients with PsA as well as in juvenile onset psoriasis. The association of *TNFA* -238A with both juvenile onset psoriasis and PsA was stronger than that with HLA-Cw6. Similarly, in our study, there were strong associations between juvenile onset psoriasis and both HLA-Cw*0602 and *TNFA* -238A, although neither allele had any relationship to the age of onset of arthritis. In our study, all patients with PsA who had at least one *TNFA* -238A allele were HLA-Cw6-positive, emphasizing the close linkage between these alleles in PsA. However, in contrast to the study by Hohler *et al.* (1997), and explainable by close linkage to HLA-Cw*0602, the *TNFA* -238A allele was only increased in patients with peripheral arthritis. It is also of interest that, in a separate study of ankylosing spondylitis, the same group found the uncommon *TNFA* -308A and -238A alleles to have a protective effect on the development of spondylitis (Hohler et al., Association of different tumor necrosis factor alpha promoter allele frequencies with ankylosing spondylitis in HLA-B27 positive individuals. Arthritis Rheum 1998; 41:1489-92).

### C. Reactive Arthritis

In reactive arthritis (ReA) the mechanism of joint damage is unclear, but it is likely that cytokines play critical roles. A more prevalent Th1 profile high levels of interferon gamma (IFN-γ) and low levels of interleukin 4 (IL-4) has been reported (Lahesmaa *et al.,* 1992; Schlaak *et al.,* 1992; Simon *et al.,* 1993; Schlaak *et al.,* 1996; Kotake *et al.,* 1999; Ribbens *et al.,* 2000), but several studies have shown relative predominance of IL-4 and IL-10 and relative lack of IFN-γ and tumour necrosis factor alpha (TNF-α) in the synovial membrane (Simon *et al.,* 1994; Yin *et al.,* 1999) and fluid (SF) (Yin *et al.,* 1999; Yin *et al.,* 1997) of reactive arthritis patients compared with rheumatoid arthritis (RA) patients. A lower level of TNF-α secretion in reactive arthritis than in RNA patients has also been reported after ex *vivo* stimulation of peripheral blood mononuclear cells (PBMC) (Braun *et al., 1999).*

It has been argued that clearance of reactive arthritis-associated bacteria requires the production of appropriate levels of IFN-γ and TNF-α, while IL-10 acts by suppressing these responses (Autenrieth *et al.,* 1994; Sieper & Braun, 1995). IL-10 is a regulatory cytokine that inhibits the synthesis of IL-12 and TNF-γ by activated macrophages (de Waal *et al.,* 1991; Hart *et al.,* 1995; Chomarat *et al.,* 1995) and of IFN-γ by T cells'(Macatonia *et al.,* 1993).

### D. Enteropathic Arthritis

Enteropathic arthritis (EA) occurs in combination with inflammatory bowel diseases (IBD) such as Crohn's disease or ulcerative colitis. It also can affect the spine and sacroiliac joints. Enteropathic arthritis involves the peripheral joints, usually in the lower extremities such as the knees or ankles. It commonly involves only a few or a limited number of joints and may closely follow the bowel condition. This occurs in approximately 11% of patients with ulcerative colitis and 21% of those with Crohn's disease. The synovitis is generally self-limited and non-deforming.

Enteropathic arthropathies comprise a collection of rheumatologic conditions that share a link to GI pathology. These conditions include reactive (*i.e*., infection-related) arthritis due to bacteria *(e.g., Shigella, Salmonella, Campylobacter, Yersinia* species, *Clostridium difficile),* parasites *(e.g., Strongyloides stercoralis, Taenia saginata, Giardia lamblia, Ascaris lumbricoides, Cryptosporidium* species), and spondyloarthropathies associated with inflammatory bowel disease (IBD). Other conditions and disorders include intestinal bypass (jejunoileal), arthritis, celiac disease, Whipple disease, and collagenous colitis.

The precise causes of enteropathic arthropathies are unknown. Inflammation of the GI tract may increase permeability, resulting in absorption of antigenic material, including bacterial antigens. These arthrogenic antigens may then localize in musculoskeletal tissues (including entheses and synovium), thus eliciting an inflammatory response. Alternatively, an autoimmune response may be induced through molecular mimicry, in which the host's immune response to these antigens cross-reacts with self-antigens in synovium.

Of particular interest is the strong association between reactive arthritis and HLA-B27, an HLA class I molecule. A potentially arthrogenic, bacterially derived antigen peptide could fit in the antigen-presenting groove of the B27 molecule, resulting in a CD8+ T-cell response. HLA-B27 transgenic rats develop features of enteropathic arthropathy with arthritis and gut inflammation.

### E. Ulcerative Colitis

Ulcerative colitis is a disease that causes inflammation and sores, called ulcers, in the lining of the large intestine. The inflammation usually occurs in the rectum and lower part of the colon, but it may affect the entire colon. Ulcerative colitis rarely affects the small intestine except for the end section, called the terminal ileum. Ulcerative colitis may also be called colitis or proctitis. The inflammation makes the colon empty frequently, causing diarrhea. Ulcers form in places where the inflammation has killed the cells lining the colon; the ulcers bleed and produce pus.

Ulcerative colitis is an inflammatory bowel disease (IBD), the general name for diseases that cause inflammation in the small intestine and colon. Ulcerative colitis can be difficult to diagnose because its symptoms are similar to other intestinal disorders and to another type of IBD, Crohn's disease. Crohn's disease differs from ulcerative colitis because it causes inflammation deeper within the intestinal wall. Also, Crohn's disease usually occurs in the small intestine, although it can also occur in the mouth, esophagus, stomach, duodenum, large intestine, appendix, and anus.

Ulcerative colitis may occur in people of any age, but most often it starts between ages 15 and 30, or less frequently between ages 50 and 70. Children and adolescents sometimes develop the disease. Ulcerative colitis affects men and women equally and appears to run in some families. Theories about what causes ulcerative colitis abound, but none have been proven. The most popular theory is that the body's immune system reacts to a virus or a bacterium by causing ongoing inflammation in the intestinal wall. People with ulcerative colitis have abnormalities of the immune system, but doctors do not know whether these abnormalities are a cause or a result of the disease. Ulcerative colitis is not caused by emotional distress or sensitivity to certain foods or food products, but these factors may trigger symptoms in some people.

The most common symptoms of ulcerative colitis are abdominal pain and bloody diarrhea. Patients also may experience fatigue, weight loss, loss of appetite, rectal bleeding, and loss of body fluids and nutrients. About half of patients have mild symptoms. Others suffer frequent fever, bloody diarrhea, nausea, and severe abdominal cramps. Ulcerative colitis may also cause problems such as arthritis, inflammation of the eye, liver disease (hepatitis, cirrhosis, and primary sclerosing cholangitis), osteoporosis, skin rashes, and anemia. No one knows for sure why problems occur outside the colon. Scientists think these complications may occur when the immune system triggers inflammation in other parts of the body. Some of these problems go away when the colitis is treated.

A thorough physical exam and a series of tests may be required to diagnose ulcerative colitis. Blood tests may be done to check for anemia, which could indicate bleeding in the colon or rectum. Blood tests may also uncover a high white blood cell count, which is a sign of inflammation somewhere in the body. By testing a stool sample, the doctor can detect bleeding or infection in the colon or rectum. The doctor may do a colonoscopy or sigmoidoscopy. For either test, the doctor inserts an endoscope - a long, flexible, lighted tube connected to a computer and TV monitor - into the anus to see the inside of the colon and rectum. The doctor will be able to see any inflammation, bleeding, or ulcers on the colon wall. During the exam, the doctor may do a biopsy, which involves taking a sample of tissue from the lining of the colon to view with a microscope. A barium enema x ray of the colon may also be required. This procedure involves filling the colon with barium, a chalky white solution. The barium shows up white on x-ray film, allowing the doctor a clear view of the colon, including any ulcers or other abnormalities that might be there.

Treatment for ulcerative colitis depends on the seriousness of the disease. Most people are treated with medication. In severe cases, a patient may need surgery to remove the diseased colon. Surgery is the only cure for ulcerative colitis. Some people whose symptoms are triggered by certain foods are able to control the symptoms by avoiding foods that upset their intestines, like highly seasoned foods, raw fruits and vegetables, or milk sugar (lactose). Each person may experience ulcerative colitis differently, so treatment is adjusted for each individual. Emotional and psychological support is important. Some people have remissions - periods when the symptoms go away - that last for months or even years. However, most patients' symptoms eventually return. This changing pattern of the disease means one cannot always tell when a treatment has helped. Some people with ulcerative colitis may need medical care for some time, with regular doctor visits to monitor the condition.

The goal of therapy is to induce and maintain remission, and to improve the quality of life for people with ulcerative colitis. Several types of drugs are available:
- Aminosalicylates - drugs that contain 5-aminosalicyclic acid (5-ASA), help control inflammation. Sulfasalazine is a combination of sulfapyridine and 5-ASA and is used to induce and maintain remission. The sulfapyridine component carries the anti-inflammatory 5-ASA to the intestine. However, sulfapyridine may lead to side effects such as include nausea, vomiting, heartburn, diarrhea, and headache. Other 5-ASA agents such as olsalazine, mesalamine, and balsalazide, have a different carrier, offer fewer side effects, and may be used by people who cannot take sulfasalazine. 5-ASAs are given orally, through an enema, or in a suppository, depending on the location of the inflammation in the colon. Most people with mild or moderate ulcerative colitis are treated with this group of drugs first.
- Corticosteroids - such as prednisone and hydrocortisone also reduce inflammation. They may be used by people who have moderate to severe ulcerative colitis or who do not respond to 5-ASA drugs. Corticosteroids, also known as steroids, can be given orally, intravenously, through an enema, or in a suppository, depending on the location of the inflammation. These drugs can cause side effects such as weight gain, acne, facial hair, hypertension, mood swings, and an increased risk of infection. For this reason, they are not recommended for long-term use.
- hnmunomodulators - such as azathioprine and 6-mercapto-purine (6-MP) reduce inflammation by affecting the immune system. They are used for patients who have not responded to 5-ASAs or corticosteroids or who are dependent on corticosteroids. However, immunomodulators are slow-acting and may take up to 6 months before the full benefit is seen. Patients taking these drugs are monitored for complications including pancreatitis and hepatitis, a reduced white blood cell count, and an increased risk of infection. Cyclosporine A may be used with 6-MP or azathioprine to treat active, severe ulcerative colitis in people who do not respond to intravenous corticosteroids.
Other drugs may be given to relax the patient or to relieve pain, diarrhea, or infection.

Occasionally, symptoms are severe enough that the person must be hospitalized. For example, a person may have severe bleeding or severe diarrhea that causes dehydration. In such cases the doctor will try to stop diarrhea and loss of blood, fluids, and mineral salts. The patient may need a special diet, feeding through a vein, medications, or sometimes surgery.

About 25-40% of ulcerative colitis patients must eventually have their colons removed because of massive bleeding, severe illness, rupture of the colon, or risk of cancer. Sometimes the doctor will recommend removing the colon if medical treatment fails or if the side effects of corticosteroids or other drugs threaten the patient's health. Surgery to remove the colon and rectum, known as proctocolectomy, is followed by one of the following:
- **Ileostomy**, in which the surgeon creates a small opening in the abdomen, called a stoma, and attaches the end of the small intestine, called the ileum, to it. Waste will travel through the small intestine and exit the body through the stoma. The stoma is about the size of a quarter and is usually located in the lower right part of the abdomen near the beltline. A pouch is worn over the opening to collect waste, and the patient empties the pouch as needed.
- **Ileoanal anastomosis,** or pull-through operation, which allows the patient to have normal bowel movements because it preserves part of the anus. In this operation, the surgeon removes the diseased part of the colon and the inside of the rectum, leaving the outer muscles of the rectum. The surgeon then attaches the ileum to the inside of the rectum and the anus, creating a pouch. Waste is stored in the pouch and passed through the anus in the usual manner. Bowel movements may be more frequent and watery than before the procedure. Inflammation of the pouch (pouchitis) is a possible complication.
Not every operation is appropriate for every person. Which surgery to have depends on the severity of the disease and the patient's needs, expectations, and lifestyle. People faced with this decision should get as much information as possible by talking to their doctors, to nurses who work with colon surgery patients (enterostomal therapists), and to other colon surgery patients. Patient advocacy organizations can direct people to support groups and other information resources.

Most people with ulcerative colitis will never need to have surgery. If surgery does become necessary, however, some people find comfort in knowing that after the surgery, the colitis is cured and most people go on to live normal, active lives.

### F. Crohn's Disease

Another disorder for which immunosuppression has been tried is Crohn's disease. Crohn's disease symptoms include intestinal inflammation and the development of intestinal stenosis and fistulas; neuropathy often accompanies these symptoms. Anti-inflammatory drugs, such as 5-aminosalicylates (*e.g*., mesalamine) or corticosteroids, are typically prescribed, but are not always effective (reviewed in V. A. Botoman *et al.,* 1998). Immunosuppression with cyclosporine is sometimes beneficial for patients resistant to or intolerant of corticosteroids (J. Brynskov *et al.,* 1989).

Nevertheless, surgical correction is eventually required in 90% of patients; 50% undergo colonic resection (Leiper *et al.,* 1998; Makowiec *et al.,* 1998). The recurrence rate after surgery is high, with 50% requiring further surgery within 5 years (Leiper *et al.,* 1998; Besnard *et al.,* 1998).

One hypothesis for the etiology of Crohn's disease is that a failure of the intestinal mucosal barrier, possibly resulting from genetic susceptibilities and environmental factors (*e.g*., smoking), exposes the immune system to antigens from the intestinal lumen including bacterial and food antigens (*e.g.,* Soderholm *et al.,* 1999; Hollander *et al.,* 1986; D. Hollander, 1992). Another hypothesis is that persistent intestinal infection by pathogens such as *Mycobacterium paratuberculosis, Listeria monocytogenes,* abnormal *Escherichia coli,* or paramyxovirus, stimulates the immune response; or alternatively, symptoms result from a dysregulated immune response to ubiquitous antigens, such as normal intestinal microflora and the metabolites and toxins they produce (Sartor, 1997). The presence of IgA and IgG anti*-Sacccharomyces cerevisiae* antibodies (ASCA) in the serum was found to be highly diagnostic of pediatric Crohn's disease (Ruemmele *et al.,* 1998; Hoffenberg *et al.,* 1999).

In Crohn's disease, a dysregulated immune response is skewed toward cell-mediated immunopathology (Murch, 1998). But immunosuppressive drugs, such as cyclosporine, tacrolimus, and mesalamine have been used to treat corticosteroid-resistant cases of Crohn's disease with mixed success (Brynskov *et al.,* 1989; Fellerman *et al.,* 1998).

Recent efforts to develop diagnostic and treatment tools against Crohn's disease have focused on the central role of cytokines (Schreiber, 1998; van Hogezand & Verspaget, 1998). Cytokines are small secreted proteins or factors (5 to 20 kD) that have specific effects on cell-to-cell interactions, intercellular communication, or the behavior of other cells. Cytokines are produced by lymphocytes, especially T_{H}1 and T_{H}2 lymphocytes, monocytes, intestinal macrophages, granulocytes, epithelial cells, and fibroblasts (reviewed in Rogler &. Andus, 1998; Galley & Webster, 1996). Some cytokines are pro-inflammatory (*e.g*., TNF-α, IL-1 (α and β), IL-6, IL-8, IL-12, or leukemia inhibitory factor [LIF]); others are anti-inflammatory (*e.g*., IL-1 receptor antagonist, IL-4, IL-10, IL-11, and TGF-β). However, there may be overlap and functional redundancy in their effects under certain inflammatory conditions.

In active cases of Crohn's disease, elevated concentrations of TNF-α and IL-6 are secreted into the blood circulation, and TNF-α, IL-1, IL-6, and IL-8 are produced in excess locally by mucosal cells (id.; Funakoshi *et al.,* 1998). These cytokines can have far-ranging effects on physiological systems including bone development, hematopoiesis, and liver, thyroid, and neuropsychiatric function. Also, an imbalance of the IL-1β/IL-1ra ratio, in favor of pro-inflammatory IL-1β, has been observed in patients with Crohn's disease (Rogler & Andus, 1998; Saiki *et al.,* 1998; Dionne *et al.,* 1998; but see S. Kuboyama, 1998). One study suggested that cytokine profiles in stool samples could be a useful diagnostic tool for Crohn's disease (Saiki *et al.,* 1998).

Treatments that have been proposed for Crohn's disease include the use of various cytokine antagonists (*e.g*., IL-1ra), inhibitors (*e.g*., of IL-1β converting enzyme and antioxidants) and anti-cytokine antibodies (Rogler and Andus, 1998; van Hogezand & Verspaget, 1998; Reimund *et al.,* 1998; N. Lugering *et al.,* 1998; McAlindon *et al.,* 1998). In particular, monoclonal antibodies against TNF-α have been tried with some success in the treatment of Crohn's disease (Targan *et al.,* 1997; Stack *et al.,* 1997; van Dullemen *et al.,* 1995).

Another approach to the treatment of Crohn's disease has focused on at least partially eradicating the bacterial community that may be triggering the inflammatory response and replacing it with a non-pathogenic community. For example, U.S. Patent 5,599,795 discloses a method for the prevention and treatment of Crohn's disease in human patients. Their method was directed to sterilizing the intestinal tract with at least one antibiotic and at least one anti-fungal agent to kill off the existing flora and replacing them with different, select, well-characterized bacteria taken from normal humans. Borody taught a method of treating Crohn's disease by at least partial removal of the existing intestinal microflora by lavage and replacement with a new bacterial community introduced by fecal inoculum from a disease-screened human donor or by a composition comprising *Bacteroides* and *Escherichia coli* species. (U.S. Patent 5,443,826). However, there has been no known cause of Crohn's disease to which diagnosis and/or treatment could be directed.

### G. Rhematoid Arthritis

The exact etiology of RA remains unknown, but the first signs of joint disease appear in the synovial lining layer, with proliferation of synovial fibroblasts and their attachment to the articular surface at the joint margin (Lipsky, 1998). Subsequently, macrophages, T cells and other inflammatory cells are recruited into the joint, where they produce a number of mediators, including the cytokines interleukin-1 (IL-1), which contributes to the chronic sequelae leading to bone and cartilage destruction, and tumour necrosis factor (TNF-α), which plays a role in inflammation (Dinarello, 1998; Arend & Dayer, 1995; van den Berg, 2001). The concentration of IL-1 in plasma is significantly higher in patients with RA than in healthy individuals and, notably, plasma IL-1 levels correlate with RA disease activity (Eastgate *et al.,* 1988). Moreover, synovial fluid levels of IL-1 are correlated with various radiographic and histologic features of RA (Kahle *et al.,* 1992; Rooney *et al.,* 1990).

In normal joints, the effects of these and other proinflaminatory cytokines are balanced by a variety of anti-inflammatory cytokines and regulatory factors (Burger & Dayer, 1995). The significance of this cytokine balance is illustrated in juvenile RA patients, who have cyclical increases in fever throughout the day (Prieur *et al.,* 1987). After each peak in fever, a factor that blocks the effects of IL-1 is found in serum and urine. This factor has been isolated, cloned and identified as IL-1 receptor antagonist (IL-1ra), a member of the IL-1 gene family (Hannum *et al*., 1990). IL-1ra, as its name indicates, is a natural receptor antagonist that competes with IL-1 for binding to type I IL-1 receptors and, as a result, blocks the effects of IL-1 (Arend *et al.,* 1998). A 10- to 100-fold excess of IL-1ra may be needed to block IL-1 effectively; however, synovial cells isolated from patients with RA do not appear to produce enough IL-1ra to counteract the effects of IL-1 (Firestein *et al.,* 1994; Fujikawa *et al.,* 1995).

### H. Systemic Lupus Erythematosus

There has also been no known cause for autoimmune diseases such as systemic lupus erythematosus. Systemic lupus erythematosus (SLE) is an autoimmune rheumatic disease characterized by deposition in tissues of autoantibodies and immune complexes leading to tissue injury (Kotzin, 1996). In contrast to autoimmune diseases such as MS and type 1 diabetes mellitus, SLE potentially involves multiple organ systems directly, and its clinical manifestations are diverse and variable (reviewed by Kotzin & O'Dell, 1995). For example, some patients may demonstrate primarily skin rash and joint pain, show spontaneous remissions, and require little medication. At the other end of the spectrum are patients who demonstrate severe and progressive kidney involvement that requires therapy with high doses of steroids and cytotoxic drugs such as cyclophosphamide (Kotzin, 1996).

The serological hallmark of SLE, and the primary diagnostic test available, is elevated serum levels of IgG antibodies to constituents of the cell nucleus, such as double-stranded DNA (dsDNA), single-stranded DNA (ss-DNA), and chromatin. Among these autoantibodies, IgG anti-dsDNA antibodies play a major role in the development of lupus glomerulonephritis (G N) (Hahn & Tsao, 1993; Ohnishi *et al.,* 1994). Glomerulonephritis is a serious condition in which the capillary walls of the kidney's blood purifying glomeruli become thickened by accretions on the epithelial side of glomerular basement membranes. The disease is often chronic and progressive and may lead to eventual renal failure.

The mechanisms by which autoantibodies are induced in these autoimmune diseases remains unclear. As there has been no known cause of SLE, to which diagnosis and/or treatment could be directed, treatment has been directed to suppressing immune responses, for example with macrolide antibiotics, rather than to an underlying cause. (*e.g*., U.S. Patent 4,843,092).

### I. Familial Mediterranean Fever

Familial Mediterranean Fever is an inherited disorder usually characterized by recurrent episodes of fever and peritonitis (inflammation of the abdominal membrane). In 1997, researchers identified the gene for FMF and found several different gene mutations that cause this inherited rheumatic disease. The gene, found on chromosome 16, codes for a protein that is found almost exclusively in granulocytes - white blood cells important in the immune response. The protein is likely to normally assist in keeping inflammation under control by deactivating the immune response - without this 'brake,' an inappropriate full-blown inflammatory reaction occurs: an attack of FMF. To explore whether a molecular diagnostic cytokine characteristic exists, serum samples from six patients with clinically diagnosed FMF were examined and the concentration of cytokines were quantified.

### J. Amyotrophic Lateral Sclerosis

Amyotrophic lateral sclerosis (ALS) is an invariably fatal, usually rapidly-progressing disease that kills motor neurons in the brainstem, spinal cord and motor cortex. Approximately 85 % of all ALS cases are sporadic. Of the remaining familial fraction, approximately 25 % are caused by mutations in Cu,Zn-superoxide dismutase (SOD1) (Cudkowicz *et al.,* 1997; Orrell *et al.,* 2000). A murine model for familial ALS has been in existence for ten years (Gurney *et al.,* 1994). In particular mice expressing high copy numbers of human mutant SOD1 with glycine to alanine substituted at residue 93 (G93A-SOD1) develop motor neuron disease at 3-4 months of age. Disease onset in these mice is marked, in part, by broad-spectrum upregulation of pro-inflammatory cytokines and selective up-regulation of some archetypal anti-inflammatory cytokines within the central nervous system (Hensley *et al.,* 2002; 2003). Other research has implicated arachidonate metabolites, particularly prostaglandins produced by cyclooxygeanse-II (COX-II), in the pathobiology of murine ALS (Drachman *et al.,* 2002). The observation of progressive neuroinflammation in the G93A-SODI mouse, combined with recent reports that neuron-specific expression of mutant SOD1 fails to recapitulate disease (Pramatarova *et al.,* 2001; Lino *et al.,* 2002), has lent credence to the hypothesis that glial dysfunction precipitates neuropathology in the murine model of ALS perhaps through disruption of normal cytokine homoeostasis.

To date, few studies have been performed to survey cytokine alterations in humans with ALS. Cytokine networks are technically difficult to study because of the dynamic interrelationships that exist amongst antagonizing or synergistic cytokines. Additionally, human ALS studies may be complicated in the latter stages by general tissue degradation and confounding effects from pharmacotherapy. This is very unfortunate, because there is an urgent need for biomarkers of disease severity (and of drug efficacy) that can be applied to human clinical studies (Bradley *et al.,* 2003). Development of robust biomarkers would facilitate the clinical evaluation of therapeutic candidates, by allowing the assessment of drug efficacy sooner than would be possible through traditional instruments such as function-of-living questionnaires and survival analyses.

### K. Irritable Bowel Syndrome

Irritable bowel syndrome (IBS) is a functional disorder characterized by abdominal pain and altered bowel habits. This syndrome may begin in young adulthood and can be associated with significant disability. This syndrome is not a homogeneous disorder. Rather, subtypes of IBS have been described on the basis of the predominant symptom--diarrhea, constipation, or pain. In the absence of "alarm" symptoms, such as fever, weight loss, and gastrointestinal bleeding, a limited workup is needed. Once a diagnosis of IBS is made, an integrated treatment approach can effectively reduce the severity of symptoms. IBS is a common disorder, although its prevalence rates have varied. In general, IBS affects about 15% of US adults and occurs about three times more often in women than in men (Jailwala *et al.,* 2000).

IBS accounts for between 2.4 million and 3.5 million visits to physicians each year. It not only is the most common condition seen by gastroenterologists but also is one of the most common gastrointestinal conditions seen by primary care physicians (Everhart *et al.,* 1991; Sandler, 1990).

IBS is also a costly disorder. Compared with persons who do not have bowel symptoms, persons with IBS miss three times as many workdays and are more likely to report being too sick to work (Drossman *et al.,* 1993; Drossman *et al.,* 1997). Moreover, those with IBS incur hundreds of dollars more in medical charges than persons without bowel disorders (Talley *et al.,* 1995).

No specific abnormality accounts for the exacerbations and remissions of abdominal pain and altered bowel habits experienced by patients with IBS. The evolving theory of IBS suggests dysregulation at multiple levels of the brain-gut axis. Dysmotility, visceral hypersensitivity, abnormal modulation of the central nervous system (CNS), and infection have all been implicated. In addition, psychosocial factors play an important modifying role. Abnormal intestinal motility has long been considered a factor in the pathogenesis of IBS. Transit time through the small intestine after a meal has been shown to be shorter in patients with diarrhea-predominant IBS than in patients who have the constipation-predominant or pain-predominant subtype (Cann *et al.,* 1983).

In studies of the small intestine during fasting, the presence of both discrete, clustered contractions and prolonged, propagated contractions has been reported in patients with IBS (Kellow & Phillips, 1987). They also experience pain with irregular contractions more often than healthy persons (Kellow & Phillips, 1987; Horwitz & Fisher, 2001)

These motility findings do not account for the entire symptom complex in patients with IBS; in fact, most of these patients do not have demonstrable abnormalities (Rothstein RD, 2000). Patients with IBS have increased sensitivity to visceral pain. Studies involving balloon distention of the rectosigmoid colon have shown that patients with IBS experience pain and bloating at pressures and volumes much lower than control subjects (Whitehead *et al.,* 1990). These patients maintain normal perception of somatic stimuli.

Multiple theories have been proposed to explain this phenomenon. For example, receptors in the viscera may have increased sensitivity in response to distention or intraluminal contents. Neurons in the dorsal horn of the spinal cord may have increased excitability. In addition, alteration in CNS processing of sensations may be involved (Drossman *et al.,* 1997). Functional magnetic resonance imaging studies have recently shown that compared with control subjects, patients with IBS have increased activation of the anterior cingulate cortex, an important pain center, in response to a painful rectal stimulus (Mertz *et al.,* 2000).

Increasingly, evidence suggests a relationship between infectious enteritis and subsequent development of IBS. Inflammatory cytokines may play a role. In a survey of patients with a history of confirmed bacterial gastroenteritis (Neal *et al.,* 1997), 25% reported persistent alteration of bowel habits. Persistence of symptoms may be due to psychologic stress at the time of acute infection (Gwee *et al.,* 1999).

Recent data suggest that bacterial overgrowth in the small intestine may have a role in IBS symptoms. In one study (Pimentel *et al.,* 2000), 157 (78%) of 202 IBS patients referred for hydrogen breath testing had test findings that were positive for bacterial overgrowth. Of the 47 subjects who had follow-up testing, 25 (53%) reported improvement in symptoms (ie, abdominal pain and diarrhea) with antibiotic treatment.

IBS may present with a range of symptoms. However, abdominal pain and altered bowel habits remain the primary features. Abdominal discomfort is often described as crampy in nature and located in the left lower quadrant, although the severity and location can differ greatly. Patients may report diarrhea, constipation, or alternating episodes of diarrhea and constipation: Diarrheal symptoms are typically described as small-volume, loose stools, and stool is sometimes accompanied by mucus discharge. Patients also may report bloating, fecal urgency, incomplete evacuation, and abdominal distention. Upper gastrointestinal symptoms, such as gastroesophageal reflux, dyspepsia, or nausea, may also be present (Lynn & Friedman, 1993).

Persistence of symptoms is not an indication for further testing; it is a characteristic of IBS and is itself an expected symptom of the syndrome. More extensive diagnostic evaluation is indicated in patients whose symptoms are worsening or changing. Indications for further testing also include presence of alarm symptoms, onset of symptoms after age 50, and a family history of colon cancer. Tests may include colonoscopy, computed tomography of the abdomen and pelvis, and barium studies of the small or large intestine.

### L. Juvenile Rheumatoid Arthritis

'Juvenile rheumatoid arthritis' (JRA), a term for the most prevalent form of arthritis in children, is applied to a family of illnesses characterized by chronic inflammation and hypertrophy of the synovial membranes. The term overlaps, but is not completely synonymous, with the family of illnesses referred to as juvenile chronic arthritis and/or juvenile idiopathic arthritis in Europe.

Jarvis (1998) and others (Arend, 2001) have proposed that the pathogenesis of rheumatoid disease in adults and children involves complex interactions between innate and adaptive immunity. This complexity lies at the core of the difficulty of unraveling disease pathogenesis.

Both innate and adaptive immune systems use multiple cell types, a vast array of cell surface and secreted proteins, and interconnected networks of positive and negative feedback (Lo *et al.,* 1999). Furthermore, while separable in thought, the innate and adaptive wings of the immune system are functionally intersected (Fearon & Locksley, 1996), and pathologic events occurring at these intersecting points are likely to be highly relevant to our understanding of pathogenesis of adult and childhood forms of chronic arthritis (Warrington, *et al.,* 2001).

Polyarticular JRA is a distinct clinical subtype characterized by inflammation and synovial proliferation in multiple joints (four or more), including the small joints of the hands (Jarvis, 2002). This subtype of JRA may be severe, because of both its multiple joint involvement and its capacity to progress rapidly over time. Although clinically distinct, polyarticular JRA is not homogeneous, and patients vary in disease manifestations, age of onset, prognosis, and therapeutic response. These differences very likely reflect a spectrum of variation in the nature of the immune and inflammatory attack that can occur in this disease (Jarvis, 1998).

### M. Sjogren's syndrome

Primary Sjögren's syndrome (SS) is a chronic, slowly progressive, systemic autoimmune disease, which affects predominantly middle-aged women (female-to-male ratio 9: 1), although it can be seen in all ages including childhood (Jonsson *et al.,* 2002). It is characterized by lymphocytic infiltration and destruction of the exocrine glands, which are infiltrated by mononuclear cells including CD4+, CD8+ lymphocytes and B-cells (Jonsson *et al.,* 2002). In addition, extraglandular (systemic) manifestations are seen in one-third of patients (Jonsson *et al*., 2001).

The glandular lymphocytic infiltration is a progressive feature (Jonsson *et al.,* 1993), which, when extensive, may replace large portions of the organs. Interestingly, the glandular infiltrates in some patients closely resemble ectopic lymphoid microstructures in the salivary glands (denoted as ectopic germinal centers) (Salomonsson *et al.,* 2002; Xanthou & Polihronis, 2001). In SS, ectopic GCs are defined as T and B cell aggregates of proliferating cells with a network of follicular dendritic cells and activated endothelial cells. These GC-like structures formed within the target tissue also portray functional properties with production of autoantibodies (and-Ro/SSA and anti-La/SSB) (Salomonsson &, Jonsson, 2003).

In other systemic autoimmune diseases, such as RA, factors critical for ectopic GCs have been identified. Rheumatoid synovial tissues with GCs were shown to produce chemokines CXCL13, CCL21 and lymphotoxin (LT)- β (detected on follicular center and mantle zone B cells). Multivariate regression analysis of these analytes identified CXCL13 and LT-β as the solitary cytokines predicting GCs in rheumatoid synovitis (Weyand & Goronzy, 2003). Recently CXCL13 and CXCR5 in salivary glands has been shown to play an essential role in the inflammatory process by recruiting B and T cells, therefore contributing to lymphoid neogenesis and ectopic GC formation in SS (Salomonsson & Larsson, 2002).

### N. Early Arthritis

The clinical presentation of different inflammatory arthropathies is similar early in the course of disease. As a result, it is often difficult to distinguish patients who are at risk of developing the severe and persistent synovitis that leads to erosive joint damage from those whose arthritis is more self-limited. Such distinction is critical in order to target therapy appropriately, treating aggressively those with erosive disease and avoiding unnecessary toxicity in patients with more self-limited disease. Current clinical criteria for diagnosing erosive arthropathies such as rheumatoid arthritis (RA) are less effective in early disease and traditional markers of disease activity such as joint counts and acute phase response do not adequately identify patients likely to have poor outcomes (Harrison & Symmons *et al.,* 1998). Parameters reflective of the pathologic events occurring in the synovium are most likely to be of significant prognostic value.

Recent efforts to identify predictors of poor outcome in early inflammatory arthritis have identified the presence of RA specific autoantibodies, in particular antibodies towards citrullinated peptides, to be associated with erosive and persistent disease in early inflammatory arthritis cohorts. On the basis of this, a cyclical citrullinated peptide (CCP) has been developed to assist in the identification of anti-CCP antibodies in patient sera. Using this approach, the presence of anti-CCP antibodies has been shown to be specific and sensitive for RA, can distinguish RA from other arthropathies, and can potentially predict persistent, erosive synovitis before these outcomes become clinically manifest (Schellekens *et al.,* 2000). Importantly, anti-CCP antibodies are often detectable in sera many years prior to clinical symptoms suggesting that they may be reflective of subclinical immune events ((Nielen *et al.,* 2004; Rantapaa-Dahlqvist *et al*., 2003).

The clinical presentation of different inflammatory arthropathies is similar early in the course of disease. As a result, it is often difficult to distinguish patients who are at risk of developing the severe and persistent synovitis that leads to erosive joint damage from those whose arthritis is more self-limited. Such distinction is critical in order to target therapy appropriately, treating aggressively those with erosive disease and avoiding unnecessary toxicity in patients with more self-limited disease. Current clinical criteria for diagnosing erosive arthropathies such as rheumatoid arthritis (RA) are less effective in early disease and traditional markers of disease activity such as joint counts and acute phase response do not adequately identify patients likely to have poor outcomes (Harrison *et al.,* 1998). Parameters reflective of the pathologic events occurring in the synovium are most likely to be of significant prognostic value.

Recent efforts to identify predictors of poor outcome in early inflammatory arthritis have identified the presence of RA specific autoantibodies, in particular antibodies towards citrullinated peptides, to be associated with erosive and persistent disease in early inflammatory arthritis cohorts. On the basis of this, a cyclical citrullinated peptide (CCP) has been developed to assist in the identification of anti-CCP antibodies in patient sera. Using this approach, the presence of anti-CCP antibodies has been shown to be specific and sensitive for RA, can distinguish RA from other arthropathies, and can potentially predict persistent, erosive synovitis before these outcomes become clinically manifest. Importantly, anti-CCP antibodies are often detectable in sera many years prior to clinical symptoms suggesting that they may be reflective of subclinical immune events (Nielen *et al.,* 2004; Rantapaa-Dahlqvist *et al.,* 2003).

### O. Psoriasis

Psoriasis is a chronic skin disease of scaling and inflammation that affects 2 to 2.6 percent of the United States population, or between 5.8 and 7.5 million people. Although the disease occurs in all age groups, it primarily affects adults. It appears about equally in males and females. Psoriasis occurs when skin cells quickly rise from their origin below the surface of the skin and pile up on the surface before they have a chance to mature. Usually this movement (also called turnover) takes about a month, but in psoriasis it may occur in only a few days. In its typical form, psoriasis results in patches of thick, red (inflamed) skin covered with silvery scales. These patches, which are sometimes referred to as plaques, usually itch or feel sore. They most often occur on the elbows, knees, other parts of the legs, scalp, lower back, face, palms, and soles of the feet, but they can occur on skin anywhere on the body. The disease may also affect the fingernails, the toenails, and the soft tissues of the genitals and inside the mouth. While it is not unusual for the skin around affected joints to crack, approximately 1 million people with psoriasis experience joint inflammation that produces symptoms of arthritis. This condition is called psoriatic arthritis.

Psoriasis is a skin disorder driven by the immune system, especially involving a type of white blood cell called a T cell. Normally, T cells help protect the body against infection and disease. In the case of psoriasis, T cells are put into action by mistake and become so active that they trigger other immune responses, which lead to inflammation and to rapid turnover of skin cells. In about one-third of the cases, there is a family history of psoriasis. Researchers have studied a large number of families affected by psoriasis and identified genes linked to the disease. People with psoriasis may notice that there are times when their skin worsens, then improves. Conditions that may cause flareups include infections, stress, and changes in climate that dry the skin. Also, certain medicines, including lithium and betablockers, which are prescribed for high blood pressure, may trigger an outbreak or worsen the disease.

### P. Neuroinflammation

Neuroinflammation encapsulates the idea that microglial and astrocytic responses and actions in the central nervous system have a fundamentally inflammation-like character, and that these responses are central to the pathogenesis and progression of a wide variety of neurological disorders. This idea originated in the field of Alzheimer's disease (Griffin *et al.,* 1989; Rogers *et al*., 1988), where it has revolutionized our understanding of this disease (Akiyama *et al.,* 2000). These ideas have been extended to other neurodegenerative diseases (Eikelenboom *et al.,* 2002; Orr *et al.,* 2002; Ishizawa & Dickson, 2001), to ischemic/toxic diseases (Gehrmann *et al.,* 1995; Touzani *et al.,* 1999), to tumor biology (Graeber *et al.,* 2002) and even to normal brain development.

Neuroinflammation incorporates a wide spectrum of complex cellular responses that include activation of microglia and astrocytes and induction of cytokines, chemokines, complement proteins, acute phase proteins, oxidative injury, and related molecular processes. These events may have detrimental effects on neuronal function, leading to neuronal injury, further glial activation, and ultimately neurodegeneration.

Neuroinflammation is a new and rapidly expanding field that has revolutionized our understanding of chronic neurological diseases. This field encompasses research ranging from population studies to signal transduction pathways, and investigators with backgrounds in fields as diverse as pathology, biochemistry, molecular biology, genetics, clinical medicine, and epidemiology. Important contributions to this field have come from work with populations, with patients, with postmortem tissues, with animal models, and with *in vitro* systems.

### III. Cytokine Assays

Traditional cytokine assays in involve measurement of cytokines in serum or plasma, but various cytokines have been detected in other biological fluids. For example, Kimball (1984) reported IL-1 bioactivity in human urine. Tamatani *et al.* (1988) disclosed the presence of IL-1α and IL-1β in human amniotic fluid, using chromatographic and bioassay methods. The same group used enzyme immunoassays to measure IL-1α and IL-1β in human amniotic fluid (Tsunoda *et al.,* 1988). Wilmott *et al.* (1988) measured IL-1β and IL-1 bioactivity in human bronchoalveolar lavage fluid in cystic fibrosis compared to other diseases. Khan *et al.* (1988) reported that high levels of IL-1-like bioactivity could be demonstrated in human ovarian follicular fluid. Lymphotoxins have been reported in blister fluid of pemphigoid patients (Jeffes *et al.,* 1984). IL-1 has also been reported in human sweat (Didierjean *et al.,* 1990). IL-1 has been reported to be found in the cerebrospinal fluid (CSF) of cats (Coceani *et al.,* 1988) and humans (see, for example, Peter *et al.,* 1991). A factor with IL-1-like bioactivity was detected in the gingival fluid of clinically normal humans (Oppenheim *et al.,* 1982), the activity being higher in inflamed than non-inflamed gingival regions.

Other methods can be used to quantify specific cytokine expression including methods that measure cytokine mRNA or cytokine polypeptide. For example, PCR™, competitive PCR™, PCR-ELISA, Microarrays, gene expression bead-based assays, and *in situ* hybridization techniques can be used to measure cytokine mRNA, and immunohistochemistry can be used to measure cytokine protein levels.

### A. Genetic Assays

One embodiment of the instant invention comprises a method for detecting variation in cytokine levels using nucleic acid based studies. The nucleic acid is isolated from cells contained in any appropriate biological sample, according to standard methodologies (Sambrook *et al.,* 1989). The nucleic acid may be genomic DNA or fractionated or whole cell RNA. Where RNA is used, it may be desired to convert the RNA to a complementary DNA. In one embodiment, the RNA is whole cell RNA; in another, it is poly-A RNA. Normally, the nucleic acid is amplified.

Depending on the format, the specific nucleic acid of interest is identified in the sample directly using amplification or with a second, known nucleic acid following amplification. Next, the identified product is detected. In certain applications, the detection may be performed by visual means (*e.g*., ethidium bromide staining of a gel). Alternatively, the detection may involve indirect identification of the product via chemiluminescence, radioactive scintigraphy of radiolabel or fluorescent label or even via a system using electrical or thermal impulse signals (Affymax Technology; Bellus, 1994).

Following detection, one may compare the results seen in a given patient with a statistically significant reference group of normal patients and patients that have cytokine-related pathologies. In this way, it is possible to correlate the amount or kind of cytokines detected with various clinical states.

### i. Southern/Northern Blotting

Blotting techniques are well known to those of skill in the art. Southern blotting involves the use of DNA as a target, whereas Northern blotting involves the use of RNA as a target. Each provide different types of information, although cDNA blotting is analogous, in many aspects, to blotting or RNA species.

Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter.

Subsequently, the blotted target is incubated with a probe (usually labeled) under conditions that promote denaturation and rehybridization. Because the probe is designed to base pair with the target, the probe will binding a portion of the target sequence under renaturing conditions. Unbound probe is then removed, and detection is accomplished as described above.

### ii. Separation Methods

It normally is desirable, at one stage or another, to separate the amplification product from the template and the excess primer for the purpose of determining whether specific amplification has occurred. In one embodiment, amplification products are separated by agarose, agarose-acrylamide or polyacrylamide gel electrophoresis using standard methods. See Sambrook *et al.,* 1989.

Alternatively, chromatographic techniques may be employed to effect separation. There are many kinds of chromatography which may be used in the present invention: adsorption, partition, ion-exchange and molecular sieve, and many specialized techniques for using them including column, paper, thin-layer and gas chromatography (Freifelder, 1982).

### Detection Methods

Products may be visualized in order to confirm amplification of the marker sequences. One typical visualization method involves staining of a gel with ethidium bromide and visualization under UV light. Alternatively, if the amplification products are integrally labeled with radio- or fluorometrically-labeled nucleotides, the amplification products can then be exposed to x-ray film or visualized under the appropriate stimulating spectra, following separation.

### iii. Kit Components

All the essential materials and reagents required for detecting and sequencing cytokines may be assembled together in a kit. This generally will comprise preselected primers and probes. Also included may be enzymes suitable for amplifying nucleic acids including various polymerases (RT, Taq, SequenaseTM, etc.), deoxynucleotides and buffers to provide the necessary reaction mixture for amplification. Such kits also generally will comprise, in suitable means, distinct containers for each individual reagent and enzyme as well as for each primer or probe.

### iv. RT-PCR™ (Relative Quantitative)

Reverse transcription (RT) of RNA to cDNA followed by relative quantitative PCR™ (RT-PCR™) can be used to determine the relative concentrations of specific mRNA species isolated from patients. By determining that the concentration of a specific mRNA species varies, it is shown that the gene encoding the specific mRNA species is differentially expressed.

In PCR™, the number of molecules of the amplified target DNA increase by a factor approaching two with every cycle of the reaction until some reagent becomes limiting. Thereafter, the rate of amplification becomes increasingly diminished until there is no increase in the amplified target between cycles. If a graph is plotted in which the cycle number is on the X axis and the log of the concentration of the amplified target DNA is on the Y axis, a curved line of characteristic shape is formed by connecting the plotted points. Beginning with the first cycle, the slope of the line is positive and constant. This is said to be the linear portion of the curve. After a reagent becomes limiting, the slope of the line begins to decrease and eventually becomes zero. At this point the concentration of the amplified target DNA becomes asymptotic to some fixed value. This is said to be the plateau portion of the curve.

The concentration of the target DNA in the linear portion of the PCR™ amplification is directly proportional to the starting concentration of the target before the reaction began. By determining the concentration of the amplified products of the target DNA in PCR™ reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundances of the specific mRNA from which the target sequence was derived can be determined for the respective tissues or cells. This direct proportionality between the concentration of the PCR™ products and the relative mRNA abundances is only true in the linear range of the PCR™ reaction.

The final concentration of the target DNA in the plateau portion of the curve is determined by the availability of reagents in the reaction mix and is independent of the original concentration of target DNA. Therefore, the first condition that must be met before the relative abundances of a mRNA species can be determined by RT-PCR™ for a collection of RNA populations is that the concentrations of the amplified PCR™ products must be sampled when the PCR™ reactions are in the linear portion of their curves.

The second condition that must be met for an RT-PCRT™ experiment to successfully determine the relative abundances of a particular mRNA species is that relative concentrations of the amplifiable cDNAs must be normalized to some independent standard. The goal of an RT-PCR™ experiment is to determine the abundance of a particular mRNA species relative to the average abundance of all mRNA species in the sample. In the experiments described below, mRNAs for ß-actin, asparagine synthetase and lipocortin II were used as external and internal standards to which the relative abundance of other mRNAs are compared.

Most protocols for competitive PCR™ utilize internal PCR™ standards that are approximately as abundant as the target. These strategies are effective if the products of the PCR™ amplifications are sampled during their linear phases. If the products are sampled when the reactions are approaching the plateau phase, then the less abundant product becomes relatively over represented. Comparisons of relative abundancies made for many different RNA samples, such as is the case when examining RNA samples for differential expression, become distorted in such a way as to make differences in relative abundances of RNAs appear less than they actually are. This is not a significant problem if the internal standard is much more abundant than the target. If the internal standard is more abundant than the target, then direct linear comparisons can be made between RNA samples.

The above discussion describes theoretical considerations for an RT-PCR™ assay for clinically derived materials. The problems inherent in clinical samples are that they are of variable quantity (making normalization problematic), and that they are of variable quality (necessitating the co-amplification of a reliable internal control, preferably of larger size than the target). Both of these problems are overcome if the RT-PCR™ is performed as a relative quantitative RT-PCR™ with an internal standard in which the internal standard is an amplifiable cDNA fragment that is larger than the target cDNA fragment and in which the abundance of the mRNA encoding the internal standard is roughly 5-100 fold higher than the mRNA encoding the target. This assay measures relative abundance, not absolute abundance of the respective mRNA species.

Other studies may be performed using a more conventional relative quantitative RT-PCR™ assay with an external standard protocol. These assays sample the PCR™ products in the linear portion of their amplification curves. The number of PCR™ cycles that are optimal for sampling must be empirically determined for each target cDNA fragment. In addition, the reverse transcriptase products of each RNA population isolated from the various tissue samples must be carefully normalized for equal concentrations of amplifiable cDNAs. This consideration is very important since the assay measures absolute mRNA abundance. Absolute mRNA abundance can be used as a measure of differential gene expression only in normalized samples. While empirical determination of the linear range of the amplification curve and normalization of cDNA preparations are tedious and time consuming processes, the resulting RT-PCR™ assays can be superior to those derived from the relative quantitative RT-PCR™ assay with an internal standard.

One reason for this advantage is that without the internal standard/competitor, all of the reagents can be converted into a single PCR™ product in the linear range of the amplification curve, thus increasing the sensitivity of the assay. Another reason is that with only one PCR™ product, display of the product on an electrophoretic gel or another display method becomes less complex, has less background and is easier to interpret.

Still other studies may be performed using "real-time" RT-PCR™ (Higuchi *et al.,* 1993). These assays detect PCR™ products as they accumulate instead of detecting the amount of PER™ products accumulated after a fixed number of cycles. A method of detecting fluorescence after each PCR™ cycle is required. The fluorescence signal is plotted versus the cycle number. The cycle number is expressed as the threshold cycle (C_{T}). The initial fluorescence defines the baseline for the plot and an accumulated PCR™ product is indicated by an increase in fluorescence above the baseline. Quantification of the amount of target in a sample is determined by measuring and comparing the C_{T} to a standard curve to determine the starting copy number.

"Real-Time" RT-PCR™ (Higuchi *et al.,* 1993) provides more precise quantitation of the amount of target because it is determined during the exponential phase of PCR™, rather than at the endpoint. It also allows higher throughput because the use of C_{T} values allow a larger dynamic range. Dilutions of each sample are no longer required.

### v. Gene Expression Bead-Based Assay

A gene expression bead-based assay a protocol was created to show oligonucleotide probes could be attached on the surface with a density optimally suited for gene expression measurement. The attachment method (creating the covalent bond between the carboxylated bead surface and the amine linked oligonucleotide probe, utilizing a carbodiimide-activated succinimide coupling chemistry) was verified with a special 40-mer oligonucleotide probe linked at the 5' end with an amine linker molecule and at the 3' end with a biotin group. The reporter of the fluorescent signal is a streptavidin-linked R-PE (R-Phycoerythrin) complex that produces signal based on the presence of biotin groups at the bead surface. By using this special sequence no hybridization was necessary to verify the oligo probe coupling protocol. Additionally, the amount of oligo probe added to the bead-coupling reaction was titrated through two orders of magnetude to gain a sense of how the surface density of attachment affects fluorescent signal. This technique enables us to attach any short oligonucleotide sequence to the bead surface to serve as probe in hybridization reactions.

The gene expression assay was initially tested with optimum conditions by using an HPLC-purified anti-sense 70-mer target with a biotinylated 5' end in the hybridization reaction and no other competitive sequences. The probes used were selected from the 70mer oligonucleotide library used in our microarray core facility. The hybridization reactions were designed to vary both time and amount of target complement in order to generate rate of reaction graphs and concentration-response curves, respectively. Additionally, cross-reactivity was shown to be under 8% (most data points were under 5% (even under non-stringent conditions at 37 °C) to demonstrate sequence specificity for the bead-based system.

Tests with cDNA transcribed from commercially available, predictably performing mRNA was first used to assess efficacy of the overall bead-based gene expression process. Using oligo-dT as a primer and biotinylated-dUTP to directly label the cDNA transcript, concentration dependant signal was assessed to create a calibration curve for the assay. This process was verified using both human and mouse RNA. To cross-validate the data, and to demonstrate the scalability from microarray platform to bead platform, particular probes chosen from microarray experiments were tested with the same pool of mouse RNA harvested from brain and liver sources. These particular probes were selected from the microarray experiments using the superior data mining techniques developed in our laboratory's bioinformatics core and were shown to be biologically unique and relevant to a particular tissue type (either brain or liver). One hundred percent of the probes assayed showed directional expression matches and concurred with QT-PCR measurements (Table 1) providing powerful evidence of the potential to streamline the costly, labor intensive microarray data onto a smaller, higher-sample throughput, more cost effective assay platform.

The significance of this approach is further augmented by the powerful clinical need for rapid, inexpensive multi-biomarker diagnostic assays requiring a thorough understanding of three main areas of research: protocol design, biosensing paradigm, and lab automation strategy.

Screening of genome-scale microarrays provides the best means of rapidly identifying gene-expression based biomarkers in preparation for next generation clinical assays. Once the previously unknown sequences of diagnostic and therapeutic relevance have been identified from samples the results will be immediately translated to the bead-based assay platform. Becaucse sequence specificity is the primary force behind specificity and signal, oligonucleotides of the exact same length and sequence used in the microarray studies are chosen for attachment to the carboxylated bead surface to mimic the hybridization chemistry on the microarray platform. These oligonucleotides are prepared with a 12-carbon spacer at the 5'-end. The attachment is achieved by using an organic linker at pH 4.5 to create the conditions for hydrolysis thereby creating the covalent bond between the amine and carboxylic acid groups. This enables the oligonucleotides to be permanently attached to the 5.6-micron diameter polystyrene beads and stored safely for up to one year at 4°C in the stabilizing buffer (TE pH 8.0).

Oligo probe attachment is verified with same length reverse complement sequences that have a biotinylated 5' end. If the probes have been successfully attached the signals will correlate directly to the amount of reverse complement sequence added. The signals are acquired via a streptavidin-phycoerythrin label that attaches itself to the biotinylated end of the reverse complement. The R-PE fluorescent signals are generated by 532nm 10mW laser and amplified with a 14-bit A/D PMT (analog to digital photomultiplier tube) for digital signal processing. The signals generated are dose dependant and serve to create a calibration curve to track variance from lot to lot of prepared beads.

By way of example, peripheral blood mononuclear cells (PBMCs) were collected from rheumatoid arthritis patients at several stages of disease severity and undergoing pleural therapeutic treatment. The cellular mRNA was extracted using the RNAeasy Kit from Qiagen cDNA is synthesized using a reverse transcriptase enzyme provided by Qiagen (Omniscript). There is no amplification of the product through multiple cycles; the cDNA is the result of a first strand synthesis. The label, bioin-16-dUTP, is directly incorporated into the cDNA during synthesis using a 3.0/2.5 ratio of label to dTTP. The reverse transcriptase is superior to those often used in the microarray scientific community due to its ability to prevent random priming. However, the removal of cDNA created from random priming results in an attenuation of fluorescent signal due to overall lower transcript abundance. The prevention of this enzymatic effect during the cDNA synthesis step allowed for our never before seen 100% validation of signal ratios via QT-PCR.

The molar ratio of 3.0/2.5 was successful in previous research studies used to validate the bead-based gene expression system. The strategy employed to determine incorporation efficiency will involve spotting a serial dilution of cDNA product (created with several different molar ratios of label) onto a nitrocellulose membrane to participate in a horseradish peroxidase-flourchrome mediated luminescence reaction. This method will enable us to demonstrate the successful creation of cDNA and document the influence of any change in synthesis conditions on cDNA production (such as using gene specific primers, random primers, oligo-dT primers, or biotinylated oligo-dT primers).

Hybridization conditions for the bead-based assay are carefully selected due to the mobility of the reaction surface (the beads move freely in the solution and not attached to any surface). Temperature and hybridization time affect not only the rate of reaction, but also the specificity of the signals generated. In order to generate signals of sufficient specificity we utilize a 5 molar TMAC (Tetramethyl-amonium chloride, Sigma) to create high-salt stringency conditions. The critical chemical property of TMAC is its ability to nearly equalize the bond strength between A-T/G-C dsDNA bonds creating a uniform Tm between the competing probes bound to serparate bead types in the multiplexed reaction. The high temperature of 58°C is chosen also for stringency reasons, and is based on the theoretical Tm of a 70-mer oligonucleotide in 5 normal TMAC. The driving force behind the buffer is to create a sequence specific exclusion reaction at a single temperature, and although creating high stringency conditions inevitably results in an attenuation of fluorescent signal, these conditions are necessary to generate diagnostically relevant results.

Keeping the beads in suspension during the hybridization is necessary to achieve equal accessibility of target to probe at the bead surface. High salt concentration conditions raise the density of the solution above that of polystyrene (the bead composition). This causes the beads to float in static conditions, changing the surface availability for the reaction, potentially lowering the sensitivity of the gene expression measurement. Fortunately the difference in density is small and gentle mixing of many types can keep the beads suspended. This environment has been simulated by using a hybridization oven (Hybaid at 58°C) capable, of rotating the sealed reaction tubes at 10 rpm yielding good results. However, these conditions are a challenge for automation and high throughput, which is why we are developing solutions to integrate the ideal hybridization conditions with the Biomek FX lab automation system.

Even though the hybridization for the bead-based assay has been tailored to mimic the microarray environment as closely as possible to generate the environment to easily scale from one assay to the next, there are subtle differences that mandate that well designed internal controls are a necessity for this project. The normalization and scaling methods for the microarray community rely upon data gathered from thousands of spots on the array, but the bead-based assay can measure at most 100 different transcripts automatically making the normalization and scaling methods different from the microarray.

The positive control is using plant RNA from *A. thaliana,* the 'RUBISCO' gene (GenBank ID: X14212). By using a known amount of sequence-dissimilar RNA spiked into the cDNA synthesis step, we can get a more clear idea of the general success of the labeling step. Furthermore, beads with the appropriate plant probe sequence help describe the complex relationship between starting mRNA concentration and fluorescent signal generation.

The negative control is designed around random 70mer sequences (with no sequence specificity in the human genome) attached to the bead surface, which in theory could not possibly have any target sequences able to bind to them. Any fluorescent signal given off by these beads would provide a measure of background noise produced by cross-hybridization. These fluorescent signals will be used to normalize the patient sample signals to a common background making the inter-well comparisons straightforward.

The most academically accepted way to demonstrate scalability from the microarray platform is to have fluorescent ratios from both systems agree with QT-PCR cross-validation experiments and match directionally. This cross-validation will serve to strengthen the argument that all systems are measuring gene expression in tandem, and differentially between two samples.

Utilizing the Beckman-Coulter Biomek FK system, an automated process to perform the gene-expression bead-based assay has been developed. The Biomek FX can easily integrate with a thermocycling device and various heat blocks to make cDNA synthesis and hybridization conditions automation friendly.

The R-PE molecule is stable at hybridization pH (8.2) and temperature (58°C), but only by a narrow margin (stability is lost at 60°C). Since proper washing has removed cross-hybridizing sequences, it is safe to perform this step across a wide range of temperatures. We have seen no difference in using 37°C versus hybridization temperature at this final labeling step for reverse complement sequences, allowing flexibility in post hybridization labeling strategies.

The final fluorescent signal is created via phycoerythrin, a high quantum yield protein produced by red algae. This protein is covalently linked to streptavidin making the biotinylated nucleotides incorporated into the cDNA accessible to this fluorescent label. The fluorescent signals are measured with the same device as the ICMP panel: a 532nm laser excites the phycoerythrin, the light produced is amplified and digitized by a PMT and filtered by a digital signal processor before being sent to the PC computer for storage and analysis.

### B. Immunodiagnosis

Antibodies (discussed above) can be used in characterizing the cytokine content of various biological samples through techniques such as ELISAs and Western blotting. This may provide a screen for the presence or absence of malignancy or as a predictor of future cancer.

The use of antibodies in an ELISA assay is specifically contemplated. For example, anti-cytokine antibodies are immobilized onto a selected surface, preferably a surface exhibiting a protein affinity such as the wells of a polystyrene microtiter plate. After washing to remove incompletely adsorbed material, it is desirable to bind or coat the assay plate wells with a non-specific protein that is known to be antigenically neutral with regard to the test antisera such as bovine serum albumin (BSA), casein or solutions of powdered milk. This allows for blocking of non-specific adsorption sites on the immobilizing surface and thus reduces the background caused by non-specific binding of antigen onto the surface.

After binding of antibody to the well, coating with a non-reactive material to reduce background, and washing to remove unbound material, the immobilizing surface is contacted with the sample to be tested in a manner conducive to immune complex (antigen/antibody) formation. Following formation of specific immunocomplexes between the test sample and the bound antibody, and subsequent washing, the occurrence and even amount of immunocomplex formation may be determined by subjecting same to a second antibody having specificity for a cytokine that differs from the first antibody. Appropriate conditions preferably include diluting the sample with diluents such as BSA, bovine gamma globulin (BGG) and phosphate buffered saline (PBS)/Tween®. These added agents also tend to assist in the reduction of nonspecific background. The layered antisera is then allowed to incubate for from about 2 to about 4 hr, at temperatures preferably on the order of about 25° to about 27°C. Following incubation, the antisera-contacted surface is washed so as to remove non-immunocomplexed material. A preferred washing procedure includes washing with a solution such as PBS/Tween®, or borate buffer.

To provide a detecting means, the second antibody will preferably have an associated enzyme that will generate a color development upon incubating with an appropriate chromogenic substrate. Thus, for example, one will desire to contact and incubate the second antibody-bound surface with a urease, alkaline phosphatase, glucose oxidase, or (horseradish) peroxidase-conjugated anti-human IgG for a period of time and under conditions which favor the development of immunocomplex formation (*e.g*., incubation for 2 hr at room temperature in a PBS-containing solution such as PBS/Tween®).

After incubation with the second enzyme-tagged antibody, and subsequent to washing to remove unbound material, the amount of label is quantified by incubation with a chromogenic substrate such as urea and bromocresol purple or 2,2'-azino-di-(3-ethyl-benzthiazoline)-6-sulfonic acid (ABTS) and H₂O₂, in the case of peroxidase as the enzyme label. Quantitation is then achieved by measuring the degree of color generation, *e.g*., using a visible spectrum spectrophotometer.

The preceding format may be altered by first binding the sample to the assay plate. Then, primary antibody is incubated with the assay plate, followed by detecting of bound primary antibody using a labeled second antibody with specificity for the primary antibody.

The antibody compositions will find great use in immunoblot or Western blot analysis. The antibodies may be used as high-affinity primary reagents for the identification of proteins immobilized onto a solid support matrix, such as nitrocellulose, nylon or combinations thereof. In conjunction with immunoprecipitation, followed by gel electrophoresis, these may be used as a single step reagent for use in detecting antigens against which secondary reagents used in the detection of the antigen cause an adverse background. Immunologically-based detection methods for use in conjunction with Western blotting include enzymatically-, radiolabel-, or fluorescently-tagged secondary antibodies against the toxin moiety are considered to be of particular use in this regard.

The antibodies may also be used in conjunction with both fresh-frozen and/or formalin-fixed, paraffin-embedded tissue blocks, such as blocks prepared from a tumor biopsy, prepared for study by immunohistochemistry (IHC). The method of preparing tissue blocks from these particulate specimens has been successfully used in previous IHC studies of various prognostic factors, and/or is well known to those of skill in the art (Brown *et al.,* 1990; Abbondanzo *et al.,* 1999; Allred *et al.,* 1990).

Briefly, frozen-sections may be prepared by rehydrating 50 ng of frozen "pulverized" tissue at room temperature in phosphate buffered saline (PBS) in small plastic capsules; pelleting the particles by centrifugation; resuspending them in a viscous embedding medium (OCT); inverting the capsule and/or pelleting again by centrifugation; snap-freezing in -70°C isopentane; cutting the plastic capsule and/or removing the frozen cylinder of tissue; securing the tissue cylinder on a cryostat microtome chuck; and/or cutting 25-50 serial sections.

Permanent-sections may be prepared by a similar method involving rehydration of the 50 mg sample in a plastic microfuge tube; pelleting; resuspending in 10% formalin for 4 hours fixation; washing/pelleting; resuspending in warm 2.5% agar; pelleting; cooling in ice water to harden the agar; removing the tissue/agar block from the tube; infiltrating and/or embedding the block in paraffin; and/or cutting up to 50 serial permanent sections.

Another form of immunodiagnosis involves protein array technology, which allows high-throughput screening. Protein arrays, in this case populated by various anti-cytokine antibodies, appear as new and versatile tools in functional genomics, enabling the translation of gene expression patterns of normal and diseased tissues into protein product catalog. These arrays amy contain thousands of different antibodies spotted onto glass slides or immobilized in tiny wells, and allow one to examine the level of protein expression for a large number of proteins at once.

The basic construction of protein chips has some similarities to DNA chips, such as the use of a glass or plastic surface dotted with an array of molecules. These molecules can be DNA or antibodies that are designed to capture proteins. Defined quantities of proteins are immobilized on each spot, while retaining some activity of the protein. With fluorescent markers or other methods of detection revealing the spots that have captured these proteins, protein microarrays are being used as powerful tools in high-throughput proteomics and drug discovery.

Glass slides are still widely used, since they are inexpensive and compatible with standard microarrayer and detection equipment. However, their limitations include multiple-based reactions, high evaporation rates, and possible cross-contamination. Matrix slides offer a number of advantages, such as reduced evaporation and no possibility of cross-contamination, but they are expensive. Nanochips for proteomics have the same advantages, in addition to reduced cost and the capability of multiple-component reactions.

The earliest and best-known protein chip is the ProteinChip by Ciphergen Biosystems Inc. (Fremont, CA). The ProteinChip is based on the surface-enhanced laser desorption and ionization (SELDI) process. Known proteins are analyzed using functional assays that are on the chip. For example, chip surfaces can contain enzymes, receptor proteins, or antibodies that enable researchers to conduct protein-protein interaction studies, ligand binding studies, or immunoassays. With state-of-the-art ion optic and laser optic technologies, the ProteinChip system detects proteins ranging from small peptides of less than 1000 Da up to proteins of 300 kDa and calculates the mass based on time-of-flight (TOF).

The ProteinChip biomarker system is the first protein biochip-based system that enables biomarker pattern recognition analysis to be done. This system allows researchers to address important clinical questions by investigating the proteome from a range of crude clinical samples (*i.e*., laser capture microdissected cells, biopsies, tissue, urine, and serum.). The system also utilizes biomarker pattern software that automates pattern recognition-based statistical analysis methods to correlate protein expression patterns from clinical samples with disease phenotypes.

Some systems can perform biomarker discovery in days and validation of large sample sets within weeks. Its robotics system accessory automates sample processing, allowing hundreds of samples to be run per week and enabling a sufficient number of samples to be run, which provides high statistical confidence in comprehensive studies for marker discovery and validation.

Microfluidics is one of the most important innovations in biochip technology. Since microfluidic chips can be combined with mass spectrometric analysis, a microfluidic device has been devised in which an electrospray interface to a mass spectrometer is integrated with a capillary electrophoresis channel, an injector, and a protein digestion bed on a monolithic substrate (Wang *et al.,* 2000). This chip thus provides a convenient platform for automated sample processing in proteomics applications.

These chips can also analyze expression levels of serum proteins with detection limits comparable to commercial enzyme-linked immunosorbent assays, with the advantage that the required volume sample is markedly lower compared with conventional technologies.

Biosite (San Diego) manufactures the Triage protein chip that simultaneously measures 100 different proteins by immunoassays. The Triage protein chip immunoassays are performed in a microfluidic plastic chip, and the results are achieved in 15 minutes with picomolar sensitivities. Microfluidic fluid flow is controlled in the protein chip by the surface architecture and surface hydrophobicity in the microcapillaries. The immunoassays utilize high-affinity antibodies and a near-infrared fluorescent label, which is read by a fluorometer.

### C. Nanoscale Protein Analysis

Most current protocols including protein purification and automated identification schemes yield low recoveries that limit the overall process in terms of sensitivity and speed. Such low protein yields and proteins that can only be isolated from limited source material (*e.g*., biopsies) can be subjected to nanoscale protein analysis: a nanocapture of specific proteins and complexes, and optimization of all subsequent sample-handling steps, leading to a mass analysis of peptide fragments. This focused approach, also termed targeted proteomics, involves examining subsets of the proteome (*e.g*., those proteins that are specifically modified, bind to a particular DNA sequence, or exist as members of higher-order complexes or any combination thereof). This approach is used to identify genetic determinants of cancer that alter cellular physiology and respond to agonists.

A new detection technique called multiphoton detection, by Biotrace Inc. (Cincinnati), can quantify subzeptomole amounts of proteins and will be used for diagnostic proteomics, particularly for cytokines and other low-abundance proteins. Biotrace is also developing supersensitive protein biochips to detect concentrations of proteins as low as 5 fg/ml (0.2 attomole/ml), thereby permitting sensitivity that is 1000 times greater than current protein biochips.

### IV. Cytokine Disease Profiles

### A. Ankylosing Spondylitis

AS can be diagnosed and/or classified as mild, intermediate, or severe based on either the profile of cytokines produced within peripheral blood, serum, plasma, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe AS condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood serum, plasma, synovial fluid, cerebrospinal fluid, a tissue sample, or other body fluid sample for the cytokines listed in the attached claims. The reference level can be the median level of the cytokine found in samples derived from a population. The population can include a population of patients having ankylosing spondylitis, a population of healthy individuals, a population of patients having extra-articular involvement, or a population of patients having axial joint destruction.

A particular level of a particular cytokine can be determined to be high or low based on the levels measured from various populations. Such populations can include, without limitation, populations of patients with ankylosing spondylitis, patients with extra-articular involvement, patients with axial joint destruction, and healthy individuals.

The invention also is based on the discovery that patients presenting similar AS symptoms can have different levels of particular cytokines within their serum, plasma, synovial fluid, tissue, cerebrospinal fluid, or other body fluid samples. Thus, determining the peripheral blood, serum, plasma, synovial fluid, tissue, cerebrospinal fluid, or other body fluid cytokine profile can be used to determine the proper treatment protocol for each individual patient. For example, two patients having similar AS symptoms may have different levels of IL-10 within their serum. The patient with low levels may benefit from a treatment of IL-10 while the patient with high levels of IL-10 may benefit from treatment with IL-10 inhibitors such as anti-IL-10 antibody drugs, a class of pharmaceuticals called biological drugs. Thus, determining the cytokine profile from a patient can help provide doctors and patients with information that can be used to determine adequate treatments and measure an individual patient's response to treatment.

Another embodiment of the invention features a method for determining the predisposition of an ankylosing spondylitis patient to develop severe disease. The method includes determining the level of a cytokine (*e.g*., CCL4, CCL2, CCL11, EGF, IL-1β, IL-2, IL-5, IL-6, IL-7, CXCL8, IL-10, IL-12, IL-13, IL-15, IL-17, TNF-α, IFNγ, GM-CSF, or G-CSF) within a sample from the patient, comparing the level of the cytokine to a reference level to obtain information about the ankylosing spondylitis condition. The sample can be from peripheral blood serum, plasma, synovial fluid, tissue biopsy (*e.g*., a synovial tissue biopsy), cerebrospinal fluid, or other body fluid. The reference level can be the median level of the cytokine found in samples derived from a population. The population can include a population of patients having ankylosing spondylitis, a population of healthy individuals, a population of patients having extra-articular involvement, or a population of patients having axial joint destruction.

### B. Psoriatic Arthritis

Psoriatic arthritis (PsA) can be diagnosed and/or classified as mild, intermediate, or severe based on either the profile of cytokines produced within a serum, tissue, cerebrospinal fluid, or other body fluid sample. Such cytokines can be, without limitation, IL-1α, IIL-1β, IL-2, IL-4, IL-5, IL-6, IL-7, IL-8/CXCL8, IL-10, IL-12, IL-13, IL-15, IL-17, IL-18, INF-α, INF-β, INF-y, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe PsA condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood serum, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild PsA condition, a population of patients having a intermediate PsA condition, a population of patients having a severe PsA condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar PsA symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar PsA symptoms may have different levels of IL-10 within their serum. The patient with low levels may benefit from a treatment of IL-I0 while the patient with high levels of IL-10 may benefit from treatment with IL-10 inhibitors such as anti-IL-10 antibodies. Thus, determining the cytokine profile from a patient can help provide clinicians and patients with information that can be used to determine adequate treatments.

Another embodiment of the invention features a method for determining the predisposition of a psoriatic arthritis patient to develop severe disease. The method includes determining the level of a cytokine (*e.g.*, GM-CSF, IL-17, IL-2, IL-10, IL-13, IFN-γ, IL-6, CCL4/MIP-1β, and CCL2/MCP-1) within a sample from the patient, comparing the level of the cytokine to a reference level to obtain information about the psoriatic arthritis condition. The sample can be from peripheral blood serum, tissue biopsy (*e.g*., a synovial tissue biopsy), cerebrospinal fluid, or other body fluid. The reference level can be the median level of the cytokine found in samples derived from a population. The population can include a population of patients having a mild psoriatic arthritis condition, a population of patients having a intermediate psoriatic arthritis condition, a population of patients having a severe psoriatic arthritis condition, a population of healthy individuals, a population of patients having extra-articular involvement, or a population of patients having major joint destruction.

### C. Reactive Arthritis

Reactive arthritis (ReA) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-y, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe ReA condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild ReA condition, a population of patients having a intermediate ReA condition, a population of patients having a severe ReA condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar ReA symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar ReA symptoms may have different levels of IL-10 within their serum. The patient with low levels may benefit from a treatment of IL-10 while the patient with high levels of IL-10 may benefit from treatment with IL-10 inhibitors such as anti-IL-10 antibodies. Thus, determining the cytokine profile from a patient can help provide clinicians and patients with information that can be used to determine adequate treatments.

Another embodiment of the invention features a method for determining the predisposition of a reactive arthritis patient to develop severe disease. The method includes determining the level of a cytokine (*e.g*., IL-12, IFN-γ, IL-1β, IL-13, IL-17, CCL2/MCP-1, TNF-α, IL-4, G-CSF, and IL-6.) within a sample from the patient, comparing the level of the cytokine to a reference level to obtain information about the reactive arthritis condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population. The population can include a population of patients having a mild reactive arthritis condition, a population of patients having a intermediate reactive arthritis condition, a population of patients having a severe reactive arthritis condition, a population of healthy individuals, a population of patients having extra-articular involvement, or a population of patients having major joint destruction.

### D. Enteropathic Arthritis

Enteropathic arthritis (EA) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe EA condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild EA condition, a population of patients having a intermediate EA condition, a population of patients having a severe EA condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar EA symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar EA symptoms may have different levels of IL-10 within their serum. The patient with low levels may benefit from a treatment of IL-10 while the patient with high levels of IL-10 may benefit from treatment with IL-10 inhibitors such as anti-IL-10 antibodies. Thus, determining the cytokine profile from a patient can help provide clinicians and patients with information that can be used to determine adequate treatments.

Another embodiment of the invention features a method for determining the predisposition of a enteropathic arthritis patient to develop severe disease. The method includes determining the level of a cytokine (*e.g*., CXCL8/IL-8, IL-1β, IL-4, G-CSF, IFN-γ, and TNF-α.) within a sample from the patient, comparing the level of the cytokine to a reference level to obtain information about the enteropathic arthritis condition. The reference level can be the median level of the cytokine found in samples derived from a population. The population can include a population of patients having a mild enteropathic arthritis condition, a population of patients having an intermediate enteropathic arthritis condition, a population of patients having a severe enteropathic arthritis condition, a population of healthy individuals, a population of patients having extra-articular involvement, or a population of patients having major joint destruction.

### E. Ulcerative Colitis

Ulcerative Colitis (UC) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-y, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe UC condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild UC condition, a population of patients having a intermediate UC condition, a population of patients having a severe UC condition, or a population of healthy individuals.

A particular level of a particular cytokine can be determined to be high or low based on the levels measured from various populations. Such populations can include, without limitation, populations of patents with a mild condition, intermediate condition, or severe condition, and healthy individuals.

The invention also is based on the discovery that patients presenting similar UC symptoms can have different levels of particular cytokines within their peripheral blood, serum, plasma, tissue, cerebrospinal fluid, or other body fluid samples. Thus, determining the serum, tissue, or cerebrospinal fluid cytokine profile can be used to determine the proper treatment protocol. For example, two patients having similar UC symptoms may have different levels of IL-10 within their serum. The patient with low levels may benefit from a treatment of IL-10. Thus, determining the cytokine profile from a patient can help provide clinicians and patients with information that can be used to determine adequate treatments.

Another embodiment of the invention features a method for determining the predisposition of a ulcerative colitis patient to develop severe disease. The method includes determining the level of a cytokine (*e.g*., IL-7, CXCL8/IL-8, IFN-γ, TNF-α, and IL-1β) within a sample from the patient, comparing the level of the cytokine to a reference level to obtain information about the psoriatic arthritis condition and determining if the patient is predisposed to develop severe disease based on the information. The sample can be from peripheral blood, serum, plasma, tissue biopsy (*e.g*., a colon tissue biopsy), cerebrospinal fluid, or other body fluid. The reference level can be the median level of the cytokine found in samples derived from a population. The population can include a population of patients having mild ulcerative colitis, a population of patients having an intermediate ulcerative colitis, a population of patients having severe ulcerative colitis, or a population of healthy individuals.

### F. Crohn's Disease

Crohn's disease (CD) can be diagnosed and/or classified as mild, intermediate, or severe based on either the profile of cytokines produced within peripheral blood, serum, plasma, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe CD condition placing the patient at a high risk for colorectal cancer. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, EL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCLA, MCP-1/CCL2, RANTES/CCL5, Eotaxin/CCL11, VEGF, EGF, or FGF. The reference level can be the median level of the cytokine found in samples derived from a population. The population can include a population of patients having a mild CD condition, a population of patients having a intermediate CD condition, a population of patients having a severe CD condition, or a population of healthy individuals.

A particular level of a particular cytokine can be determined to be high or low based on the levels measured from various populations. Such populations can include, without limitation, populations of patents with a mild condition, intermediate condition, or severe condition, and healthy individuals.

The invention also is based on the discovery that patients presenting similar CD symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar CD symptoms may have different levels of IL-10 within their serum or tissue. The patient with low levels may benefit from a treatment of IL-10. Thus, determining the cytokine profile from a patient can help provide clinicians and patients with information that can be used to determine adequate treatments.

Another embodiment of the invention features a method for determining the predisposition of a Crohn's disease patient to develop severe disease. The method includes determining the level of a cytokine (*e.g*., TNF-α, IFN-γ, IL-1β, IL-6, IL-7, IL-13, IL-2, IL-4, GM-CSF, G-CSF, CCL2/MCP-1, and CXCL8/IL-8) within a sample from the patient, comparing the level of the cytokine to a reference level to obtain information about the severity of Crohn's disease in the patient. The sample can be from peripheral blood, serum, plasma, tissue biopsy (*e.g*., colon tissue biopsy), or other body fluid. The reference level can be the median level of the cytokine found in samples derived from a population. The population can include a population of patients having mild Crohn's disease, a population of patients having intermediate Crohn's disease, a population of patients having a severe Crohn's disease, or a population of healthy individuals.

### G. Rheumatoid Arthritis

Rheumatoid arthritis (RA) can be diagnosed and/or classified as mild, intermediate, or severe based on either the profile of cytokines produced within peripheral blood, serum, plasma, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe RA condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild RNA condition, a population of patients having a intermediate RA condition, a population of patients having a severe RA condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar RA symptoms can have different levels of particular cytokines within their peripheral blood, serum, plasma, tissue, cerebrospinal fluid, or other body fluid samples. Thus, determining the serum, tissue, or cerebrospinal fluid cytokine profile can be used to determine the proper treatment protocol. For example, two patients having similar RA symptoms may have different levels of IL-10 within their serum. The patient with low levels may benefit from a treatment of IL-10 while the patient with high levels of IL-10 may benefit from treatment with IL-10 inhibitors such as anti-IL-10 antibodies. Thus, determining the cytokine profile from a patient can help provide clinicians and patients with information that can be used to determine adequate treatments.

Another embodiment of the invention features a method for determining the predisposition of a rheumatoid arthritis patient to develop severe disease. The method includes determining the level of a cytokine (*e.g*., IFN-γ, IL-1β, TNF-α, G-CSF, GM-CSF, IL-6, IL-4, IL-10, IL-13, IL-5, and IL-7) within a sample from the patient, comparing the level of the cytokine to a reference level to obtain information about the rheumatoid arthritis condition. The sample can be from peripheral blood, serum, plasma, tissue biopsy (*e.g.,* a synovial tissue biopsy), cerebrospinal fluid, or other body fluid. The reference level can be the median level of the cytokine found in samples derived from a population. The population can include a population of patients having a mild rheumatoid arthritis condition, a population of patients having a intermediate rheumatoid arthritis condition, a population of patients having a severe rheumatoid arthritis condition, a population of healthy individuals, a population of patients having extra-articular involvement, or a population of patients having major joint destruction.

### H. Systemic Lupus Erythematosus

Systemic lupus erythematosus (SLE) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1P/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe SLE condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-10, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild SLE condition, a population of patients having a intermediate SLE condition, a population of patients having a severe SLE condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar SLE symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar SLE symptoms may have different levels of IL-10 within their serum. The patient with low levels may benefit from a treatment of 1L-10 while the patient with high levels of IL-10 may benefit from treatment with IL-10 inhibitors such as anti-IL-10 antibodies. Thus, determining the cytokine profile from a patient can help provide clinicians and patients with information that can be used to determine adequate treatments.

Another embodiment of the invention features a method for determining the predisposition of the systemic lupus erythematosus patient to develop severe disease. The method includes determining the level of a cytokine (*e.g*., IL-10, IL-2, IL-4, IL-6, IFN-γ, CCL2/MCP-1, CXCL8/IL-8, and IL-17.) within a sample from the patient, comparing the level of the cytokine to a reference level to obtain information about the SLE condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population. The population can include a population of patients having a mild systemic lupus erythematosus condition, a population of patients having an intermediate systemic lupus erythematosus condition, a population of patients having a severe systemic lupus erythematosus condition, a population of healthy individuals, a population of patients having extra-articular involvement, or a population of patients having major joint destruction.

### I. Familial Mediterranean Fever

Familial Mediterranean fever (FMF) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, EotaxWCCL11, VEGF, EGF, or FGF.

If the cytokine profiles indicate that a patient has severe disease, then that patient also can be classified as being predisposed to develop a severe FMF condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood serum, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF The reference level can be the median level of the cytokine found in samples derived from a population. The population can include a population of patients having a mild FMF condition, a population of patients having a intermediate FMF condition, a population of patients having a severe FMF condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention also is based on the discovery that patients presenting similar FMF symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar FMF symptoms may have different levels of IL-8 within their serum. A patient with high levels of IL-8 may benefit from treatment with IL-8 antagonists. Thus, determining the cytokines profile from a patient can help provide clinicians and patients with information that can be used to determine adequate treatments.

Another embodiment of the invention features a method for determining the predisposition of a familial Mediterranean fever patient to develop severe disease. The method includes determining the level of a cytokine (*e.g*., IL-17, IL-6, CCL2/MCP-1, TNF-αINF-γ, GM-CSF, IL-13, IL-4, G-CSF, and CXCL8/IL-8) within a sample from the patient, comparing the level of the cytokine to a reference level to obtain information about the familial Mediterranean fever condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population. The population can include a population of patients having a mild familial Mediterranean fever condition, a population of patients having an intermediate familial Mediterranean fever condition, a population of patients having a severe familial Mediterranean fever condition, a population of healthy individuals, a population of patients having extra-articular involvement, or a population of patients having major joint destruction.

### J. Amyotrophic Lateral Sclerosis

Amyotrophic Lateral Sclerosis (ALS) can be diagnosed by cerebrospinal fluid, other body fluid, or tissue samples. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

Specifically, the invention involves analyzing cerebrospinal fluid, other body fluid or tissue samples for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. A particular level of a particular cytokine can be determined to be high or low based on the levels measured from various populations.

The invention also is based on the discovery that patients presenting similar ALS symptoms can have different levels of particular cytokines within their cerebrospinal fluid, other body fluid, or tissue samples. Thus, determining the cerebrospinal fluid, body fluid, or tissue samples cytokine profile can be used to determine the proper treatment protocol. For example, two patients having similar ALS symptoms may have different levels of IL-12 within their serum. A patient with low levels of IL-12 may benefit from treatment with IL-12 agonists. Thus, determining the cytokine profile from a patient can help provide clinicians and patients with information that can be used to determine adequate treatments.

### K. Irritable Bowel Syndrome

Irritable Bowel Syndrome (SLE) can be diagnosed and/or classified as mild, intermediate, or severe based on either the profile of cytokines produced within a serum, tissue, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe gastrointestinal condition including major organ involvement with an increased risk in gastrointestinal cancers.

Specifically, the invention involves analyzing peripheral blood, serum, plasma, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild IBS condition, a population of patients having a intermediate IBS condition, a population of patients having a severe IBS condition, or a population of healthy individuals.

A particular level of a particular cytokine can be determined to be high or low based on the levels measured from various populations. Such populations can include, without limitation, populations of patents with a mild condition, intermediate condition, or severe condition, and patients with extra-gatrointestinal immune system involvement.

The invention is also based on the discovery that patients presenting similar IBS symptoms can have different levels of particular cytokines within their serum, tissue, or other body fluid samples. Thus, determining the serum, tissue, or other fluid cytokine profile can be used to determine the proper treatment protocol. For example, two patients having similar IBS symptoms may have different levels of CXCL8/IL-8 within their serum. The patient with high levels may benefit from a treatment of an anti-CXCL8/IL-8 drug or biologic while the patients with low levels of CXCL8/IL-8 may benefit from less aggressive treatment alternatives. Thus, determining the cytokine profile from a patient can help provide clinicians and patients with information that can be used to determine adequate treatments.

Another embodiment of the invention features a method for determining the predisposition of the IBS patient to develop severe disease. The method includes determining the level of a cytokines (*e.g*., TNF-α, IFN-γ, IL-1β, IL-6, IL-7, GM-CSF, G-CSF, CCL2/MCP-1, and CXCL8/IL-8) within a sample from the patient, comparing the level of the cytokine to a reference level to obtain information about the IBS condition. The sample can be from peripheral blood, serum, plasma, tissue biopsy (*e.g*., colonic tissue biopsy), or other body fluid. The reference level can be the median level of the cytokine found in samples derived from a population. The population can include a population of patients having a mild IBS condition, a population of patients having an intermediate IBS condition, a population of patients having a severe IBS condition, or a population of healthy individuals.

### L. Juvenile Rheumatoid Arthritis

Juvenile Rheumatoid Arthritis (JRA) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe JRA condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild JRA condition, a population of patients having a intermediate JRA condition, a population of patients having a severe JRA condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention is also based on the discovery that patients presenting similar JRA symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar JRA symptoms may have different levels of IL-1β within their serum. The patient with high levels of 1L-1β may benefit from treatment with IL-1β inhibitors. Thus, determining the cytokine profile from a patient can help provide clinicians and patients with information that can be used to determine adequate treatments.

Another embodiment of the invention features a method for determining the predisposition of a JRA patient to develop severe disease. The method includes determining the level of a cytokine (*e.g*., IFN-γ, IL-1β, TNF-α, G-CSF, GM-CSF, IL-6, IL-4, IL-10, IL-13, IL-5, and IL-7) within a sample from the patient, comparing the level of the cytokine to a reference level to obtain information about the JRA condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population. The population can include a population of patients having a JRA condition, a population of patients having a intermediate JRA condition, a population of patients having a severe JRA condition, a population of healthy individuals, a population of patients having extra-articular involvement, or a population of patients having major joint destruction.

### M. Sjogren's Syndrome

Sjogren's Syndrome (SS) can be diagnosed and/or classified based on the profile of cytokines produced within a serum, tissue, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

Specifically, the invention involves analyzing peripheral blood, serum, plasma, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild SS condition, a population of patients having a intermediate SS condition, a population of patients having a severe SS condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention is also ibased on the discovery that patients presenting similar SS symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar SS symptoms may have different levels of IL-2 within their serum. patient with high levels of IL-2 may benefit from treatment with IL-2 inhibitors such as anti-IL-2 biological drugs. Thus, determining the cytokine profile from a patient can help provide clinicians and patients with information that can be used to determine adequate treatments.

Another embodiment of the invention features a method for determining the predisposition of the SS patient to develop severe disease. The method includes determining the level of a cytokine (*e.g*., CCL2/MCP-1, IL-12, CXCL8/IL-8, CCL11/Eotaxin, TNFα, IL-2, IFNα, IL-15, IL17, IL-1α, IL-1β, IL-6, and GM-CSF) within a sample from the patient, comparing the level of the cytokine to a reference level to obtain information about the SS condition. The sample can be from peripheral blood, serum, plasma, tissue biopsy (*e.g*., a salivary gland or lip tissue biopsy), or other body fluid. The reference level can be the median level of the cytokine found in samples derived from a population.

### N. Early Arthritis

Early Arthritis (EArth) can be diagnosed and/or classified as mild, intermediate, or severe based on the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe EArth condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild EArth condition, a population of patients having a intermediate EArth condition, a population of patients having a severe EArth condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition, patients with extra-articular involvement, patients with major joint destruction, and healthy individuals.

The invention is also based on the discovery that patients presenting similar EArth symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar EArth symptoms may have different levels of IL-6 within their serum. The patient with high levels of IL-6 may benefit from treatment with IL-6 inhibitors. Thus, determining the cytokine profile from a patient can help provide clinicians and patients with information that can be used to determine adequate treatments.

Another embodiment of the invention features a method for determining the predisposition of the EArth patient to develop severe disease. The method includes determining the level of a cytokines (*e.g*., IL-6. IL-2, IL-12, IFNγ, GM-CSF, IL-5, IL-4, CCL4/MIP-1β, and CXCL8/IL-8) within a sample from the patient, comparing the level of the cytokine to a reference level to obtain information about the psoriatic arthritis condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population.

### O. Psoriasis

Psoriasis (PSO) can be diagnosed and/or classified as mild, intermediate, or severe based on either the profile of cytokines produced within a skin, serum, tissue, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe PSO-condition including major organ involvement and major joint destruction. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild PSO condition, a population of patients having a intermediate PSO condition, a population of patients having a severe PSO condition, a population of healthy individuals, a population of patients having extra-articular involvement, or a population of patients having major joint destruction.

A particular level of a particular cytokine can be determined to be high or low based on the levels measured from various populations. Such populations can include, without limitation, populations of patents with a mild condition, intermediate condition, or severe condition.

The invention is also based on the discovery that patients presenting similar PSO symptoms can have different levels of particular cytokines within their skin, serum, tissue, or other body fluid samples. Thus, determining the skin, serum, tissue, or cytokine profile can be used to determine the proper treatment protocol. For example, two patients having similar PSO symptoms may have different levels of IL-6 within their serum. The patient with high levels of IL-6 may benefit from treatment with IL-6 inhibitors. Thus, determining the cytokine profile from a patient can help provide clinicians and patients with information that can be used to determine adequate treatments.

Another embodiment of the invention features a method for determining the predisposition of the PSO patient to develop severe disease. The method includes determining the level of a cytokine (*e.g*., IL-6, IL-10, IL-2, IL-4, IFN-γ, CCL2/MCP-1, and IL-17.) within a sample from the patient, comparing the level of the cytokine to a reference level to obtain information about the PSO condition. The sample can be from peripheral blood, serum, plasma, skin, tissue biopsy, or other body fluid. The reference level can be the median level of the cytokine found in samples derived from a population.

### P. Neuroinflammation

Neuroinflammation (NI) can be diagnosed and/or classified as mild, intermediate, or severe based on either the profile of cytokines produced within a serum, tissue, cerebrospinal fluid, or other body fluid sample. Such cytokines can be, without limitation, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF.

If the cytokine profiles indicate that a patient has severe disease, then that patient also can be classified as being predisposed to develop a severe NI condition including central nervous system involvement and peripheral nervous system involvement. If the cytokine profile indicates that a patient has mild or intermediate disease, then further analysis can be performed to determine that patient's predisposition to develop severe disease.

Specifically, the invention involves analyzing peripheral blood, serum, plasma, cerebrospinal fluid, a tissue sample, or other body fluid sample for particular cytokines such as, IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The reference level can be the median level of the cytokine found in samples derived from a population. The population can include a population of patients having a mild N1 condition, a population of patients having a intermediate NI condition, a population of patients having a severe NI condition, a population of healthy individuals, a population of patients having a central nervous system condition, or a population of patients having a peripheral nervous system condition.

A particular level of a particular cytokine can be determined to be high or low based on the levels measured from various populations. Such populations can include, without limitation, populations of patents with a mild condition, intermediate condition, or severe condition, a population of patients having a central nervous system condition, or a population of patients having a peripheral nervous system condition.

The invention is also based on the discovery that patients presenting similar NI symptoms can have different levels of particular cytokines within their serum, tissue, cerebrospinal fluid, or other body fluid samples. Thus, determining the serum, tissue, or cerebrospinal fluid cytokine profile can be used to determine the proper treatment protocol. For example, two patients having similar NI symptoms may have different levels of CCL2/MCP-1 within their cerebrospinal fluid. The patient with high levels of CCL2/MCP-1 may benefit from treatment with CCL2/MCP-1inhibitors. Thus, determining the cytokine profile from a patient can help provide clinicians and patients with information that can be used to determine adequate treatments.

Another embodiment of the invention features a method for determining the predisposition of the NI patient to develop severe disease. The method includes determining the level of a cytokine (*e.g*., CCL2/MCP-1, IL-12, GM-CSF, G-CSF, M-CSF, IL-6, and IL-17.) within a sample from the patient, comparing the level of the cytokine to a reference level to obtain information about the NI condition. The sample can be from peripheral blood, serum, plasma, tissue biopsy (*e.g*., a synovial tissue biopsy), cerebrospinal fluid, or other body fluid. The reference level can be the median level of the cytokine found in samples derived from a population.

### V. Examples

Specific embodiments of the invention will now be further described by the following, nonlimiting examples which will serve to illustrate in some detail various features. The following examples are included to facilitate an understanding of ways in which the invention may be practiced. It should be appreciated that the examples which follow represent embodiments discovered to function well in the practice of the invention, and thus can be considered to constitute preferred modes for the practice of the invention. However, it should be appreciated that many changes can be made in the exemplary embodiments which are disclosed.

### Example 1: Distinct Cytokine Patterns in Ankylosing Spondylitis (AS)

A distinct profile of cytokines was generated from patients with ankylosing spondylitis (AS). This cytokine profile was determined by sampling peripheral blood serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum was obtained from more than 44 patients with active ankylosing spondylitis who fulfilled the diagnostic criteria and classification by the European Spondylarthropathy Study Group (ESSG) - Bath Criteria (Calin, A., J. D. Taurog., Eds. 1998, "The Spondylarthritides.")

### 2. Cytokine Measurement

The samples were analyzed using a comprehensive biometric multiplex cytokine assay (BioSource, Camarillo, CA, USA). This assay quantitates 28 cytokines in a single 50-microliter sample, including IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, or FGF. The assay is a bead-based fluorescent multiplex protein analysis system with a Luminex-100 analyzer (Luminex Corporation, Austin, TX, USA).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) was used for selection of the set of cytokines with maximal discriminatory capabilities between groups of samples from patients and unaffected controls. We used variant DFA named the Forward Stepwise Analysis.

In stepwise DFA, the model for discrimination is built step-by-step. Specifically, at each step we review all variables and evaluate which one will contribute most to the discrimination between groups. This variable is then included in the model, and the process proceeds to the next step. The stepwise procedure is "guided" by the respective F to enter values. The F value for a variable indicates its statistical significance in the discrimination between groups, that is, it is a measure of the extent to which a variable makes a unique contribution into the discriminative functions - roots, which are linear combinations of the gene expressions with constant coefficients and used for the prediction of group membership. In general, we continue to choose variables to be included in the model, as long as the respective F values for those variables are larger than the user-specified F to enter.

The statistical significance of discriminative power of each cytokine can also characterized by partial Wilk's Lambda - equivalent to the partial correlation coefficient in multiple regression analysis. Wilk's Lambda is a ratio of within group difference to the sum of within plus between group differences (standard deviations). Its value ranges from 1.0 (no discriminatory power) to 0.0 (perfect discriminatory power).

DFA was carried out with use of the package Statistica (StatSoft, Tulsa OK). This software produces list of cytokines having maximal discriminatory capability and roots - linear combinations of these cytokines having similar within group values and different for each group. The discriminant potential of the final equations can be observed in a simple multi-dimensional plot of the values of the roots obtained for each group. This provides a graphical representation of the similarity among the various groups.

Variant of cluster analysis with the Pearson correlation coefficient as a measure of similarity and the threshold r = 0.8 was used for further characterization of samples heterogeneity. Data obtained from AS patients, HLA B27 positive unaffected controls, and HLA B27 negative unaffected controls were analyzed with these methods.

### 4. Results

The ankylosing spondylitis patients shared similar cytokine profiles. MHC Class I HLA-B27 positive healthy controls also shared distinct cytokine characteristics with patients, but not with HLA-B27 negative controls, suggesting that HLA-B27 plays a principal role in both the etiology and pathophysiology of spondyloarthritic disease. Ankylosing spondylitis (AS) has a strong genetic association with the MHC I - HLA-B27 locus. However, the role of HLA-B27 in AS pathophysiology remains controversial.

Using a comprehensive multiplex cytokine assay we found a specific set of cytokines that are upregulated in the peripheral blood of ankylosing spondylitis patients including: CCL4/MIP-1β, IL-17, IL-6, CCL2/MCP-1, TNF-α, IFN-γ, GM-CSF, IL-13, IL-4, G-CSF, and CXCL8/IL-8. HLA-B27 positive healthy controls also had elevated IFN-γ (p=0.07) and IL-8 (p=0.08) levels compared to HLA-B27 negative controls, suggesting that HLA-B27 may function to predispose patients to disease. Levels of IL-1β were not significantly elevated in AS patients, even when levels of TNF-α and IFN-γ were significantly elevated. The cytokines upregulated in these patients are unique to AS (this group was not characteristic of other spondyloarthropathy subtypes, rheumatoid arthritis, or SLE), suggesting this complex immune activity specifically induces spondylitis and/or sacroilitis. Moreover, cytokine titers correlated with disease activity, suggesting that this group of cytokines can be used to guide biologic therapy and as a surrogate of disease activity in AS.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. The inventors used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of psoriatic arthritis. Immune deviation is now widely accepted as a concept in explaining the pathogenesis of autoimmune diseases. This model implies that chronic inflammation is a consequence of the aberrant commitment to an immune pathway in response to a given antigen (Finkelman F D, J Exp Med, pp.182:279-282, 1995).

Psoriatic arthritis (PsA) is a chronic systemic inflammatory rheumatic disorder of the axial skeleton with extraskeletal manifestations, primarily psoriasis. Mechanisms underlying the phenotypic heterogeneity of psoriatic arthritis are not known. To explore whether a molecular diagnostic characteristic exists, serum samples from 72 patients with clinically diagnosed PsA were examined and the concentration of cytokines were quantified. Based upon the quantative values of cytokines, the samples were categorized into three distinct subsets. Seventy-two were characterized. In all specimens IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. The cytokines elevated in PsA patients include GM-CSF, IL-17, IL-2, IL-10, IL-13, IFN-γ, IL-6, CCL4/MIP-1β, and CCL3/MCP-1.

### Example 3: Distinct Cytokine Patterns in Reactive Arthritis (ReA)

A distinct profile of cytokines was produced from patients with reactive arthritis (ReA). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum from 13 patients diagnosed with reactive arthritis were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokine were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of reactive arthritis. Immune deviation is now widely accepted as a concept in explaining the pathogenesis of autoimmune diseases (Finkelman F D, J Exp Med, pp.182:279-282 (1995).

Reactive arthritis (ReA) is a chronic systemic inflammatory rheumatic disorder of e axial skeleton with or without extraskeletal manifestations. Mechanisms underlying the phenotypic heterogeneity of reactive arthritis are not known, but bacterial and viral antigens are suspected to contribute to disease etiology. To explore whether a molecular diagnostic characteristic exists, serum samples from 13 patients with clinically diagnosed ReA were examined and the concentration of cytokines were quantified.

In all specimens IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. Each of the defined variants of ReA displayed a unique cytokine profile, although all of the variants shared a cytokine profile that was unique to ReA. The cytokines elevated in ReA patients include IL-12(p70), IFN-γ, IL-1β, IL-13, IL-17, CCL2/MCP-1, TNF-α, IL-4, G-CSF, and IL-6.

### Example 4: Distinct Cytokine Patterns in Enteropathic Arthritis (EA)

A distinct profile of cytokines was produced from patients with enteropathic arthritis (EA). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum from 12 patients diagnosed with enteropathic arthritis were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of EA. Immune deviation is now widely accepted as a concept in explaining the pathogenesis of autoimmune diseases (FD Finkelman, J Exp Med, pp.182:279-282, 1995).

Enteropathic arthritis is a chronic systemic inflammatory rheumatic disorder of the axial skeleton with or without extraskeletal manifestations. Mechanisms underlying the phenotypic heterogeneity of enteropathic arthritis are not known, but gastrointestinal bacterial antigens are suspected to play an important role in the development of the disease. To explore whether a molecular diagnostic characteristic exists, serum samples from 12 patients with clinically diagnosed with enteropathic arthritis were examined and the concentration of cytokines were quantified.

In all specimens IL-1α, IL-1β, IL-2, IL-4, IL-5, IL-6, IL-7, IL-8/CXCL8, I-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. Each of the defined variants of enteropathic arthritis displayed a unique cytokine profile, although all of the variants shared a cytokine profile that was unique to EA. The cytokines elevated in EA patients include: IL-8, IL-1β, IL-4, G-CSF, IFN-γ, and TNF-α.

### Example 5: Distinct Cytokine Patterns in Ulcerative Colitis (UC)

A distinct profile of cytokines was produced from patients with Ulcerative Colitis (UC). This distinct cytokines profile was determined by sampling serum for the presence of cytokines. Patients were found to have predictive molecular cytokines profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Tissue biopsy samples from 10 patients diagnosed with ulcerative colitis (UC) were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. The inventors used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of UC.

Ulcerative colitis is a chronic systemic inflammatory disorder of the gastrointestinal system with or without extraintestinal manifestations. Mechanisms underlying the phenotypic heterogeneity of irritable bowel disease are not known. To explore whether a molecular diagnostic characteristic exists, tissue biopsy samples from 10 patients with clinically diagnosed UC were examined and the concentration of cytokines were quantified.

In all specimens EL-1α, IL,-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. The cytokines elevated in UC patients include IL-7, CXCL8/IL-8, IFN-γ, TNF-α, and IL-1β.

### Example 6: Distinct Cytokine Patterns in Crohn's Disease (CD)

A distinct profile of cytokines was produced from patients with Crohn's Disease (CD). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Tissue biopsy samples from 9 patients diagnosed with Crohn's disease were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (describe above) was used for selection of the set of cytokines with maximal discriminatory capabilities between groups of samples from patients and unaffected controls. The inventors used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of CD. Immune deviation is now widely accepted as a concept in explaining the pathogenesis of autoimmune diseases.

Crohn's Disease (CD) is a chronic systemic inflammatory rheumatic disorder of the colon with or without extracolonic manifestations. Mechanisms underlying the phenotypic heterogeneity of Crohn's Disease are not known. To explore whether a molecular diagnostic characteristic exists, serum samples from 9 patients with clinically diagnosed CD were examined and the concentration of cytokines were quantified.

In all specimens IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. The cytokines elevated in CD patients include: TNF-α, IFN-γ, IL-1β, IL-6, IL-7, IL-13, IL-2, I-4, GM-CSF, G-CSF, CCL2/MCP-1, and CXCL8/IL-8.

### Example 7: Distinct Cytokine Patterns in Rheumatoid Arthritis (RA)

A distinct profile of cytokines was produced from patients with Rheumatoid Arthritis (RA). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum from 29 patients diagnosed with rheumatoid arthritis were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.
Discriminant function analysis (DFA) (described above) was used for selection of the set of cytokines with maximal discriminatory capabilities between groups of samples from patients and unaffected controls. The inventors used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of RA. Immune deviation is now widely accepted as a concept in explaining the pathogenesis of autoimmune diseases.

Rheumatoid arthritis is a chronic systemic inflammatory rheumatic disorder with or without extraskeletal manifestations. Mechanisms underlying the phenotypic heterogeneity of rheumatoid arthritis are not known. To explore whether a molecular diagnostic characteristic exists, serum samples from 29 patients with clinically diagnosed RA were examined and the concentration of cytokines were quantified.

In all specimens IL-1α, IL-1β, IL-2, IL-4, IL-5, IL-6, IL-7, IL-8/CXCL8, TL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. Each of the defined variants of RA displayed a unique cytokine profile, although all of the variants shared a cytokine profile that was unique to RA. The cytokines elevated in RA patients include IFN-γ, IL-1β, TNF-α, G-CSF, GM-CSF, IL-6, IL-4, IL-10, IL-13, IL-5, and IL-7. IL-2, CXCL8/IL-8, IL-12, and CCL2/MCP-1 were not significantly elevated in RA patients.

### Example 8: Distinct Cytokine Patterns in Systemic Lupus Erythematosus (SLE)

A distinct profile of cytokines was produced from patients with systemic lupus erythematosus (SLE). This distinct cytokine profile was determined by sampling serum for the presence of cytokines. Patients were found to have predictive molecular cytokine profiles based on clinical disease phenotype and disease severity. Specifically, cytokine were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum from twenty-two patients with SLE and twelve normal controls were used in this study. Among the SLE samples, 5 had anti-Ro 60 and anti-La antibodies, 4 had anti-Sm and anti-nRNP antibodies, 4 had anti-P antibodies, 3 had anti-dsDNA antibodies and 6 had anti-APL antibodies. Two SLE patients, one who developed anti-Ro over time and another who had autoantibodies at the time of SLE diagnosis, were observed longitudinally for a period of 13 years.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of SLE. Immune deviation is now widely accepted as a concept in explaining the pathogenesis of autoimmune diseases.

Systemic Lupus Erythematosus (SLE) is a chronic systemic inflammatory rheumatic disorder with or without major organ involvement. Mechanisms underlying the phenotypic heterogeneity of SLE are not known. To explore whether a molecular diagnostic characteristic exists, serum samples from 22 patients with clinically diagnosed SLE were examined and the concentration of cytokines were quantified.

In all specimens IL-1β, IL-2, IL-4, IL-5, IL-6, IL-7, IL-8, IL 10, EL-12(p70), IL-13, IL-17, IFN-γ, TNF-α, GM-CSF, G-CSF, MCP-1, and MIP-1β were analyzed using a comprehensive biometric multiplex cytokine assay. The various subsets of SLE shared a distinctive cytokine profile compared to normal controls. Values above the mean control values plus three standard deviations for each cytokine were considered as elevated. Patients with anti-Ro 60 and anti-La had elevated levels of all the cytokines studied, except for IL-5, IL-12 and MIP-1β, with IFN-γ increasing to 245-fold. Patients with APL antibodies had increased levels of all cytokines studied, except for IL-5, IL-7, IL-12 and G-CSF. Patients with anti-dsDNA antibodies, anti-P antibodies or anti-Sm/RNP antibodies had elevated levels of IL-8, IL-10, GM-CSF, IFN-γ and MCP-1. In the patient who developed anti-Ro under longitudinal observation, it was observed that the cytokines IL-2, IL-6, IL-8 and IFN-γ spiked when the patient developed anti-Ro. However, IL-17 and MCP-1 was found to be elevated almost through the entire period of observation in this patient while G-CSF dropped after the initial 7 years to normal levels. On the contrary another patient who had autoantibodies at the time of SLE diagnosis had all cytokines elevated, except for IL-10, GM-CSF, IL-5, IL-7, IL-12, IL-13 and MIP-1β for an initial period of 7 years after which levels dropped to normal.

Pro-inflammatory cytokines and the anti-inflammatory cytokine IL-10 were elevated in all subsets of lupus patients. Only cytokines IL-2, IL-4, IL-6, IFN-γ, and MCP-1 had markedly elevated values in patients with anti-Ro 60 and anti-La antibodies compared to other subsets. Anti-Ro subset had the highest IL-6 and IFN-γ levels. IL-8, IL-17 and MCP-1 were high in all lupus subsets studied. However, IL-5 and IL-12 were low in all lupus subsets. Both Th₁ and Th₂ type responses were observed in each sub-set.

### Example 9: Distinct Cytokine Patterns in Familial Mediterranean Fever (FMF)

A distinct profile of cytokines was produced from patients with Familial Mediterranean Fever (FMF). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

Peripheral blood serum from five patients diagnosed with Familial Mediterranean Fever were analyzed.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using a standard Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of FMF. Immune deviation is now widely accepted as a concept in explaining the pathogenesis of autoimmune diseases.

Familial Mediterranean Fever is an inherited disorder usually characterized by recurrent episodes of fever and peritonitis (inflammation of the abdominal membrane). In 1997, researchers identified the gene for FMF and found several different gene mutations that cause this inherited rheumatic disease. The gene, found on chromosome 16, codes for a protein that is found almost exclusively in granulocytes - white blood cells important in the immune response. The protein is likely to normally assist in keeping inflammation under control by deactivating the immune response - without this 'brake,' an inappropriate full-blown inflammatory reaction occurs: an attack of FMF. To explore whether a molecular diagnostic cytokine characteristic exists, serum samples from five patients with clinically diagnosed FMF were examined and the concentration of cytokines were quantified.

In all specimens IL-1α, IL-1β, IL-2, IL-4, IL-5, IL-6, IL-7, IL-8/CXCL8, IL-10, IL-12, IL-13, IL-15, IL-17, IL-18, INF-α, INF-β, INF-γ, TNF-α, GM-CSF, G-CSF, MIP-1α/CCL3, MIP-1β/CCL4, MCP-1/CCL2, RANTES/CCL5, Eotaxin/CCL11, VEGF, EGF, and FGF were analyzed using a comprehensive biometric multiplex cytokine assay. Each of the defined variants of RA displayed a unique cytokine profile, although all of the variants shared a cytokine profile that was unique to FMF. The cytokines elevated in FMF patients include: G-CSF, IL-2, IFN-γ, TNF-α, IL-1 and CXCL8/IL-8.

### Example 10: Distinct Cytokine Patterns in Amyotrophic Lateral Sclerosis (ALS)

A distinct profile of cytokine was produced from patients with Amyotrophic Lateral Sclerosis (ALS). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

A multiplex suspension array technology was used to quantify 28 cytokines and chemokines in archived CSF from 15 patients with intermediate stage ALS, and from 15 age-matched patients who were not suffering from known neurological disease.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The data presented herein indicate the contribution of different cytokines in controlling inflammation and also emphasize the necessity to re-evaluate the role of the immune pathways in the pathogenesis of ALS. Immune deviation is now widely accepted as a concept in explaining the pathogenesis of autoimmune diseases.

The neuroinflammatory hypothesis contends that dysregulated cytokine networks contribute to neuropathology in amyotrophic lateral sclerosis (ALS) and other neurodegenerative diseases. Recent findings indicate that cytokine expression changes correlate with disease onset and progression in mouse models of familial amyotrophic lateral sclerosis (ALS). A study was undertaken to determine whether similar cytokine alterations could be observed in cerebrospinal fluid (CSF) obtained from humans with ALS. A multiplex suspension array technology was used to quantify 17 cytokines and chemokines in archived CSF from 15 patients with intermediate stage ALS, and from 15 age-matched patients who were not suffering from known neurological disease.

Additionally, enzyme-linked immunosorbent assays (ELISAs) were employed to measure inflammatory molecules prostaglandin E₂ (PGE₂), leukotriene B₄ (LTB₄) and the apoptosis biomarker cleaved tau (c-tau) in the same CSF samples. Mean levels of all analytes except IL-12 were increased in the ALS group relative to non-afflicted subjects; mean IL-12 decreased by 50% in the ALS group. Macrophage inflammatory protein 1-beta (MIP-1β) was significantly increased at the 95% confidence level while IL-13 and IL-8 were significant at the 90% level. As a fraction of the total cytokine pool, IL-13 concentrations tended to be elevated (p<0.07) and IL-12 were decreased (p<0.08) in the ALS group. Consequently, the ratio of IL-13 / IL-12 increased 4-fold in ALS (p<0.01). These data suggest an alteration in the Th1 / Th2 cytokine blance, and suggest that multiplex cytokine suspension arrays may be useful for indexing neurological disease in human clinical studies.

The results strongly suggest a broad-spectrum dysregulation of cytokine components in the intermediate stages of human sporadic ALS. In particular, four proteins (MIP-1β, IL-8, IL-12p70 and IL-13) are especially notable analytes that differ between ALS and non-diseased cerebrospinal fluid. These findings begin to corroborate the neuroinflammatory hypothesis of ALS in the human disease.

### Example 11: Distinct Cytokine Profiles in Sjogren's Syndrome (SS)

A distinct profile of cytokines was produced from patients with Sjogren's Syndrome (SS) This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

A multiplex suspension array technology was used to quantify 28 cytokines and chemokines in serum and plasma of 11 patients who fulfilled the revised European classification criteria for primary SS, and healthy controls.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

The key cytokines/chemokines that discriminate SSGC+ patients from GC- patients and controls are BAFF, CCL2 (MCP-1), IL-12p40, CXCL8 (IL-8) and CCL11 (Eotaxin). We further demonstrated the dominant discriminative role of BAFF and CCL2 (MCP-1) between SSGC+ and SSGC- and signified its important role in ectopic salivary GC development in SS. The role of BAFF in GC formation in animal models has been investigates and showed normal induction but attenuated progression of GC responses in BAFF and BAFF-R signaling-deficient mice (14). Our results indicate that BAFF has a crucial 'driving force' in the development of ectopic GCs, a potential pre-stage of lymphomagenesis characterized by B-cell hyperactivity, proliferation and autoantibody secretion. Interestingly, patients with B-cell non-Hodgkin's lymphoma (NHL) have elevated BAFF serum levels and in positive correlation with disease-severity. Also, BAFF levels correlate with response to therapy in patients with B-cell NHL. Responding patients had a significantly lower BAFF level than those with progressive disease (42). Considering these findings, we hypothesize that in SS, elevated levels of BAFF, together with a unique pattern of elevated circulating cytokines, such as CCL2 (MCP-1), IL-12p40, CXCL8 (IL-8) and CCL11 (Eotaxin) among other factors, lead to GC formation and presumably this chronic stimulation might lead to B-cell neoplastic transformation. Moreover, since BAFF is produced by macrophages, monocytes and dendritic cells (43-45), the discriminant potential of CCL2 (MCP-1) is presumably by stimulating monocytes/macrophages to produce BAFF, therefore indirectly support the BAFF-mediated processes. The key cytokines/chemokines that discriminate SSGC+ patients from GC- patients and controls are BAFF, CCL2 (MCP-1), IL-12p40, CXCL8 (IL-8) and CCL11 (Eotaxin). We further demonstrated the dominant discriminative role of BAFF and CCL2 (MCP-1) between SSGC+ and SSGC- and signified its important role in ectopic salivary GC development in SS. The role of BAFF in GC formation in animal models has been investigates and showed normal induction but attenuated progression of GC responses in BAFF and BAFF-R signaling-deficient mice (14). Our results indicate that BAFF has a crucial 'driving force' in the development of ectopic GCs, a potential pre-stage of lymphomagenesis characterized by B-cell hyperactivity, proliferation and autoantibody secretion. Interestingly, patients with B-cell non-Hodgkin's lymphoma (NHL) have elevated BAFF serum levels and in positive correlation with disease-severity. Also, BAFF levels correlate with response to therapy in patients with B-cell NHL. Responding patients had a significantly lower BAFF level than those with progressive disease (42). Considering these findings, we hypothesize that in SS, elevated levels of BAFF, together with a unique pattern of elevated circulating cytokines, such as CCL2 (MCP-1), IL-12p40, CXCL8 (IL-8) and CCL11 (Eotaxin) among other factors, lead to GC formation and presumably this chronic stimulation might lead to B-cell neoplastic transformation. Moreover, since BAFF is produced by macrophages, monocytes and dendritic cells (43-45), the discriminant potential of CCL2 (MCP-1) is presumably by stimulating monocytes/macrophages to produce BAFF, therefore indirectly support the BAFF-mediated processes. Our results imply that a complex disorder of circulating serum cytokines contribute to Sjogren's Syndromes. We believe that the utilization of the multiplex cytokine array system in SS provides a powerful tool to sub-categorize the disease and find the relevant features of heterogeneous patients and along with common clinical and laboratory parameters help to evaluate these two types of the disease.

The key cytokines/chemokines that discriminate patients and controls are CCL2/MCP-1, IL-12, CXCL8/IL-8, CCL11/Eotaxin, TNFα, IL-2, IFNα, IL-15, IL17, IL-1α IL-1β, IL-6, and GM-CSF. In separate ongoing studies utilizing the multiplex cytokine assay, serial serum cytokine level measurements did not fluctuate significantly. Moreover, to exclude modulations in the circulating cytokine levels we selected patients in this study, who at the time of the analysis were not taking immunmodulatory medications, which would seriously affect the cytokine levels.

The inventors conclude that the multiplex cytokine assay along with the utilization of DFA can clearly distinguish SS patients into distinct subsets and from healthy controls. This information represents a powerful tool to pinpoint candidate discriminative cytokines on the basis of which the follow-up of patients can be carried out. The identification of cytokine-markers of the potentially prelymphomatous disease entity can be important not only in diagnosis, but also in designing selective anti-cytokine therapies that would inhibit the perpetuation of ectopic germinal center formation commonly found in Sjogren's Syndrome.

### Example 12: Distinct Cytokine Profiles in Early Arthritis

A distinct profile of cytokines was produced from patients with Early Arthritis. This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

A multiplex suspension array technology was used to quantify 28 cytokines and chemokines in plasma from 41 patients with early and undifferentiated arthritis and 21 healthy controls.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. The inventors used variant DFA named the Forward Stepwise Analysis.

### 4. Results

Both pro- and anti-inflammatory cytokine were elevated in patients over controls. Ther cytokines upregulated in patients included CCL4/MIP1β, CXCL8/IL-8, IL-2, IL-12, IL-17, IL-13, TNFα, IL-4, IL-5, and IL-10. Early untreated inflammatory arthritis can be categorized into distinct subgroups based on cytokine profiles. Integration of cytokine profiles may assist prognostication and treatment decisions in these patients.

### Example 13: Distinct Cytokine Profiles in Psoriasis (PSO)

A distinct profile of cytokines was produced from patients with Psoriasis (PSO). This distinct cytokine profile was determined by sampling serum for the presence of cytokines. Patients were found to have predictive molecular cytokine profiles based on clinical disease phenotype and disease severity. Specifically, cytokines were found to be similar within the specific disease classifications, but the levels of cytokines were somewhat heterogeneous with regard to individual patient, raising the possibility that various stages of disease and disease severity may be distinguished by this molecular diagnostic mechanism.

### 1. Study Population

A multiplex suspension array technology was used to quantify 28 cytokines and chemokines in serum 5 patients with clinically diagnosed psoriasis and 5 healthy controls.

### 2. Cytokine Measurement

The samples were analyzed using a comprehensive biometric multiplex cytokine assay (BioSource, Camarillo, CA, USA) (described above).

### 3. Statistical Analysis

The clinical presentations of the cytokine profiles in the defined patient subsets were compared by using Student's t-test, stepwise discriminate function tests, and a robust cluster analysis. Differentially expressed cytokines were identified first by a paired Student's t-test with the commonly accepted significance threshold of p<0.05. The Student's t-test was carried out using Microsoft Excel (Microsoft, Inc., Redman WA). Taking into consideration individual variances among the patient population, a second method of selection was also utilized.

Discriminant function analysis (DFA) (described above) was used for selection of the set of cytokines with maximal discriminatory capabilities between groups of samples from patients and unaffected controls. We used variant DFA named the Forward Stepwise Analysis.

### 4. Results

Serum cytokines IL-1β, IL-2, CXCL8/IL,-8, IL-13, G-CSF, and CCL4/MIP-1β were upregulated in psoriasis patients. FGF was the down regulated to levels lower than that seen in controls. The series of serum cytokines in this profile is unique to psoriasis The identification of cytokine-markers in psoriasis can be important not only in diagnosis, but also in designing selective anti-cytokine therapies that would prevent the scaring and physical discomfort of the disease.

### VI. References

The following reference have been discussed hereinabove.
U.S. Patent 6,555,320
U.S. Patent 6,406,884
U.S. Patent 6,190,857
U.S. Patent 6,183,951
U.S. Patent 6,162,604
U.S. Patent 5,965,379
U.S. Patent 5,965,366
U.S. Patent 5,786,154
U.S. Patent 5,753,442
U.S. Patent 5,646,005
U.S. Patent 5,599,795
U.S. Patent 5,587,294
U.S. Patent 5,443,826
U.S. Patent 3,817,837
U.S. Patent 3,850,752
U.S. Patent 3,939,350
U.S. Patent 3,996,345
U.S. Patent 4,843,092
U.S. Patent 4,275,149
U.S. Patent 4,277,437
U.S. Patent 4,366,241
U.S. Publication No. 2002/0137082
U.S. Publication No. 2003/0017493
U.S. Publication No. 2003/0017492
U.S. Publication No. 2003/0021780
Akira et al. FASEB Journal 4: 2860-7 (1990).
Akiyama et al., Neurobiol Aging 21:383-421 (2000).
Arend, Arthritis Rheum. 44:2224-2234 (2001).
Arend et al., Annu Rev Immunol 16:27-55 (1998).
Arend & Dayer, Arthritis Rheum 38:151-60 (1995).
Armitage JO, Semin. Hematology 29: s14-8 (1992).
Autenrieth et al., Infect Immun 62:2590-9 (1994).
Baggiolini et al., Advances in Immunology 55: 97-179 (1994).
Ball, Ann. Rheum. Dis., 30:213-223, 1971.
Bauer and Herrmann, Ann. Hematology 62: 203-10 (1991).
Beall et al., Journal of Biological Chemistry 267: 3455-9 (1992).
Bendzen et al., Scand J Rheumatol 28:599-606 (1988).
Berthelot et al., Joint Bone Spine, 69(2):114-122, 2002.
Bertone et al., Vet. Surg., 30(6):528-538, 2001.
Besnard et al., Gut 43(5):634-38 (1998).
Beutler and Cerami, Annual Review of Biochemistry 57: 505-18 (1988).
Bigda et al., Leuk. Lymphoma 10: 121-5 (1993).
Bischoff et al., European Journal of Immunology 23: 761-7 (1993).
Bischoff et al., Journal of Experimental Medicine 175: 1271-5 (1992).
Blay et al., Blood 82: 2169-74 (1993).
Blumberg et al., Arthritis Rheum 7:93-7 (1964).
Botoman et al., Am. Fam. Physician 57(1):57-68 (1998).
Boulay and Paul, Current Opinion in Immunology. 4: 294-8 (1992).
Bowness, Rheumatology (Oxford), 41(8):857-868, 2002.
Brach MA and Herrmann F. International Journal of Clinical and Laboratory Research 22: 143-51 (1992).
Brandt et al., Arthritis Rheum 43:1346-52 (2000).
Braun et al., Arthritis Rheum 42:2039-44 (1999).
Brewerton et al., Lancet, 1:904-907 (1973a).
Brewerton et al., Lancet, 1:956-957 (1973b).
Broom et al., British Journal of Cancer. 66: 1185-7 (1992).
Brown et al., Ann. Rheum. Dis., 61(8):764-765 (2002).
Brynskov et al., N. Engl. J. Med. 321(13):845-50 (1989).
Burbach et al., Current Eye Research 23(1): 64-8 (2001).
Burger & Dayer, Neurology 45(6 Suppl. 6):S39-43 (1995).
Calin et al., In: The Spondylarthritides, Calin et al. (Eds.), Oxford, UK. Oxford University Press, p. 179, 1998.
Callard RE. British Journal of Haematology 78: 293-9 (1991)
Canete et al., Ann. Rheum. Dis., 59(4):263-268 (2000).
Cann et al., Gut 24(5):405-11 (1983).
Cannon et al., Lymphokine Res., 7:457-465 (1988).
Capper et al., Cytokine, 2:182-189 (1990).
Charon et al., Infec. Immun., 38:1190-11.95 (1982).
Chehimi et al., European Journal of Immunology 23: 1826-30 (1993).
Chomarat et al., Arthritis Rheum 38:1046-54 (1995).
Cocchi et al., Science 270: 1810-1814 (1995).
Coceani et al., Brain Res., 446:245-250 (1988).
Cocks et al., International Immunology 5: 657-63 (1993).
Coffman et al., Science 245: 308-10 (1989).
Conti et al., J. Biological Chemistry 272(4): 2035-7 (1997).
Conti et al., Brain Research and Molecular Brain Research 67(1): 46-52 (1999).
Dancescu et al., Journal of Experimental Medicine 176: 1319-26 (1992).
De Jager et al., Clin. Diagn. Lab. Immunol. 10(1):133-139 (2003).
De Maeyer De Maeyer-Guignard, Current Opinion in Immunology 4: 321-6 (1992).
de Saint-Vis B et al., J Immunology 160(4): 1666-76 (1998).
de Waal-Malefyt R et al., Current Opinion in Immunology 4: 314-20 (1992).
Demetri and Griffin, Blood 78: 2791-2808 (1991).
Derkx et al., Lancet 342: 173-4 (1993).
de Waal et al., Exp Med 174:1209-20 (1991).
Didierjean et al., Cytokine 2:438-446 (1990).
Dinarello, Int Rev Immunol 16:457-99 (1998).
Dionne et al., Clin. Exp. Imunol. 112(3):435-42 (1998).
Doran et al., J. Rheumatol. 30(2):316-20 (2003).
Drossman et al., Dig Dis Sci. 38(9):1569-80 (1999).
Drossman et al., Gastroenterology 112(6):2120-37 (1997).
Dullens et al., In vivo 5: 567-70 (1991).
Dullens et al., In vivo 5: 567-70 (1991).
Eastgate et al., Lancet 2:706-9 (1988).
Eikelenboom et al., GLIA 40:232-239 (2002).
Ettehadi et al., Clin Exp Immunol 96:146-51 (1994).
Everhart and Renault, Gastroenterology 100(4):998-1005 (1991).
Fan et al., In vivo 5: 571-8 (1991).
Fearon and Locksley, Science 72:50-53 (1996).
Feldtkeller et al., Rheumatol. Int. 23(2):61-66 (2003).
Fellerman et al., Am. J. Gastroenterol. 93(10):1860-66 (1998).
Finkelman, J. Exp. Med. 182:279-282 (1995).
Firestein et al., Arthritis Rheum 37:644-52 (1994).
Fleischmann, Clin. Ther. 21(9):1429-1442 (1999).
Fodor et al., Biochemistry 30(33):8102-8108, 1991.
Freund and Kleine, Infection 20: S84-92 (1992).
Fujikawa et al., Ann Rheum Dis 54:318-20 (1995).
Funakoshi et al., Digestion 59(1):73-78 (1998).
Furukawa et al., European Journal of Pediatr. 151: 44-7 (1992).
Gabrilove, Growth Factors 6: 187-91 (1992).
Galley & Webster, Br. J. Anaesth. 77:11-16 (1996).
Gardella et al., FEBS Letters 481 (3): 245-8 (2000).
Gehrmann et al., Neuropathol Appl Neurobiol. 21:277-289 (11995).
Gianni et al., Journal of Clin. Oncol. 10: 1955-62 (1992).
Gifford and Duckworth, Biotherapy 3: 103-11 (1991).
Gillitzer et al., Journal of Investigative Dermatology 97: 73-9 (1991).
Gladman, Rheum Dis Clin North Am, 18:247-56 (1992).
Gladman et al., J Rheumatol 22:675-9 (1995).
Gladman et al., Q J Med 62:127-141 (1987).
Graeber et al., GLIA 40:252-259 (2002).
Gray and Goeddel, Lymphokines 13: 151-62 (1987).
Griffin et al., Proc Natl Acad Sci USA. 86, 7611-7615 (1989).
Gu et al., J. Rheumatol. 29(10):2159-2164 (2002).
Gu et al., Rheumatology (Oxford), 41(7):759-766 (2002).
Gwee et al., Gut 44(3):400-6 (1999).
Hacia et al., Nature Genet. 14:441-449 (1996).
Hahn & Tsao, Antibodies to DNA, In: Dubois' Lupus Erythematosus, 4th ed., Wallace & Hahn, eds., Lea and Febiger, Philadelphia, pp, 195-201 (1993).
Hanifin et al. J Am. Acad. Dermatology 28: 189-97 (1993).
Hannum et al., Nature 343:336-40 (1990).
Harrison et al., J Rheumatol. 25(12):2324-2330 (1998).
Hart et al., Clin. Exp. Immunol. 99(3):331-337 (1995).
Hart et al., Immunology 84:536-42 (1995).
Heinzel et al., J. Exp. Med., 169:59-72 (1989).
Helliwell et al., Br J Rheumatol 30-339-45 (1991).
Herbert et al., FEBS Letters 328: 268-70 (1993).
Hersh et al., Journal of Immunother. 10: 426-31 (1991).
Hock et al., Journal of Experimental Medicine 174: 1291-8 (1991).
Hoffenberg et al., J. Pediatr. 134(4):447-52 (1999).
Hollander et al., Ann. Intern. Med. 105:883-85 (1986).
Hollander, Scand. J. Gastroenterol. 27:721-26 (1992).
Hora et al., Proc Natl Acad Science USA 89: 1745-9 (1992).
Horwitz and Fisher, NEngl J Med. 344(24):1846-50 (2001).
Horwood et al., J Clinical Investigation 101(3): 595-603 (1998).
Howard and O'Garra, Immunology Today 13: 198-200 (1992).
Hsu et al., Hum. Pathol. 24: 833-9 (1993).
Imseis et al., J. Soc. Gynecol. Investig. 4(2):90-94 (1997).
Ishizawa and Dickson, JNeuropathol Exp Neurol. 60:647-657 (2001).
Jacob et al., Proc Natl Acad Sci USA 87:1233-7 (1990).
Jailwala et al., Ann Intern Med.133(2):136-47 (2000).
Jakubowski et al., New England Journal of Medicine 320: 38-42 (1989).
Jandinski et al., J. Dent. Res., 67:2307 (1988a).
Jandinski et al., J. Dent. Res., 68:1233 (1988c).
Jandinski et al., J. Dent. Res., 68:6526 (1988b).
Jansen et al., Blut 60: 269-74 (1990).
Jarvis; Curr Opin Rheumatol. 10:459-467 (1998).
Jarvis, Pediatr Ann. 31:437-446 (2002).
Jeffes et al., J. Clin. Immunol. 4:31-35 (1984).
Jiang et al., J. Immunology 148: 2423-8 (1992).
Jones et al., Br J Rheumatol 33:834-9 (1994).
Jonsson et al., Br J Rheumatol. 32: 578-81 (1993).
Jonsson et al., Oral Dis. 8:130-140 (2002).
Jonsson and Brokstad, A Textbook of Rheumatology. 6th ed. Philadelphia: Lippincott Williams & Wilkins, pp. 495-504 (2001).
Jordan et al., J. Heart Lung Transplant, 11 (5):1001-1004, 1992.
Kabashimi et al., Infec. Immun., 58:2621-2627, 1990.
Kahle et al., Ann Rheum Dis 51:731-4 (1992).
Kellow and Phillips, Gastroenterology 92(6):1885-93 (1987).
Khan et al., Mol. Cell Endocrinol., 58:221-230, 1988.
Khan, Ann. Intern. Med. 136(12):896-907, 2002.
Khan, Clin. Exp. Rheumatol. 20(6):6-10, 1998.
Khan, In: Ankylosing spondylitis and related spondyloarthropathies, Spine, State of the Art Reviews, 1990.
Kikkawa et al., Biochemical Biophysical Research Communications 281(2): 461-7 (2001). Kimball et al., J. Immunol. 133:256-260, 1984. Kishimoto, Blood 74: 1-10 (1990).
Klein et al., Cytokine 11(6): 451-8 (1999).
Koch et al., Journal of Immunology 147: 2187-95 (1991).
Kollnberger et al., Arthritis Rheum., 469(11):2972-2982, 2002.
Kotake et al., Infect Immun 67:2682-6 (1999).
Kotzin, Cell 85:303-06 (1996).
Kotzin & O'Dell, Systemic lupus erythematosus, In: Samler's Immunologic Diseases, 5th ed., M. M. Frank et al., eds., Little Brown & Co., Boston, pp.667-97 (1995).
Kuboyama, Kurume Med J. 45(1):33-37 (1998).
Lahesmaa et al., J Immunol 148:3079-85 (1992).
Lee et al., Exp. Mol. Med., 31(3):159-156, 1999.
Leger-Ravet et al., Blood 78: 2923-30 (1991).
Leiper et al., Baillieres Clin. Gastroenterol. 12(1):179-99 (1998).
Leonard et al., Advances in Experimental Medicine and Biology 305: 57-64 (1991).
Leonard et al., Immunology Today. 11: 97-101 (1990).
Lieschke et al., British Journal of Haematology 82: 589-95 (1992).
Lindemann et al., Blood 73: 880-4 (1989).
Lipsky, PE. Rheumatoid arthritis. In: Fauci et al., eds. Harrison's principles of internal medicine. 14th edition, New York: McGraw-Hill, pp. 1880-8 (1998).
Lo et al., Immunol Rev. 169:225-239 (1999).
Lord et al., Blood 79: 2605-2609 (1992).
Lugering et al., Ital. J. Gastroenterol. Hepatol. 30(3):338-44 (1998).
Lynn and Friedman, N Engl J Med. 329(26):1940-5 (1993).
McAlindon et al., Gut 42(2):214-19 (1998).
Macatonia et al., J Immunol 150:3755-65 (1993).
Machold et al., Ann. Rheum. Dis. 51: 1039-43 (1992).
Makowiec et al., Z. Gastroenterol. 36(8):619-24 (1998).
Marsal et al., Rheumatology 38:332-7 (1999).
Matsushima et al., Advances in Experimental Medicine and Biology 305: 31-8 (1991).
McGonagle et al., Curr Opin Rheumatol 11:244-50 (1999).
McGonagle et al., Arthritis Rheum 41:694-700 (1998).
McKenzie et al., Proceedings of the National Academy of Science (USA) 90: 3735-9 (1993).
Mease et al., Lancet 356:385-90 (2000).
Mertz et al., Gastroenterology 118(5):842-8 (2000).
Micallef et al., Cancer Immunology and Immunotherapy 43:361-367 (1997).
Micallef et al., European Journal of Immunology 26: 1647-1651 (1996).
Minty et al., Nature (London) 352: 248-50 (1993).
Moll & Wright, Semin Arthritis Rheum 3:55-78 (1973).
Moll & Wright, Ann Rheum Dis 32:181-201 (1973).
Montaner et al., Journal of Experimental Medicine 178: 743-7 (1993).
Moore, Annual Review of Immunology 9: 159-91 (1991).
Morel et al., Laboratory Investigations 81(10): 1371-83 (2001).
Mule et al., Research Immunology 143: 777-9 (1992).
Murch, Nutrition 14:780-83 (1998).
Nakamura et al., In: Handbook of Experimental Immunology (4th Ed.), Weir et al., (Eds). 1:27, Blackwell Scientific Publ., Oxford, 1987.
Nakanishi et al., Annual Reviews of Immunology 19: 423-74 (2001).
Neal et al., BMJ314(7083):779-82 (1997).
Negrin and Greenberg, Advances in Pharmacol. 23: 263-296 (1992).
Nielen et al., Arthritis Rheum. 50(2):380-386 (2004).
Numasaki et al., Blood 101(7):2620-7 (2002).
Ochensberger et al., Blood 88(8): 3028-37 (1996).
Ohnishi et al., Int. Immunol. 6:817-30 (1994).
Ohzato et al., Surgery 111: 201-9 (1992).
Okabe et al., Lymphoma 8: 57-63 (1992).
Okamura et al., Nature 378: 88-91 (1995).
Oppenheim et al., Transplant. Proc. 14:553-555, 1982.
Orr et al., Progr Neurobiol. 68:325-340 (2002).
Ortiz, Rheumatol. Int. 21(5):182-188, 2002.
Partsch et al., Br J Rheumatol 24:518-23 (1997).
Paul, Blood 77: 1859-70 (1991).
Paul, FASEB Journal 1: 456-61 (1987).
Pavletic et al., Experimental Hematology 21: 1371-8 (1993).
Pease et al., Proc. Natl. Acad. Sci. USA, 91:5022-5026, 1994.
Peichl et al., Scandinavian Journal of Immunology 34: 333-9 (1991).
Peter et al., Neurology, 41 :121-123,1991.
Pimentel et al., Am J Gastroenterol. 95(12):3503-6 (2000).
Pizarro et al., Immunology 162: 6829-6835 (1999).
Pociot et al., Scand J Immunol 42:501-4 (1995).
Prieur et al., Lancet 2:1240-2 (1987).
Punnonen et al., Proc Natl Acad Sci USA 90: 3730-4 (1993).
Rantapaa-Dahlqvist et al., Arthritis Rheum. 48(10):2741-2749 (2003).
Reimund et al., Eur. J. Clin. Invest. 28(2):145-50 (1998).
Ribbens et al., Eur Cytokine Netw 11:669-76 (2000).
Rogers et al., Neurobiol Aging 9:339-349 (1988).
Rogler &. Andus, World J. Surg. 22(4):382-89 (1998).
Rooney et al., Rheumatol Int 10:217-9 (1990).
Rothstein, Med Clin North Am. 84(5):1247-57 (2000).
Rudwaleit and Hohler, Curr. Opin. Rheumatol. 13(4):250-254, 2001.
Rudwaleit et al., Ann. Rheum. Dis. 61(11):968-974, 2002.
Ruef and Coleman, Rev. Infect. Dis. 12: 41-62 (1990).
Ruemmele et al., Gastroenterol. 115(4):822-29 (1998).
Saiki et al., Scand. J. Gastroenterol. 33(6):616-22 (1998).
Salomonsson et al., Arthritis Rheum. 48:3187-201 (2003).
Salomonsson et al., Scand J Immunol. 55: 336-42 (2002).
Salvarani et al., Curr Opin Rheumatol 10:299-305 (1998).
Sanderson et al., Immunological Reviews 102: 29-50 (1988).
Sandler, Gastroenterology 99(2):409-15 (1990).
Sartor, Am. J. Gastroenterol. 92(12):5S-11S (1997).
Schellekens et al., Arthritis Rheum. 43(1):155-163 (2000).
Schlaak et al., Clin Exp Rheumatol 14:155-62 (1996).
Schlaak et al., Eur J Immunol 22:2771-6 (1992).
Schlosstein et al., NE J. Medicine 288:704-706, 1973.
Schreiber, Neth. J. Med. 53(6):S24-31 (1998).
Schuster, Infection 20: S95-9 (1992).
Seki et al., J Immunology 166(4): 2651-7 (2001).
Shoemaker et al., Nature Genetics 14:450-456 (1996).
Sieper & Braun, Arthritis Rheum 38:1547-54 (1995).
Signore et al., Cytokine 12(10):1445-1454 (2000).
Simon et al., Clin Exp Immunol 94:122-6 (1993).
Simon et al., Proc Natl Acad Sci USA 91:8562-6 (1994).
Smith, Science 240: 1169-76 (1988).
Soderholm et al., Gastroenterol. 117:65-72 (1999).
Sonderlin, Ann. Rheum. Dis. 61(10):911-915 (2002).
Stack et al., Lancet 349(9051):521-24 (1997).
Steinborn et al., Z Geburtshilfe Perinatol 198(1):1-5 (1994).
Stober et al., J Immunology 167(2): 957-65 (2001).
Stoll et al., European Journal of Immunology 28(10): 3231-9 (1998).
Stoll et al., J Immunology 159: 298-302 (1997).
Stotter et al., J Immunology 146: 150-5 (1991).
Strieter et al., Crit. Care Med. 21: S447-63 (1993).
Stuart-Harris et al., Med. Journal of Aust. 156: 869-72 (1992).
Suk et al., Immunology Letters 77(2): 79-85 (2001).
Takatsu, Current Opinion in Immunology 4: 299-306 (1992).
Takeuchi et al., Cell Tissue Research 289: 499-503 (1997).
Talley et al., Gastroenterology 109(6):1736-41 (1995).
Tamatani et al., Immunology 65:337-342 (1988).
Targan et al., N. Engl. J. Med 337(15):1029-35 (1997).
Todd et al., Science 240:1003 (1988).
Toi et al., Cancer Research 52: 275-9 (1992).
Torigoe et al., J Biological Chemistry 272(41): 25737-25742 (1997).
Touzani et al., J Neuroimmunol. 100:203-215 (1999).
Trinchieri et al., Immunology Today 14: 335-8 (1993).
Trinchieri et al., Progress in Growth Factor Research 4: 355-68 (1992).
Tsunoda et al., Lymphokine Res. 7:333 (1988).
Tsutsui et al., J Immunology 157: 3967-3973 (1996).
Udagawa et al., J Experimental Medicine 185: 1005-1012 (1997).
Ushio et al., J Immunology 156: 4274-4279 (1996).
van den Berg, Semin Arthritis Rheum 30(5 Suppl. 2):7-16 (2001).
van Dullemen et al., Gastroenterol. 109(1):129-35 (1995).
van Hogezand & Verspaget, Drugs 56(3):299-305 (1998).
Van Oers et al., Ann. Hematology 66: 219-23 (1993).
Van Snick, Annual Review of Immunology 8: 253-78 (1990).
Villiger et al., Journal of Clinical Investigation 1 90: 488-96 (1992).
Waldmann et al., Annals of the New York Academy of Sciences 685: 603-10 (1993).
Walker et al., J Immunology 162: 5894-5901 (1999).
Warrington et al., Arthritis Rheum. 44:13-20 (2001).
Watanabe et al., J Clinical Immunology 17(4): 282-292 (1997).
Waxman and Balkwill, (eds), Interleukin 2. Blackwell Scientific Publ. Oxford (1992).
Westacott et al., Ann. Rheum. Dis. 49:676-681 (1990).
Weyand and Goronzy, Ann NY Acad Sci. 987:140-9 (2003).
Whitehead et al., Gastroenterology 98(5 Pt 1):1187-92 (2000).
Williams et al., Hematology Pathol. 5: 45-55 (1991).
Williams et al., Proc Natl Acad Science USA 89: 9784-8 (1992).
Wilmott et al., Lymphokine Res. 7:334 (1988).
Wolvekamp and Marquet, Immunology Letters 24: 1-9 (1990).
Wordsworth, In: Genes and Arthritis, Brit. Medical Bulletin 51:249-266 (1995).
Wright, V., Clin Orthop Related Res 143:8-14 (1979).
Wright, V., Ann Rheum Dis 15:348-56 (1956).
Wu et al., Transplantation 54(2):326-332 (1992).
Xanthou et al., Arthritis Rheum. 44: 408-18 (2001).
Yamamura et al., Science 254:277-279 {1991}.
Yao et al., Immunity 3(6): 811-821 (1995).
Yin et al., Rheumatology 38:1058-67 (1999).
Yin et al., Arthritis Rheum 40:1788-97 (1997).
Ylä-Herttuala et al., Proceedings of the National Academy of Science (USA) 88: 5252-6 (1991).
Yoshimoto et al., Proc Natl Acad Sci USA 94(8); 3948-53 (1997).
Zlotnik and Moore, Cytokines 3: 366-71 (1991).
Zwahlen et al., International Rev. Experimental Pathol. 34: 27-42 (1993).

## Claims

1. A method for diagnosing ankylosing spondylitis, the method comprising the steps of:
(a) measuring the levels of eleven cytokines within a sample previously obtained from a patient;
(b) comparing each level of the eleven cytokines with the reference level of the eleven cytokines respectively, wherein each reference level is the median level of each of the eleven cytokines found in samples of a population of healthy individuals and/or in samples of a population of patients having ankylosing spondylitis; and
(c) determining if a patient has ankylosing spondylitis based on the comparison in step (b),
wherein the eleven cytokines are MIP-1β/CCL4, IL-4, IL-6, IL-8/CXCL8, IL-13, IL-17, TNF-α, IFNγ, CCL2/MCP-1, GM-CSF, and G-CSF.

2. The method of claim 1, wherein the step of determining further comprises the step of classifying the patient as having mild, intermediate, or severe ankylosing spondylitis.

3. The method of claim 1, wherein the patient sample is from a healthy subject or from a diseased patient.

4. A method for assessing treatment for ankylosing spondylitis, the method comprising the steps of:
(a) measuring each level of eleven cytokines within a sample previously obtained from a patient, wherein the patient is an ankylosing spondylitis patient that had previously been subjected to a treatment;
(b) comparing each level of the eleven cytokines with the reference level of each of the eleven cytokines respectively, wherein each reference level is the median level of each of the eleven cytokines found in samples of a population of healthy individuals and/or in samples of a population of patients having ankylosing spondylitis; and
(c) determining if the treatment is efficacious,
wherein the eleven cytokines are MIP-1β/CCL4, IL-4, IL-6, IL-8/CXCL8, IL-13, IL-17, TNF-α, IFNγ, CCL2/MCP-1, GM-CSF, and G-CSF.

5. The method of claim 4, wherein the reference levels comprise pretreatment levels of the eleven cytokines, levels of the eleven cytokines observed in healthy subjects and/or in a patient with ankylosing spondylitis.

6. A method for determining if a patient with ankylosing spondylitis is predisposed to develop a severe ankylosing spondylitis, the method comprising the steps of:
(a) measuring the levels of eleven cytokines within a sample previously obtained from a patient;
(b) comparing each level of the eleven cytokines with the reference level of each of the eleven cytokines respectively, wherein each reference level is the median level of each of the eleven cytokines found in samples of a population of healthy individuals and/or in samples of a population of patients having ankylosing spondylitis; and
(c) determining if said patient is predisposed to develop severe ankylosing spondylitis,
wherein the eleven cytokines are MIP-1β/CCL4, IL-4, IL-6, IL-8/CXCL8, IL-13, IL-17, TNF-α, IFNγ, CCL2/MCP-1, GM-CSF, and G-CSF.

7. The method of any of claims 1, 4, or 6, wherein the patient sample comprises peripheral blood, serum, plasma, cerebrospinal fluid, tissue, skin, or another body fluid.

8. A kit for providing diagnostic information about ankylosing spondylitis, the kit comprising nucleic acid or antibody probes for determining the level of the following eleven cytokines: MIP-1β/CCL4, IL-4, IL-6, IL-8/CXCL8, IL-13, IL-17, TNF-α, IFNγ, CCL2/MCP-1, GM-CSF, and G-CSF.

## Patentansprüche

1. Ein Verfahren zur Diagnostizierung von Spondylitis ankylosans, wobei das Verfahren die folgenden Schritte umfasst:
a) Messen der Mengen von elf Cytokinen in einer Probe, die zuvor von einem Patienten erhalten wurde,
b) Vergleichen jeder Menge der elf Cytokine mit der Referenzmenge der elf Cytokine, wobei jede Referenzmenge die mittlere Menge eines jeden der elf Cytokine ist, die in Proben einer Population gesunder Individuen und/oder in Proben einer Population von Patienten, die Spondylitis ankylosans haben, gefunden werden, und
c) Bestimmen, ob ein Patient Spondylitis ankylosans hat, basierend auf dem Vergleich in Schritt b), wobei die elf Cytokine MIP-1β/CCL4, IL-4, IL-6, IL-8/CXCL8, IL-13, IL-17, TNF-α, IFNγ, CCL2/MCP-1, GM-CSF und G-CSF sind.

2. Das Verfahren nach Anspruch 1, wobei der Schritt des Bestimmens ferner den Schritt des Klassifizierens des Patienten als milde, mittlere oder schwere Spondylitis ankylosans umfasst.

3. Das Verfahren nach Anspruch 1, wobei die Patientenprobe von einem gesunden Subjekt oder von einem erkrankten Patienten ist.

4. Ein Verfahren zur Bewertung der Behandlung von Spondylitis ankylosans, wobei das Verfahren die folgenden Schritte umfasst:
a) Messen jeder Menge der elf Cytokine in einer Probe, die zuvor von einem Patienten erhalten wurde, wobei der Patient ein Spondylitis ankylosans-Patient ist, der zuvor einer Behandlung unterzogen wurde,
b) Vergleichen jeder Menge der elf Cytokine mit der Referenzmenge der elf Cytokine, wobei jede Referenzmenge die mittlere Menge eines jeden der elf Cytokine ist, die in Proben einer Population gesunder Individuen und/oder in Proben einer Population von Patienten, die Spondylitis ankylosans haben, gefunden werden, und
c) Bestimmen, ob die Behandlung wirksam ist, wobei die elf Cytokine MIP-1β/CCL4, I-4, IL-6, IL-8/CXCL8, IL-13, IL-17, TNF-α, IFNγ, CCL2/MCP-1, GM-CSF und G-CSF sind.

5. Das Verfahren nach Anspruch 4, wobei die Referenzmengen Mengen der elf Cytokine in Patienten vor ihrer Behandlung, Mengen der elf Cytokine, die in gesunden Subjekten und/oder in einem Patienten mit Spondylitis ankylosans beobachtet werden, umfassen.

6. Ein Verfahren zur Bestimmung, ob ein Patient mit Spondylitis ankylosans prädisponiert ist eine schwere Spondylitis ankylosans zu entwickeln, wobei das Verfahren die folgenden Schritte umfasst:
a) Messen der Menge von elf Cytokinen in einer Probe, die zuvor von einem Patienten erhalten wurde,
b) Vergleichen jeder Menge der elf Cytokine mit der Referenzmenge der elf Cytokine, wobei jede Referenzmenge die mittlere Menge eines jeden der elf Cytokine ist, die in Proben einer Population gesunder Individuen und/oder in Proben einer Population von Patienten, die Spondylitis ankylosans haben, gesunden werden, und
c) Bestimmen, ob der Patient Spondylitis ankylosans hat, , wobei die elf Cytokine MIP-1β/CCL4, IL-4, IL-6, IL-8/CXCL8, IL-13, IL-17, TNF-α, IFNγ, CCL2/MCP-1, GM-CSF und G-CSF sind.

7. Das Verfahren nach einem der Ansprüche 1, 4 oder 6, wobei die Patientenprobe peripheres Blut, Serum, Plasma, cerebrospinale Flüssigkeit, Gewebe, Haut oder andere Körperflüssigkeiten umfasst.

8. Ein Kit zur Bereitstellung diagnostischer Information zu Spondylitis ankylosans, wobei das Kit Nukleinsäuren- oder Antikörper-Sonden zur Bestimmung der Menge der folgenden elf Cytokine umfasst: MIP-1β/CCL4, IL-4, IL-6, IL-8/CXCL8, IL-13, IL-17, TNF-α, IFNγ, CCL2/MCP-1, GM-CSF und G-CSF.

## Revendications

1. Méthode pour diagnostiquer la spondylarthrite ankylosante, ladite méthode comprenant les étapes suivantes:
(a) la mesure des niveaux de onze cytokines dans un échantillon préalablement obtenu d'un patient ;
(b) la comparaison de chaque niveau des onze cytokines avec le niveau de référence des onze cytokines respectivement, où chaque niveau de référence est le niveau médian de chacune des onze cytokines trouvées dans des échantillons d'une population d'individus sains et/ou dans des échantillons d'une population de patients ayant la spondylarthrite ankylosante; et
(c) déterminer si un patient a la spondylarthrite ankylosante sur la base de la comparaison de l'étape (b), où les onze cytokines sont MIP-1β/CCL4, IL-4, IL-6, IL8/CXCL8, IL-13, IL-17, TNF-α, IFNγ, CCL2/MCP-1, GM-CSF, and G-CSF.

2. Méthode selon la revendication 1, dans laquelle l'étape de détermination comprend en outre l'étape de classification du patient comme ayant une spondylarthrite ankylosante légère, intermédiaire, ou sévère.

3. Méthode selon la revendication 1, dans laquelle l'échantillon de patient provient d'un sujet sain ou d'un patient malade.

4. Méthode pour évaluer un traitement de la spondylarthrite ankylosante, ladite méthode comprenant les étapes suivantes :
(a) la mesure de chaque niveau de onze cytokines dans un échantillon préalablement obtenu d'un patient, où le patient est un patient avec la spondylarthrite ankylosante qui avait été préalablement soumis à un traitement;
(b) la comparaison de chaque niveau des onze cytokines avec le niveau de référence de chacune des onze cytokines respectivement, où chaque niveau de référence est le niveau médian de chacune des onze cytokines trouvées dans des échantillons d'une population d'individus sains et/ou dans des échantillons d'une population de patients ayant la spondylarthrite ankylosante; et
(c) déterminer si le traitement est efficace ;
où les onze cytokines sont MIP-1β/CCL4, IL-4, IL-6, IL8/CXCL8, IL-13, IL-17, TNFα, IFNγ, CCL2/MCP-1, GM-CSF, and G-CSF.

5. Méthode selon la revendication 4, dans laquelle les niveaux de référence comprennent des niveaux prétraitement des onze cytokines, des niveaux des onze cytokines observés chez des sujets sains et/ou chez un patient avec la spondylarthrite ankylosante.

6. Méthode pour déterminer si un patient avec une spondylarthrite ankylosante est prédisposé à développer une spondylarthrite ankylosante sévère, ladite méthode comprenant les étapes suivantes:
(a) la mesure des niveaux de onze cytokines dans un échantillon préalablement obtenu d'un patient ;
(b) la comparaison de chaque niveau des onze cytokines avec le niveau de référence de chacune des onze cytokines respectivement, où chaque niveau de référence est le niveau médian de chacune des onze cytokines trouvées dans des échantillons d'une population d'individus sains et/ou dans des échantillons d'une population de patients ayant la spondylarthrite ankylosante; et
(c) détenniner si ledit patient est prédisposé à développer une spondylarthrite ankylosante sévère,
où les onze cytokines sont MIP-1β/CCL4, IL-4, IL-6, IL8/CXCL8, IL-13, IL-17, TNF-α, IFNγ, CCL2/MCP-1, GM-CSF, and G-CSF.

7. Méthode selon l'une quelconque des revendications 1, 4, ou 6, dans laquelle l'échantillon de patient comprend du sang périphérique, du sérum, du plasma, du fluide cérébro-rachidien, un tissu, de la peau, ou tout autre fluide du corps.

8. Kit pour fournir une information diagnostique sur la spondylarthrite ankylosante, le kit comprenant des sondes d'acide nucléique ou d'anticorps pour déterminer le niveau des onze cytokines suivantes : MIP-1β/CCL4, IL-4, IL-6, IL8/CXCL8, IL-13, IL-17, TNF-α, IFNγ, CCL2/MCP-1, GM-CSF, and G-CSF.
